# EUROPEAN PATENT SPECIFICATION

(11) **EP 4 364 741 B1**
(45) Date of publication and mention of the grant of the patent: **25.11.2026**
(21) Application number: 24152903.1
(22) Date of filing: 17.08.2018
(51) Int. Cl.: A61P 35/00, A61K 31/529, A61K 45/06, A61K 31/519, C07D 498/08

(54) **POLYMORPHIC FORM OF TG02**
POLYMORPHE FORM VON TG02
FORME POLYMORPHE DE TG02

(30) Priority: 18.08.2017 US 201762547157 P
(43) Date of publication of application: 08.05.2024
(62) Document number(s) of the earlier application(s) in accordance with Art. 76 EPC: 18846214.7 / 3 668 876
(73) Proprietor: Cothera Bioscience, Inc., Grand Cayman, KY11111 (KY)
(72) Inventor: MANSFIELD, Robert, K., San Marcos, CA 92078 (US); PARROTT, Tracy, San Diego, CA 92104 (US)
(74) Representative: Schlich

(56) References cited:
- WO-A1-2007/058628
- US-A1- 2013 150 378
- PALLIS MONICA ET AL: "The multi-kinase inhibitor TG02 overcomes signalling activation by survival factors to deplete MCL1 and XIAP and induce cell death in primary acute myeloid leukaemia cells", BRITISH JOURNAL OF HAEMATOLOGY, BLACKWELL PUBLISHING LTD, vol. 159, no. 2, 1 October 2012 (2012-10-01), pages 191 - 203, XP002794766, ISSN: 1365-2141, DOI: 10.1111/BJH.12018

## Description

### BACKGROUND OF THE INVENTION

TG02 is a pyrimidine-based multi-kinase inhibitor that inhibits CDKs 1, 2, 5, 7, and 9 together with JAK2 and FLT3. It dose-dependently inhibits signaling pathways downstream of CDKs, JAK2 and FLT3 in cancer cells with the main targets being CDKs. TG02 is anti-proliferative in a broad range of tumor cell lines, inducing G1 cell cycle arrest and apoptosis. Primary cultures of progenitor cells derived from acute myeloid leukemia (AML) and polycythemia vera patients are very sensitive to TG02. Comparison with reference inhibitors that block only one of the main targets of TG02 demonstrate the benefit of combined CDK and JAK2/FLT3 inhibition in cell lines as well as primary cells. *See* Goh et al., Leukemia 26:236-43 (2012). TG02 is also known as SB1317 and by its chemical name: (16E)-14-methyl-20-oxa-5,7,14,26-tetraazatetracyclo[19.3.1.1(2,6).1(8,12)]heptacosa-1(25),2(26),3,5,8(27),9,11,16,21,23-decaene.

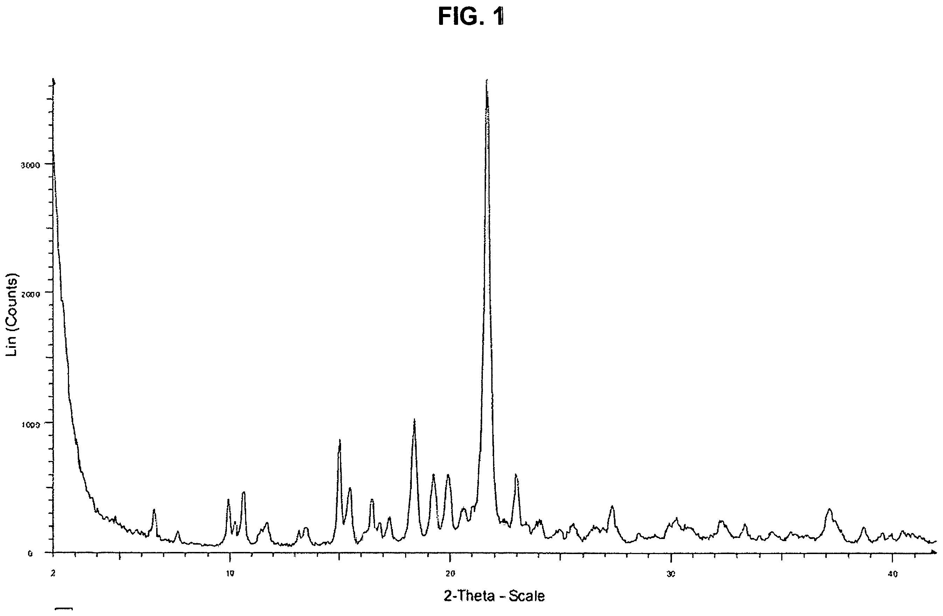

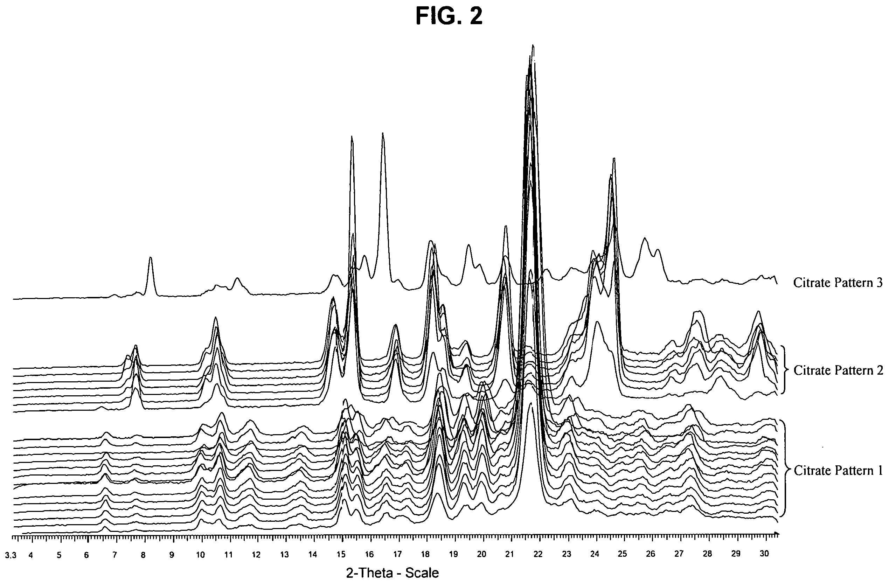

US 8,143,255 discloses TG02 as Compound 1. US 9,120,815 discloses various salt and crystalline forms of TG02, including TG02 citrate polymorphs refered to as Citrate Pattern 1, Citrate Pattern 2, and Citrate Pattern 3. The powder x-ray diffraction (PXRD or XRPD) pattern of TG02 Citrate Pattern 1, Citrate Pattern 2, and Citrate Pattern 3 are provided in Figs. 1 and 2.

### BRIEF SUMMARY OF THE INVENTION

The present inventionprovides a crystalline polymorphic form of the citrate salt of (16E)-14-methyl-20-oxa-5,7,14,26-tetraazatetracyclo[19.3.1.1(2,6).1(8,12)]heptacosa-1(25),2(26),3,5,8(27),9,11,16,21,23-decaene (TG02 Form X (citrate)) characterized as having a powder x-ray diffraction pattern with peaks at 15.2 ± 0.2 degrees 20, 15.5 ± 0.2 degrees 20, 21.7 ± 0.2 degrees 20, 22.1 ± 0.2 degrees 20, 23.0 ± 0.2 degrees 20, 26.2 ± 0.2 degrees 20, and 29.9 ± 0.2 degrees 20, wherein the powder x-ray diffraction pattern is collected using Cu Kα radiation, for use in the treatment of cancer in a patient, wherein said treatment further comprises administering to the patient a therapeutically effective amount of an anti-PD-1 antibody, an anti-PD-L1 antibody, or temozolomide.

### BRIEF DESCRIPTION OF DRAWINGS

Fig. 1 is a PXRD of Citrate Pattern 1 of US 9,120,815.
Fig. 2 is the PXRD of Citrate Patterns 1, 2, and 3 of US 9,120,815.
Fig. 3 is the PXRD of TG02 FB Form I.
Fig. 4 is the PXRD of TG02 FB Form II.
Fig. 5 is the PXRD of TG02 FB Form III.
Fig. 6 is the PXRD of TG02 FB Form IV.
Fig. 7 is the PXRD of TG02 FB Form V.
Fig. 8 is the PXRD of TG02 HCl Form VI.
Fig. 9 is the PXRD of TG02 HCl Form VII.
Fig. 10 is the PXRD of TG02 HCl Form VIII.
Fig. 11 is the PXRD of TG02 citrate Form X.

### DETAILED DESCRIPTION OF THE INVENTION

The present disclosure provides Form X (citrate), characterized as having a PXRD pattern with peaks at 15.2, 15.5, 21.7, 22.1, 23.0, 26.2, and 29.9 degrees 20.

In another embodiment, Form X (citrate) is characterized as having a PXRD pattern with peaks at 8.6, 9.4, 11.9, 15.2, 15.5, 17.0, 17.4, 19.6, 21.7, 22.1, 23.0, 26.2, and 29.9 degrees 20.

In another embodiment, Form X (citrate) is characterized as having a PXRD pattern with peaks at 8.6, 9.4, 11.9, 12.5, 14.3, 15.2, 15.5, 16.1, 16.4, 17.0, 17.4, 17.9, 19.0, 19.6, 20.3, 20.6, 21.2, 21.7, 22.1, 23.0, 23.5, 23.9, 24.2, 24.8, 26.2, 27.3, 28.0, and 29.9 degrees 20.

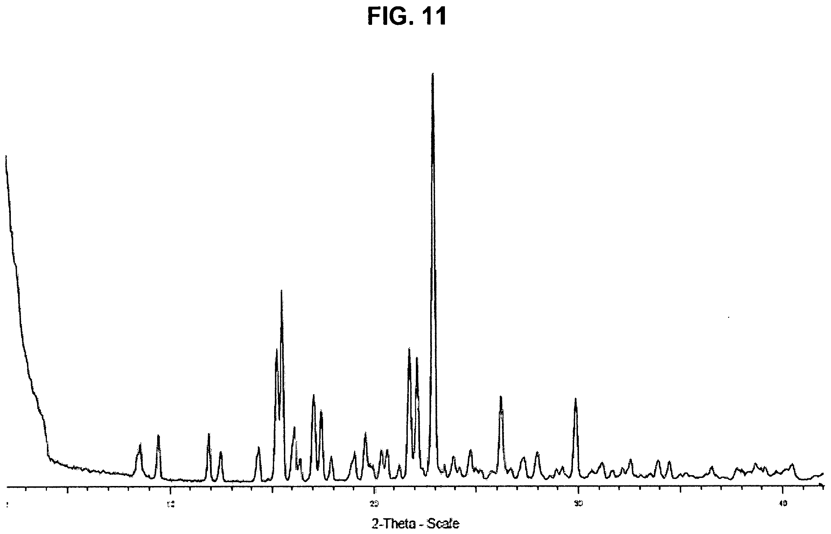

In another embodiment, Form X (citrate) is characterized as having a PXRD pattern that is essentially the same as Fig. 11.

In another embodiment, the present disclosure provides substantially pure Form X (citrate).

In another embodiment, the present disclosure provides pure Form X (citrate).

The Form X described above may be referred to herein as the "Tg02 citrate salt polymorphic form".

The present disclosure also provides a micronized form of the above TG02 citrate salt polymorphic form. In one embodiment, the average particle size distribution of the micronized TG02 citrate salt polymorphic form is about 20 µm or less, e.g., about 19 µm, about 18 µm, about 17 µm, about 16 µm, about 15 µm, about 14 µm, about 13 µm, about 12 µm, or about 11 µm, or less, as determined, for example, by laser diffraction spectroscopy. In another embodiment, the average particle size distribution is about 10 µm or less, e.g., about 9 µm, about 8 µm, about 7 µm, about 6 µm, or about 5 µm, or less. In another embodiment, the average particle size distribution is about 5 µm or less, *e.g.,* about 4 µm, about 3 µm, about 2 µm, or about 1 µm, or less. In another embodiment, the average particle size distribution is about 1 µm or less, e.g., about 0.9 µm, about 0.8 µm, about 0.7 µm, about 0.6 µm, about 0.5 µm, about 0.4 µm, about 0.3 µm, about 0.2µm, about 0.1 µm, about 0.09 µm, about 0.08 µm, about 0.07 µm, about 0.06 µm, about 0.05 µm, about 0.04 µm, about 0.03 µm, about 0.02 µm, or about 0.01 µm or less.

Also described herein are methods of making TG02 polymorphic forms. Methods of making TG02 polymorphic forms are described in the Examples provided herein below.

The references to methods of treatment in the subsequent paragraphs of this description are to be interpreted as references to the claimed polymorphic form for use in the method of treatment.

In another embodiment, the present disclosure provides therapeutic methods of treating a patient having cancer, the method comprising administering to the patient a therapeutically effective amount of the TG02 citrate salt polymorphic form, wherein one or more of the genes listed in Table 1, see below, is differentially present in a biological sample taken from the patient as compared with a biological sample taken from a subject of another phenotypic status. In another embodiment, MYC overexpression is differentially present in a sample taken from the patient. In another embodiment, MCL1 overexpression is differentially present in a sample taken from the patient.

In another embodiment, the present disclosure provides therapeutic methods of treating a patient having cancer, the method comprising administering to the patient a therapeutically effective amounts of the TG02 citrate salt polymorphic form and a second therapeutic agent, e.g., an immune checkpoint inhibitor, wherein one or more of the genes listed in Table 1, see below, is differentially present in a biological sample taken from the patient as compared with a biological sample taken from a subject of another phenotypic status. In another embodiment, MYC overexpression is differentially present in a sample taken from the patient. In another embodiment, MCL1 overexpression is differentially present in a sample taken from the patient. In another embodiment, the TG02 citrate salt polymorphic form is administered to the patient before the anti-PD-1 antibody, anti-PD-L1 antibody, or temozolomide. In another embodiment, the TG02 citrate salt polymorphic form is administered to the patient after the anti-PD-1 antibody, anti-PD-L1 antibody, or temozolomide. In another embodiment, the TG02 citrate salt polymorphic form is administered to the patient at the same time as the anti-PD-1 antibody, anti-PD-L1 antibody, or temozolomide.

In another embodiment, the present disclosure provides kits comprising the TG02 citrate salt polymorphic form and an anti-PD-1 antibody, an anti-PD-L1 antibody, or temozolomide.

In another embodiment, the kit is packaged in a manner that facilitates its use to practice methods of the present disclosure.

The disclosure provides the TG02 citrate salt polymorphic form for use in treating cancer. In one embodiment, the cancer is a solid tumor. In another embodiment, the cancer is a hematological malignancy. In another embodiment, the cancer selected from any one or more of the cancers of Table 2.

**Table 2**

| | | | |
|---|---|---|---|
| adrenal cancer | acinic cell carcinoma | acoustic neuroma | acral lentigious melanoma |
| acrospiroma | acute eosinophilic leukemia | acute erythroid leukemia | acute lymphoblastic leukemia |
| acute megakaryoblastic leukemia | acute monocytic leukemia | acute promyelocytic leukemia | adenocarcinoma |
| adenoid cystic carcinoma | adenoma | adenomatoid odontogenic tumor | adenosquamous carcinoma |
| adipose tissue neoplasm | adrenocortical carcinoma | adult T-cell leukemia/lymphoma | aggressive NK-cell leukemia |
| AIDS-related lymphoma | alveolar rhabdomyosarcoma | alveolar soft part sarcoma | ameloblastic fibroma |
| anaplastic large cell lymphoma | anaplastic thyroid cancer | angioimmunoblastic T-cell lymphoma | angiomyolipoma |
| angiosarcoma | astrocytoma | atypical teratoid rhabdoid tumor | B-cell chronic lymphocytic leukemia |
| B-cell prolymphocytic leukemia | B-cell lymphoma | basal cell carcinoma | biliary tract cancer |
| bladder cancer | blastoma | bone cancer | Brenner tumor |
| Brown tumor | Burkitt's lymphoma | breast cancer | brain cancer |
| carcinoma | carcinoma in situ | carcinosarcoma | cartilage tumor |
| cementoma | myeloid sarcoma | chondroma | chordoma |
| choriocarcinoma | choroid plexus papilloma | clear-cell sarcoma of the kidney | craniopharyngioma |
| cutaneous T-cell lymphoma | cervical cancer | colorectal cancer | Degos disease |
| desmoplastic small round cell tumor | diffuse large B-cell lymphoma | dysembryoplastic neuroepithelial tumor | dysgerminoma |
| embryonal carcinoma | endocrine gland neoplasm | endodermal sinus tumor | enteropathyassociated T-cell lymphoma |
| esophageal cancer | fetus in fetu | fibroma | fibrosarcoma |
| follicular lymphoma | follicular thyroid cancer | ganglioneuroma | gastrointestinal cancer |
| germ cell tumor | gestational choriocarcinoma | giant cell fibroblastoma | giant cell tumor of the bone |
| glial tumor | glioblastoma | glioma | gliomatosis cerebri |
| glucagonoma | gonadoblastoma | granulosa cell tumor | gynandroblastoma |
| gallbladder cancer | gastric cancer | hairy cell leukemia | hemangioblastoma |
| head and neck cancer | hemangiopericytoma | hematological malignancy | hepatoblastoma |
| hepatocellular carcinoma | hepatosplenic T-cell lymphoma | Hodgkin's lymphoma | non-Hodgkin's lymphoma |
| invasive lobular carcinoma | intestinal cancer | kidney cancer | laryngeal cancer |
| lentigo maligna | lethal midline carcinoma | leukemia | leydig cell tumor |
| liposarcoma | lung cancer | lymphangioma | lymphangiosarcoma |
| lymphoepithelioma | lymphoma | acute lymphocytic leukemia | acute myelogeous leukemia |
| chronic lymphocytic leukemia | liver cancer | small cell lung cancer | non-small cell lung cancer |
| MALT lymphoma | malignant fibrous histiocytoma | malignant peripheral nerve sheath tumor | malignant triton tumor |
| mantle cell lymphoma | marginal zone B-cell lymphoma | mast cell leukemia | mediastinal germ cell tumor |
| medullary carcinoma of the breast | medullary thyroid cancer | medulloblastoma | melanoma |
| meningioma | merkel cell cancer | mesothelioma | metastatic urothelial carcinoma |
| mixed Mullerian tumor | mucinous tumor | multiple myeloma | muscle tissue neoplasm |
| mycosis fungoides | myxoid liposarcoma | myxoma | myxosarcoma |
| nasopharyngeal carcinoma | neurinoma | neuroblastoma | neurofibroma |
| neuroma | nodular melanoma | ocular cancer | oligoastrocytoma |
| oligodendroglioma | oncocytoma | optic nerve sheath meningioma | optic nerve tumor |
| oral cancer | osteosarcoma | ovarian cancer | Pancoast tumor |
| papillary thyroid cancer | paraganglioma | pinealoblastoma | pineocytoma |
| pituicytoma | pituitary adenoma | pituitary tumor | plasmacytoma |
| polyembryoma | precursor T-lymphoblastic lymphoma | primary central nervous system lymphoma | primary effusion lymphoma |
| preimary peritoneal cancer | prostate cancer | pancreatic cancer | pharyngeal cancer |
| pseudomyxoma periotonei | renal cell carcinoma | renal medullary carcinoma | retinoblastoma |
| rhabdomyoma | rhabdomyosarcoma | Richter's transformation | rectal cancer |
| sarcoma | Schwannomatosis | seminoma | Sertoli cell tumor |
| sex cord-gonadal stromal tumor | signet ring cell carcinoma | skin cancer | small blue round cell tumors |
| small cell carcinoma | soft tissue sarcoma | somatostatinoma | soot wart |
| spinal tumor | splenic marginal zone lymphoma | squamous cell carcinoma | synovial sarcoma |
| Sezary's disease | small intestine cancer | squamous carcinoma | stomach cancer |
| T-cell lymphoma | testicular cancer | thecoma | thyroid cancer |
| transitional cell carcinoma | throat cancer | urachal cancer | urogenital cancer |
| urothelial carcinoma | uveal melanoma | uterine cancer | verrucous carcinoma |
| visual pathway glioma | vulvar cancer | vaginal cancer | Waldenstrom's macroglobulinemia |
| Warthin's tumor | Wilms' tumor | diffuse pontine glioma | |

In another embodiment, the cancer is selected from the group consisting of squamous cell carcinoma of the head and neck, adenocarcinoma squamous cell carcinoma of the esophagus, adenocarcinoma of the stomach, adenocarcinoma of the colon, hepatocellular carcinoma, cholangiocarcinoma of the biliary system, adenocarcinoma of gall bladder, adenocarcinoma of the pancreas, ductal carcinoma in situ of the breast, adenocarcinoma of the breast, adenocarcinoma of the lungs, squamous cell carcinoma of the lungs, transitional cell carcinoma of the bladder, squamous cell carcinoma of the bladder, squamous cell carcinoma of the cervix, adenocarcinoma of the cervix, endometrial carcinoma, penile squamous cell carcinoma, and squamous cell carcinoma of the skin.

In another embodiment, the cancer is selected from the group consisting of multiple myeloma, hepatocellular carcinoma, glioblastoma, lung cancer, breast cancer, head and neck cancer, prostate cancer, melanoma, colorectal cancer, and diffuse pontine glioma.

In another embodiment, the cancer is diffuse pontine glioma.

In another embodiment, a precancerous tumor is selected from the group consisting of leukoplakia of the head and neck, Barrett's esophagus, metaplasia of the stomach, adenoma of the colon, chronic hepatitis, bile duct hyperplasia, pancreatic intraepithelial neoplasia, atypical adenomatous hyperplasia of the lungs, dysplasia of the bladder, cervical initraepithelial neoplasia, penile intraepithelial neoplasia, and actinic keratosis of the skin.

In another embodiment, the patient has tumors that overexpress MYC, MCL1, or both. The tumors may be determined to overexpress MYC, MCL1, or both, by methods known in the art.

In another embodiment, the cancer is selected from the group consisting of hepatocellular carcinoma, glioblastoma, lung cancer, breast cancer, head and neck cancer, prostate cancer, melanoma, and colorectal cancer.

In another embodiment, the cancer has become resistant to conventional cancer treatments. The term "conventional cancer treatments" as used herein refers to any cancer drugs or biologics or radiation therapy, or combination of cancer drugs and/or biologics and/or radiation therapy that have been tested and/or approved for therapeutic use in humans by the U.S. Food and Drug Administration, European Medicines Agency, or similar regulatory agency.

In another embodiment, the patient has been treated previously with an immune checkpoint inhibitor without TG02. For example, the previous immune checkpoint therapy may be an anti-PD-1 therapy.

In another embodiment, the present disclosure provides therapeutic methods of treating a patient having cancer, the method comprising administering to the patient a therapeutically effective amount of the TG02 citrate polymorphic form, wherein the phenotypic status of the patient is overexpression of MYC, overexpression of MCL1, or overexpression of MYC and MCL1. In another embodiment, the cancer is selected from the group consisting of hepatocellular carcinoma, glioblastoma, lung cancer, breast cancer, head and neck cancer, prostate cancer, melanoma, and colorectal cancer.

In another embodiment, the anti-PD-1 antibody is selected from the group consisting of nivolumab, pembrolizumab, pidilizumab and STI-1110. In another embodiment, the anti-PD-L1 antibody is selected from the group consisting of avelumab, atezolizumab, durvalumab, and STI-1014

In another embodiment, the present disclosure provides personalized medicine for cancer patients, and encompasses the selection of treatment options with the highest likelihood of successful outcome for individual cancer patients. In another aspect, the disclosure relates to the use of an assay(s) to predict the treatment outcome, e.g., the likelihood of favorable responses or treatment success, in patients having cancer.

The present disclosure also provides methods of selecting a patient for treatment of cancer with the TG02 citrate salt polymorphic formand an anti-PD-1 antibody, an anti-PD-L1 antibody, or temozolomide, comprising obtaining a biological sample, e.g., blood cells, from the patient, testing a biological sample from the patient for the presence of a biomarker, e.g., overexpression of MYC, overexpression of MCL1, or both, and selecting the patient for treatment if the biological sample contains that biomarker. In another embodiment, the methods further comprise administering a therapeutically effective amount of the TG02 citrate salt polymorphic form and an anti-PD-1 antibody, an anti-PD-L1 antibody, or temozolomide, if the biological sample contains the biomarker. Examples of cancer biomarkers are provided in Table 1. In another embodiment, the cancer is a solid tumor. In another embodiment, the cancer is a hematological malignancy. In another embodiment, the cancer is selected from the group consisting of hepatocellular carcinoma, glioblastoma, lung cancer, breast cancer, head and neck cancer, prostate cancer, melanoma, and colorectal cancer.

In another embodiment, the present disclosure provides methods of predicting treatment outcomes in a patient having cancer, comprising obtaining a biological sample, from the patient, testing the biological sample from the patient for the presence of a biomarker, e.g., overexpression of MYC, overexpression of MCL1, or both, wherein the detection of the biomarker indicates the patient will respond favorably to administration of a therapeutically effective amount of the TG02 citrate salt polymorphic form and an anti-PD-1 antibody, an anti-PD-L1 antibody, or temozolomide. Favorable responses include, but are not limited to, a decrease in tumor size and an increase in progressionfree or overall survival.

In another embodiment, the patient is selected for treatment with the TG02 citrate salt polymorphic form and an anti-PD-1 antibody, an anti-PD-L1 antibody, or temozolomide, after the patient's cells have been determined to contain an overexpression of MYC. In another embodiment, the patient is selected for treatment after the patient's cells have been determined to contain an overexpression of MCL1. In another embodiment, the patient is selected for treatment after the patient's cells have been determined to contain an overexpression of MYC and an overexpression of MCL1.

In another embodiment, the method of treating a patient having cancer comprises obtaining a biological sample from the patient, determining whether the biological sample contains a biomarker, *e.g.,* overexpression of MYC, overexpression of MCL1, or both, and administering to the patient a therapeutically effective amount of the TG02 citrate salt polymorphic form and an anti-PD-1 antibody, an anti-PD-L1 antibody, or temozolomide, if the biological sample contains the biomarker. In another embodiment, the methods provided herein comprise determining whether the patient's cells contain an overexpression of MYC. In another embodiment, the methods provided herein comprise determining whether the patient's cells contain an overexpression of MCL1. In another embodiment, the methods provided herein comprise determining whether the patient's cells contain an overexpression of MYC and MCL1.

In another embodiment, the disclosure provides a method of treating a subject having cancer, the method comprising obtaining a biological sample from the subject, determining the expression level of MYC, MCL1, or both in the biological sample; and administering a therapeutically effective amount of the TG02 citrate salt polymorphic form and an anti-PD-1 antibody, an anti-PD-L1 antibody, or temozolomide, to the subject if the biological sample shows overexpression of MYC, MCL1, or both.

### I. Optional Therapeutic agents

In some therapeutic methods of the disclosure, a further therapeutic agent is administered to a cancer patient in combination with the TG02 citrate salt polymorphic form and the anti-PD-1 antibody, anti-PD-L1 antibody, or temozolomide.

In some therapeutic methods of the disclosure, a third therapeutic agent are administered to a cancer patient in combination with the TG02 citrate salt polymorphic form.

In some therapeutic methods of the disclosure, a third therapeutic agent and a fourth therapeutic agent are administered to a cancer patient in combination with the TG02 citrate salt polymorphic form.

The third, and fourth therapeutic agents used in the therapeutic methods of the present disclosure are referred to as "optional therapeutic agents." Such optional therapeutic agents useful in the treatment of cancer patients are known in the art. In one embodiment, the optional therapeutic agent combined with the TG02 citrate saltpolymorphic form is an anticancer agent. Optional therapeutic agents include, but are not limited to, daunorubicin, doxorubicin, epirubicin, idarubicin, valrubicin, cisplatin, bortezomib, carfilzomib, lenalidomide, sorafenib, regorafenib, radiotherapy, immune checkpoint inhibitors.

Optional therapeutic agents are administered in an amount to provide their desired therapeutic effect. The effective dosage range for each optional therapeutic agent is known in the art, and the optional therapeutic agent is administered to an individual in need thereof within such established ranges.

The TG02 citrate salt polymorphic form, the anti-PD-1 antibody, anti-PD-L1 antibody, or temozolomide and the optional therapeutic agent can be administered together as a single-unit dose or separately as multi-unit doses, and in any orderOne or more doses of the TG02 citrate salt polymorphic form and the additionallytherapeutic agent can be administered to the patient.

Immune checkpoint inhibitors are therapies that blockade immune system inhibitor checkpoints. In some therapeutic methods of the disclosure, an immune checkpoint inhibitor is administered to a cancer patient in combination with the TG02 citrate salt polymorphic form.

Immune checkpoints can be stimulatory or inhibitory. Blockade of inhibitory immune checkpoint activates immune system function and can be used for cancer immunotherapy. Pardoll, Nature Reviews. Cancer 12:252-64 (2012). Tumor cells turn off activated T cells when they attach to specific T-cell receptors. Immune checkpoint inhibitors prevent tumor cells from attaching to T cells, which results in T cells remaining activated. In effect, the coordinated action by cellular and soluble components combats pathogens and injuries by cancers. The modulation of immune system pathways may involve changing the expression or the functional activity of at least one component of the pathway to then modulate the response by the immune system. U.S. 2015/0250853. Examples of immune checkpoint inhibitors include PD-1 inhibitors, PD-L1 inhibitors, CTLA-4 inhibitors, LAG3 inhibitors, TIM3 inhibitors, cd47 inhibitors, and B7-H1 inhibitors. Thus, in one embodiment, the immune checkpoint inhibitor is selected from the group consisting of a PD-1 inhibitor, a PD-L1 inhibitor, a CTLA-4 inhibitor, a LAG3 inhibitor, a TIM3 inhibitor, and a cd47 inhibitor.

In another embodiment, the immune checkpoint inhibitor is a programmed cell death (PD-1) inhibitor. PD-1 is a T-cell co inhibitory receptor that plays a pivotal role in the ability of tumor cells to evade the host's immune system. Blockage of interactions between PD-1 and PD-L1, a ligand of PD-1, enhances immune function and mediates antitumor activity. Examples of PD-1 inhibitors include antibodies that specifically bind to PD-1. Particular anti-PD-1 antibodies include, but are not limited to nivolumab, pembrolizumab, STI-1014, and pidilzumab. For a general discussion of the availability, methods of production, mechanism of action, and clinical studies of anti-PD-1 antibodies, see U.S. 2013/0309250, U.S. 6,808,710, U.S. 7,595,048, U.S. 8,008,449, U.S. 8,728,474, U.S. 8,779,105, U.S. 8,952,136, U.S. 8,900,587, U.S. 9,073,994, U.S. 9,084,776, and Naido et al., British Journal of Cancer 111:2214-19 (2014).

In another embodiment, the immune checkpoint inhibitor is a PD-L1 (also known as B7-H1 or CD274) inhibitor. Examples of PD-L1 inhibitors include antibodies that specifically bind to PD-L1. Particular anti-PD-L1 antibodies include, but are not limited to, avelumab, atezolizumab, durvalumab, and BMS-936559. For a general discussion of the availability, methods of production, mechanism of action, and clinical studies, see U.S. 8,217,149, U.S. 2014/0341917, U.S. 2013/0071403, WO 2015036499, and Naido et al., British Journal of Cancer 111:2214-19 (2014).

In another embodiment, the immune checkpoint inhibitor is a CTLA-4 inhibitor. CTLA-4, also known as cytotoxic T-lymphocyte antigen 4, is a protein receptor that downregulates the immune system. CTLA-4 is characterized as a "brake" that binds costimulatory molecules on antigen-presenting cells, which prevents interaction with CD28 on T cells and also generates an overtly inhibitory signal that constrains T cell activation. Examples of CTLA-4 inhibitors include antibodies that specifically bind to CTLA-4. Particular anti-CTLA-4 antibodies include, but are not limited to, ipilimumab and tremelimumab. For a general discussion of the availability, methods of production, mechanism of action, and clinical studies, see U.S. 6,984,720, U.S. 6,207,156, and Naido et al., British Journal of Cancer 111:2214-19 (2014).

In another embodiment, the immune checkpoint inhibitor is a LAG3 inhibitor. LAG3, Lymphocyte Activation Gene 3, is a negative co-simulatory receptor that modulates T cell homeostatis, proliferation, and activation. In addition, LAG3 has been reported to participate in regulatory T cells (Tregs) suppressive function. A large proportion of LAG3 molecules are retained in the cell close to the microtubule-organizing center, and only induced following antigen specific T cell activation. U.S. 2014/0286935. Examples of LAG3 inhibitors include antibodies that specifically bind to LAG3. Particular anti-LAG3 antibodies include, but are not limited to, GSK2831781. For a general discussion of the availability, methods of production, mechanism of action, and studies, see, U.S. 2011/0150892, U.S. 2014/0093511, U.S. 20150259420, and Huang et al., Immunity 21:503-13 (2004).

In another embodiment, the immune checkpoint inhibitor is a TIM3 inhibitor. TIM3, T-cell immunoglobulin and mucin domain 3, is an immune checkpoint receptor that functions to limit the duration and magnitude of T_{H}1 and T_{C}1 T-cell responses. The TIM3 pathway is considered a target for anticancer immunotherapy due to its expression on dysfunctional CD8⁺ T cells and Tregs, which are two reported immune cell populations that constitute immunosuppression in tumor tissue. Anderson, *Cancer Immunology Research 2*:393-98 (2014). Examples of TIM3 inhibitors include antibodies that specifically bind to TIM3. For a general discussion of the availability, methods of production, mechanism of action, and studies of TIM3 inhibitors, see U.S. 20150225457, U.S. 20130022623, U.S. 8,522,156, Ngiow et al., Cancer Res 71: 6567-71 (2011), Ngiow, et al., Cancer Res 71:3540-51 (2011), and Anderson, Cancer Immunology Res 2:393-98 (2014).

In another embodiment, the immune checkpoint inhibitor is a cd47 inhibitor. *See* Unanue, E.R., PNAS 110:10886-87 (2013).

The term "antibody" is meant to include intact monoclonal antibodies, polyclonal antibodies, multispecific antibodies formed from at least two intact antibodies, and antibody fragments, so long as they exhibit the desired biological activity. In another embodiment, "antibody" is meant to include soluble receptors that do not possess the Fc portion of the antibody. In one embodiment, the antibodies are humanized monoclonal antibodies and fragments thereof made by means of recombinant genetic engineering.

Another class of immune checkpoint inhibitors include polypeptides that bind to and block PD-1 receptors on T-cells without triggering inhibitor signal transduction. Such peptides include B7-DC polypeptides, B7-H1 polypeptides, B7-1 polypeptides and B7-2 polypeptides, and soluble fragments thereof, as disclosed in U.S. Pat. 8,114,845.

Another class of immune checkpoint inhibitors include compounds with peptide moieties that inhibit PD-1 signaling. Examples of such compounds are disclosed in U.S. Pat. 8,907,053 and have the structure: 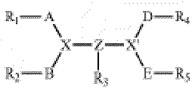 or a pharmaceutically acceptable salt thereof, wherein the compound comprises at least 5 amino acids useful as therapeutic agents capable of inhibiting the PD-1 signaling pathway.

Another class of immune checkpoint inhibitors include inhibitors of certain metabolic enzymes, such as indoleamine 2,3 dioxygenase (IDO), which is expressed by infiltrating myeloid cells and tumor cells. The IDO enzyme inhibits immune responses by depleting amino acids that are necessary for anabolic functions in T cells or through the synthesis of particular natural ligands for cytosolic receptors that are able to alter lymphocyte functions. Pardoll, Nature Reviews. Cancer 12:252-64 (2012); Löb, Cancer Immunol Immunother 58:153-57 (2009). Particular IDO blocking agents include, but are not limited to levo-1-methyl typtophan (L-1MT) and 1-methyl-tryptophan (1MT). Qian et al., Cancer Res 69:5498-504 (2009); and Löb et al., Cancer Immunol Immunother 58:153-7 (2009).

In another embodiment, the immune checkpoint inhibitor is nivolumab, pembrolizumab, pidilizumab, STI-1110, avelumab, atezolizumab, durvalumab, STI-1014, ipilimumab, tremelimumab, GSK2831781, BMS-936559 or MED14736.

In another embodiment, the optional therapeutic agent is an epigenetic drug. As used herein, the term "epigenetic drug" refers to a therapeutic agent that targets an epigenetic regulator. Examples of epigenetic regulators include the histone lysine methyltransferases, histone arginine methyl transferases, histone demethylases, histone deacetylases, histone acetylases, and DNA methyltransferases. Histone deacetylase inhibitors include, but are not limited to, vorinostat.

In another embodiment, the optional therapeutic agent is a chemotherapeutic agent or other anti-proliferative agent that can be administered in combination with the TG02 citrate salt polymorphic form and anti-PD-1 antibody, anti-PD-L1 antibody, or temozolomide to treat cancer. Examples of therapies and anticancer agents that can be used in combination with the TG02 citrate salt polymorphic form and anti-PD-1 antibody, anti-PD-L1 antibody, or temozolomide include surgery, radiotherapy (e.g., gammaradiation, neutron beam radiotherapy, electron beam radiotherapy, proton therapy, brachytherapy, and systemic radioactive isotopes), endocrine therapy, a biologic response modifier (e.g., an interferon, an interleukin, tumor necrosis factor (TNF), hyperthermia and cryotherapy, an agent to attenuate any adverse effect (e.g., an antiemetic), and any other approved chemotherapeutic drug.

Nonlimiting exemplary antiproliferative compounds include an aromatase inhibitor; an anti-estrogen; an anti-androgen; a gonadorelin agonist; a topoisomerase I inhibitor; a topoisomerase II inhibitor; a microtubule active agent; an alkylating agent, e.g., temozolomide; a retinoid, a carontenoid, or a tocopherol; a cyclooxygenase inhibitor; an MMP inhibitor; an mTOR inhibitor; an antimetabolite; a platin compound; a methionine aminopeptidase inhibitor; a bisphosphonate; an antiproliferative antibody; a heparanase inhibitor; an inhibitor of Ras oncogenic isoforms; a telomerase inhibitor; a proteasome inhibitor; a compound used in the treatment of hematologic malignancies; a Flt-3 inhibitor; an Hsp90 inhibitor; a kinesin spindle protein inhibitor; a MEK inhibitor; an antitumor antibiotic; a nitrosourea; a compound targeting/decreasing protein or lipid kinase activity, a compound targeting/decreasing protein or lipid phosphatase activity, or any further anti-angiogenic compound.

Nonlimiting exemplary aromatase inhibitors include steroids, such as atamestane, exemestane, and formestane, and non-steroids, such as aminoglutethimide, roglethimide, pyridoglutethimide, trilostane, testolactone, ketokonazole, vorozole, fadrozole, anastrozole, and letrozole.

Nonlimiting anti-estrogens include tamoxifen, fulvestrant, raloxifene, and raloxifene hydrochloride. Anti-androgens include, but are not limited to, bicalutamide. Gonadorelin agonists include, but are not limited to, abarelix, goserelin, and goserelin acetate.

Nonlimiting exemplary topoisomerase I inhibitors include topotecan, gimatecan, irinotecan, camptothecin and its analogues, 9-nitrocamptothecin, and the macromolecular camptothecin conjugate PNU-166148. Topoisomerase II inhibitors include, but are not limited to, anthracyclines, such as doxorubicin, daunorubicin, epirubicin, idarubicin, and nemorubicin; anthraquinones, such as mitoxantrone and losoxantrone; and podophillotoxines, such as etoposide and teniposide.

Microtubule active agents include microtubule stabilizing, microtubule destabilizing compounds, and microtubulin polymerization inhibitors including, but not limited to, taxanes, such as paclitaxel and docetaxel; vinca alkaloids, such as vinblastine, vinblastine sulfate, vincristine, and vincristine sulfate, and vinorelbine; discodermolides; cochicine and epothilones and derivatives thereof.

Nonlimiting exemplary alkylating agents include cyclophosphamide, ifosfamide, melphalan, and nitrosoureas, such as carmustine and lomustine.

Nonlimiting exemplary matrix metalloproteinase inhibitors ("MMP inhibitors") include collagen peptidomimetic and nonpeptidomimetic inhibitors, tetracycline derivatives, batimastat, marimastat, prinomastat, metastat, BMS-279251, BAY 12-9566, TAA211, MMI270B, and AAJ996.

Nonlimiting exemplary mTOR inhibitors include compounds that inhibit the mammalian target of rapamycin (mTOR) and possess antiproliferative activity such as sirolimus, everolimus, CCI-779, and ABT578.

Nonlimiting exemplary antimetabolites include 5-fluorouracil (5-FU), capecitabine, gemcitabine, DNA demethylating compounds, such as 5-azacytidine and decitabine, methotrexate and edatrexate, and folic acid antagonists, such as pemetrexed.

Nonlimiting exemplary platin compounds include carboplatin, cis-platin, cisplatinum, and oxaliplatin.

Nonlimiting exemplary methionine aminopeptidase inhibitors include bengamide or a derivative thereof and PPI-2458.

Nonlimiting exemplary bisphosphonates include etridonic acid, clodronic acid, tiludronic acid, pamidronic acid, alendronic acid, ibandronic acid, risedronic acid, and zoledronic acid.

Nonlimiting exemplary heparanase inhibitors include compounds that target, decrease, or inhibit heparin sulfate degradation, such as PI-88 and OGT2115.

Nonlimiting exemplary compounds which target, decrease, or inhibit the oncogenic activity of Ras include farnesyl transferase inhibitors, such as L-744832, DK8G557, tipifamib, and lonafamib.

Nonlimiting exemplary telomerase inhibitors include compounds that target, decrease, or inhibit the activity of telomerase, such as compounds that inhibit the telomerase receptor, such as telomestatin.

Nonlimiting exemplary proteasome inhibitors include compounds that target, decrease, or inhibit the activity of the proteasome including, but not limited to, bortezomib. In some embodiments, the proteasome inhibitor is carfilzomib.

Nonlimiting exemplary FMS-like tyrosine kinase inhibitors, which are compounds targeting, decreasing or inhibiting the activity of FMS-like tyrosine kinase receptors (Flt-3R) include interferon, I-β-D-arabinofuransylcytosine (ara-c), and bisulfan; and ALK inhibitors, which are compounds which target, decrease, or inhibit anaplastic lymphoma kinase.

Nonlimiting exemplary Flt-3 inhibitors include PKC412, midostaurin, a staurosporine derivative, SU11248, and MLN518.

Nonlimiting exemplary HSP90 inhibitors include compounds targeting, decreasing, or inhibiting the intrinsic ATPase activity of HSP90; or degrading, targeting, decreasing or inhibiting the HSP90 client proteins via the ubiquitin proteosome pathway. Compounds targeting, decreasing or inhibiting the intrinsic ATPase activity of HSP90 are especially compounds, proteins, or antibodies that inhibit the ATPase activity of HSP90, such as 17-allylamino,17-demethoxygeldanamycin (17AAG), a geldanamycin derivative; other geldanamycin related compounds; radicicol and HDAC inhibitors.

Nonlimiting exemplary protein tyrosine kinase and/or serine and/or threonine kinase inhibitors or lipid kinase inhibitors, include a) a compound targeting, decreasing, or inhibiting the activity of the platelet-derived growth factor-receptors (PDGFR), such as a compound that targets, decreases, or inhibits the activity of PDGFR, such as an N-phenyl-2-pyrimidine-amine derivatives, such as imatinib, SUlOl, SU6668, and GFB-111; b) a compound targeting, decreasing, or inhibiting the activity of the fibroblast growth factor-receptors (FGFR); c) a compound targeting, decreasing, or inhibiting the activity of the insulin-like growth factor receptor I (IGF-IR), such as a compound that targets, decreases, or inhibits the activity of IGF-IR; d) a compound targeting, decreasing, or inhibiting the activity of the Trk receptor tyrosine kinase family, or ephrin B4 inhibitors; e) a compound targeting, decreasing, or inhibiting the activity of the Axl receptor tyrosine kinase family; f) a compound targeting, decreasing, or inhibiting the activity of the Ret receptor tyrosine kinase; g) a compound targeting, decreasing, or inhibiting the activity of the Kit/SCFR receptor tyrosine kinase, such as imatinib; h) a compound targeting, decreasing, or inhibiting the activity of the c-Kit receptor tyrosine kinases, such as imatinib; i) a compound targeting, decreasing, or inhibiting the activity of members of the c-Abl family, their gene-fusion products (e.g. Bcr-Abl kinase) and mutants, such as an N-phenyl-2-pyrimidine-amine derivative, such as imatinib or nilotinib; PD180970; AG957; NSC 680410; PD173955; or dasatinib; j) a compound targeting, decreasing, or inhibiting the activity of members of the protein kinase C (PKC) and Raf family of serine/threonine kinases, members of the MEK, SRC, JAK, FAK, PDK1, PKB/Akt, and Ras/MAPK family members, and/or members of the cyclin-dependent kinase family (CDK), such as a staurosporine derivative disclosed in U.S. Patent No. 5,093,330, such as midostaurin; examples of further compounds include UCN-01, safingol, BAY 43-9006, bryostatin 1, perifosine; ilmofosine; RO 318220 and RO 320432; GO 6976; Isis 3521 ; LY333531/LY379196; a isochinoline compound; a farnesyl transferase inhibitor; PD184352 or QAN697, or AT7519; k) a compound targeting, decreasing or inhibiting the activity of a protein-tyrosine kinase, such as imatinib mesylate or a tyrphostin, such as Tyrphostin A23/RG-50810; AG 99; Tyrphostin AG 213; Tyrphostin AG 1748; Tyrphostin AG 490; Tyrphostin B44; Tyrphostin B44 (+) enantiomer; Tyrphostin AG 555; AG 494; Tyrphostin AG 556, AG957 and adaphostin (4-{[(2,5-dihydroxyphenyl)methyl]amino}-benzoic acid adamantyl ester; NSC 680410, adaphostin); 1) a compound targeting, decreasing, or inhibiting the activity of the epidermal growth factor family of receptor tyrosine kinases (EGFR, ErbB2, ErbB3, ErbB4 as homo- or heterodimers) and their mutants, such as CP 358774, ZD 1839, ZM 105180; trastuzumab, cetuximab, gefitinib, erlotinib, OSI-774, Cl-1033, EKB-569, GW-2016, antibodies El.l, E2.4, E2.5, E6.2, E6.4, E2.11, E6.3 and E7.6.3, and 7H-pyrrolo-[2,3-d]pyrimidine derivatives; and m) a compound targeting, decreasing, or inhibiting the activity of the c-Met receptor.

Nonlimiting exemplary compounds that target, decrease, or inhibit the activity of a protein or lipid phosphatase include inhibitors of phosphatase 1, phosphatase 2A, or CDC25, such as okadaic acid or a derivative thereof.

Further anti-angiogenic compounds include compounds having another mechanism for their activity unrelated to protein or lipid kinase inhibition, e.g., thalidomide and TNP-470.

Additional, nonlimiting, exemplary chemotherapeutic compounds, one or more of which may be used in combination with the claimed invention, include: avastin, daunorubicin, adriamycin, Ara-C, VP-16, teniposide, mitoxantrone, idarubicin, carboplatinum, PKC412, 6-mercaptopurine (6-MP), fludarabine phosphate, octreotide, SOM230, FTY720, 6-thioguanine, cladribine, 6-mercaptopurine, pentostatin, hydroxyurea, 2-hydroxy-lH-isoindole-l,3-dione derivatives, l-(4-chloroanilino)-4-(4-pyridylmethyl)phthalazine or a pharmaceutically acceptable salt thereof, 1-(4-chloroanilino)-4-(4-pyridylmethyl)phthalazine succinate, angiostatin, endostatin, anthranilic acid amides, ZD4190, ZD6474, SU5416, SU6668, bevacizumab, rhuMAb, rhuFab, macugon; FLT-4 inhibitors, FLT-3 inhibitors, VEGFR-2 IgGI antibody, RPI 4610, bevacizumab, porfimer sodium, anecortave, triamcinolone, hydrocortisone, 11-a-epihydrocotisol, cortex olone, 17a-hydroxyprogesterone, corticosterone, desoxycorticosterone, testosterone, estrone, dexamethasone, fluocinolone, a plant alkaloid, a hormonal compound and/or antagonist, a biological response modifier, such as a lymphokine or interferon, an antisense oligonucleotide or oligonucleotide derivative, shRNA, and siRNA.

A number of suitable optional therapeutic, e.g., anticancer, agents are contemplated for use in the therapeutic methods provided herein. Indeed, the methods provided herein can include, but are not limited to, administration of numerous optional therapeutic agents such as: agents that induce apoptosis; polynucleotides (*e.g.,* anti-sense, ribozymes, siRNA); polypeptides (*e.g.,* enzymes and antibodies); biological mimetics (*e.g.,* gossypol or BH3 mimetics); agents that bind (*e.g.,* oligomerize or complex) with a Bcl-2 family protein such as Bax; alkaloids; alkylating agents; antitumor antibiotics; antimetabolites; hormones; platinum compounds; monoclonal or polyclonal antibodies (*e.g.,* antibodies conjugated with anticancer drugs, toxins, defensins), toxins; radionuclides; biological response modifiers (*e.g.,* interferons (*e.g.,* IFN-α) and interleukins (*e.g.,* IL-2)); adoptive immunotherapy agents; hematopoietic growth factors; agents that induce tumor cell differentiation (*e.g.,* all-trans-retinoic acid); gene therapy reagents (*e.g.,* antisense therapy reagents and nucleotides); tumor vaccines; angiogenesis inhibitors; proteosome inhibitors: NF-KB modulators; anti-CDK compounds; HDAC inhibitors; and the like. Numerous other examples of optional therapeutic agents such as chemotherapeutic compounds and anticancer therapies suitable for co-administration with the disclosed compounds are known to those skilled in the art.

In certain embodiments, anticancer agents comprise agents that induce or stimulate apoptosis. Agents that induce or stimulate apoptosis include, for example, agents that interact with or modify DNA, such as by intercalating, cross-linking, alkylating, or otherwise damaging or chemically modifying DNA. Agents that induce apoptosis include, but are not limited to, radiation (*e.g.,* X-rays, gamma rays, UV); tumor necrosis factor (TNF)-related factors (*e.g.,* TNF family receptor proteins, TNF family ligands, TRAIL, antibodies to TRAIL-R1 or TRAIL-R2); kinase inhibitors (*e.g.,* epidermal growth factor receptor (EGFR) kinase inhibitor. Additional anticancer agents include: vascular growth factor receptor (VGFR) kinase inhibitor, fibroblast growth factor receptor (FGFR) kinase inhibitor, platelet-derived growth factor receptor (PDGFR) kinase inhibitor, and Bcr-Abl kinase inhibitors (such as GLEEVEC)); antisense molecules; antibodies (*e.g.,* HERCEPTIN, RITUXAN, ZEVALIN, and AVASTIN); anti-estrogens *(*e*.g.,* raloxifene and tamoxifen); anti-androgens *(e.g.,* flutamide, bicalutamide, finasteride, aminoglutethamide, ketoconazole, and corticosteroids); cyclooxygenase 2 (COX-2) inhibitors (*e.g.,* celecoxib, meloxicam, NS-398, and non-steroidal anti-inflammatory drugs (NSAIDs)); anti-inflammatory drugs (*e.g.,* butazolidin, DECADRON, DELTASONE, dexamethasone, dexamethasone intensol, DEXONE, HEXADROL, hydroxychloroquine, METICORTEN, ORADEXON, ORASONE, oxyphenbutazone, PEDIAPRED, phenylbutazone, PLAQUENIL, prednisolone, prednisone, PRELONE, and TANDEARIL); and cancer chemotherapeutic drugs (*e.g.,* irinotecan (CAMPTOSAR), CPT-11, fludarabine (FLUDARA), dacarbazine (DTIC), dexamethasone, mitoxantrone, MYLOTARG, VP-16, cisplatin, carboplatin, oxaliplatin, 5-FU, doxorubicin, gemcitabine, bortezomib, gefitinib, bevacizumab, TAXOTERE or TAXOL); cellular signaling molecules; ceramides and cytokines; staurosporine, and the like.

In still other embodiments, the therapeutic methods provided herein include administering to a cancer patient a therapeutically effective amount of the TG02 citrate salt polymorphic form, anti-PD-1 antibody, anti-PD-L1 antibody, or temozolomide and at least one additional anti-hyperproliferative or antineoplastic agent selected from alkylating agents, antimetabolites, and natural products (*e.g.,* herbs and other plant and/or animal derived compounds).

Alkylating agents suitable for use in the present methods include, but are not limited to: 1) nitrogen mustards (*e.g.,* mechlorethamine, cyclophosphamide, ifosfamide, melphalan (L-sarcolysin); and chlorambucil); 2) ethylenimines and methylmelamines (*e.g.,* hexamethylmelamine and thiotepa); 3) alkyl sulfonates *(*e.g., busulfan); 4) nitrosoureas (*e.g.,* carmustine (BCNU); lomustine (CCNU); semustine (methyl-CCNU); and streptozocin (streptozotocin)); and 5) triazenes (*e.g.,* dacarbazine (DTIC; dimethyltriazenoimid-azolecarboxamide).

In some embodiments, antimetabolites suitable for use in the present methods include, but are not limited to: 1) folic acid analogs (*e.g.,* methotrexate (amethopterin)); 2) pyrimidine analogs (*e.g.,* fluorouracil (5-fluorouracil; 5-FU), floxuridine (fluorodeoxyuridine; FudR), and cytarabine (cytosine arabinoside)); and 3) purine analogs (*e.g.,* mercaptopurine (6-mercaptopurine; 6-MP), thioguanine (6-thioguanine; TG), and pentostatin (2'-deoxycoformycin)).

In still further embodiments, chemotherapeutic agents suitable for use in the methods of the present disclosure include, but are not limited to: 1) vinca alkaloids (*e.g.,* vinblastine (VLB), vincristine); 2) epipodophyllotoxins (*e.g.,* etoposide and teniposide); 3) antibiotics (*e.g.,* dactinomycin (actinomycin D), daunorubicin (daunomycin; rubidomycin), doxorubicin, bleomycin, plicamycin (mithramycin), and mitomycin (mitomycin C)); 4) enzymes (*e.g.,* L-asparaginase); 5) biological response modifiers (*e.g.,* interferon-alfa); 6) platinum coordinating complexes (*e.g.,* cisplatin (cis-DDP) and carboplatin); 7) anthracenediones (*e.g.,* mitoxantrone); 8) substituted ureas (*e.g.,* hydroxyurea); 9) methylhydrazine derivatives (*e.g.,* procarbazine (N-methylhydrazine; MIH)); 10) adrenocortical suppressants (*e.g.,* mitotane (o,p'-DDD) and aminoglutethimide); 11) adrenocorticosteroids (*e.g.,* prednisone); 12) progestins (*e.g.,* hydroxyprogesterone caproate, medroxyprogesterone acetate, and megestrol acetate); 13) estrogens (*e.g.,* diethylstilbestrol and ethinyl estradiol); 14) antiestrogens (*e.g.,* tamoxifen); 15) androgens (*e.g.,* testosterone propionate and fluoxymesterone); 16) antiandrogens (*e.g.,* flutamide): and 17) gonadotropin-releasing hormone analogs (*e.g.,* leuprolide).

Any oncolytic agent that is routinely used in a cancer therapy context finds use in the therapeutic methods of the present disclosure. For example, the U.S. Food and Drug Administration (FDA) maintains a formulary of oncolytic agents approved for use in the United States. International counterpart agencies to the FDA maintain similar formularies. Those skilled in the art will appreciate that the "product labels" required on all U.S. approved chemotherapeutics describe approved indications, dosing information, toxicity data, and the like, for the exemplary agents.

Anticancer agents further include compounds which have been identified to have anticancer activity. Examples include, but are not limited to, 3-AP, 12-O-tetradecanoylphorbol-13-acetate, 17AAG, 852A, ABI-007, ABR-217620, ABT-751, ADI-PEG 20, AE-941, AG-013736, AGRO100, alanosine, AMG 706, antibody G250, antineoplastons, AP23573, apaziquone, APC8015, atiprimod, ATN-161, atrasenten, azacitidine, BB-10901, BCX-1777, bevacizumab, BG00001, bicalutamide, BMS 247550, bortezomib, bryostatin-1, buserelin, calcitriol, CCI-779, CDB-2914, cefixime, cetuximab, CG0070, cilengitide, clofarabine, combretastatin A4 phosphate, CP-675,206, CP-724,714, CpG 7909, curcumin, decitabine, DENSPM, doxercalciferol, E7070, E7389, ecteinascidin 743, efaproxiral, eflomithine, EKB-569, enzastaurin, erlotinib, exisulind, fenretinide, flavopiridol, fludarabine, flutamide, fotemustine, FR901228, G17DT, galiximab, gefitinib, genistein, glufosfamide, GTI-2040, histrelin, HKI-272, homoharringtonine, HSPPC-96, hu14.18-interleukin-2 fusion protein, HuMax-CD4, iloprost, imiquimod, infliximab, interleukin-12, IPI-504, irofulven, ixabepilone, lapatinib, lenalidomide, lestaurtinib, leuprolide, LMB-9 immunotoxin, lonafarnib, luniliximab, mafosfamide, MB07133, MDX-010, MLN2704, monoclonal antibody 3F8, monoclonal antibody J591, motexafin, MS-275, MVA-MUC1-IL2, nilutamide, nitrocamptothecin, nolatrexed dihydrochloride, nolvadex, NS-9, O6-benzylguanine, oblimersen sodium, ONYX-015, oregovomab, OSI-774, panitumumab, paraplatin, PD-0325901, pemetrexed, PHY906, pioglitazone, pirfenidone, pixantrone, PS-341, PSC 833, PXD101, pyrazoloacridine, R115777, RAD001, ranpirnase, rebeccamycin analogue, rhuAngiostatin protein, rhuMab 2C4, rosiglitazone, rubitecan, S-1, S-8184, satraplatin, SB-715992, SGN-0010, SGN-40, sorafenib, regorafenib, SR31747A, ST1571, SU011248, suberoylanilide hydroxamic acid, suramin, talabostat, talampanel, tariquidar, temsirolimus, TGFa-PE38 immunotoxin, thalidomide, thymalfasin, tipifamib, tirapazamine, TLK286, trabectedin, trimetrexate glucuronate, TroVax, UCN-1, valproic acid, vinflunine, VNP40101M, volociximab, vorinostat, VX-680, ZD1839, ZD6474, zileuton, and zosuquidar trihydrochloride.

For a more detailed description of anticancer agents and other optional therapeutic agents, those skilled in the art are referred to any number of instructive manuals including, but not limited to, the Physician's Desk Reference and to Goodman and Gilman's "Pharmaceutical Basis of Therapeutics" tenth edition, Eds. Hardman *et al.,* 2002.

In some embodiments, methods provided herein comprise administering a the TG02 citrate salt polymorphic form and anti-PD-1 antibody, anti-PD-L1 antibody, or temozolomide to a cancer patient in combination with radiation therapy. The methods provided herein are not limited by the types, amounts, or delivery and administration systems used to deliver the therapeutic dose of radiation to a patient. For example, the patient may receive photon radiotherapy, particle beam radiation therapy, other types of radiotherapies, and combinations thereof. In some embodiments, the radiation is delivered to the patient using a linear accelerator. In still other embodiments, the radiation is delivered using a gamma knife.

The source of radiation can be external or internal to the patient. External radiation therapy is most common and involves directing a beam of high-energy radiation to a tumor site through the skin using, for instance, a linear accelerator. While the beam of radiation is localized to the tumor site, it is nearly impossible to avoid exposure of normal, healthy tissue. However, external radiation is usually well tolerated by patients. Internal radiation therapy involves implanting a radiation-emitting source, such as beads, wires, pellets, capsules, particles, and the like, inside the body at or near the tumor site including the use of delivery systems that specifically target cancer cells (*e.g.,* using particles attached to cancer cell binding ligands). Such implants can be removed following treatment, or left in the body inactive. Types of internal radiation therapy include, but are not limited to, brachytherapy, interstitial irradiation, intracavity irradiation, radioimmunotherapy, and the like.

The patient may optionally receive radiosensitizers (*e.g.,* metronidazole, misonidazole, intra-arterial Budr, intravenous iododeoxyuridine (IudR), nitroimidazole, 5-substituted-4-nitroimidazoles, 2H-isoindolediones, [[(2-bromoethyl)-amino]methyl]-nitro-1H-imidazole-1-ethanol, nitroaniline derivatives, DNA-affinic hypoxia selective cytotoxins, halogenated DNA ligand, 1,2,4 benzotriazine oxides, 2-nitroimidazole derivatives, fluorine-containing nitroazole derivatives, benzamide, nicotinamide, acridineintercalator, 5-thiotretrazole derivative, 3-nitro-1,2,4-triazole, 4,5-dinitroimidazole derivative, hydroxylated texaphrins, cisplatin, mitomycin, tiripazamine, nitrosourea, mercaptopurine, methotrexate, fluorouracil, bleomycin, vincristine, carboplatin, epirubicin, doxorubicin, cyclophosphamide, vindesine, etoposide, paclitaxel, heat (hyperthermia), and the like), radioprotectors (*e.g.,* cysteamine, aminoalkyl dihydrogen phosphorothioates, amifostine (WR 2721), IL-1, IL-6, and the like). Radiosensitizers enhance the killing of tumor cells. Radioprotectors protect healthy tissue from the harmful effects of radiation.

Any type of radiation can be administered to an patient, so long as the dose of radiation is tolerated by the patient without unacceptable negative side-effects. Suitable types of radiotherapy include, for example, ionizing (electromagnetic) radiotherapy (*e.g.,* X-rays or gamma rays) or particle beam radiation therapy (*e.g.,* high linear energy radiation). Ionizing radiation is defined as radiation comprising particles or photons that have sufficient energy to produce ionization, *i.e.,* gain or loss of electrons (as described in, for example, U.S. 5,770,581 incorporated herein by reference in its entirety). The effects of radiation can be at least partially controlled by the clinician. In one embodiment, the dose of radiation is fractionated for maximal target cell exposure and reduced toxicity.

In one embodiment, the total dose of radiation administered to a patient is about .01 Gray (Gy) to about 100 Gy. In another embodiment, about 10 Gy to about 65 Gy (e.g., about 15 Gy, 20 Gy, 25 Gy, 30 Gy, 35 Gy, 40 Gy, 45 Gy, 50 Gy, 55 Gy, or 60 Gy) are administered over the course of treatment. While in some embodiments a complete dose of radiation can be administered over the course of one day, the total dose is ideally fractionated and administered over several days. Desirably, radiotherapy is administered over the course of at least about 3 days, *e.g.,* at least 5, 7, 10, 14, 17, 21, 25, 28, 32, 35, 38, 42, 46, 52, or 56 days (about 1-8 weeks). Accordingly, a daily dose of radiation will comprise approximately 1-5 Gy (*e.g.,* about 1 Gy, 1.5 Gy, 1.8 Gy, 2 Gy, 2.5 Gy, 2.8 Gy, 3 Gy, 3.2 Gy, 3.5 Gy, 3.8 Gy, 4 Gy, 4.2 Gy, or 4.5 Gy), or 1-2 Gy *(e.g.,* 1.5-2 Gy). The daily dose of radiation should be sufficient to induce destruction of the targeted cells. If stretched over a period, in one embodiment, radiation is not administered every day, thereby allowing the animal to rest and the effects of the therapy to be realized. For example, radiation desirably is administered on 5 consecutive days, and not administered on 2 days, for each week of treatment, thereby allowing 2 days of rest per week. However, radiation can be administered 1 day/week, 2 days/week, 3 days/week, 4 days/week, 5 days/week, 6 days/week, or all 7 days/week, depending on the animal's responsiveness and any potential side effects. Radiation therapy can be initiated at any time in the therapeutic period. In one embodiment, radiation is initiated in week 1 or week 2, and is administered for the remaining duration of the therapeutic period. For example, radiation is administered in weeks 1-6 or in weeks 2-6 of a therapeutic period comprising 6 weeks for treating, for instance, a solid tumor. Alternatively, radiation is administered in weeks 1-5 or weeks 2-5 of a therapeutic period comprising 5 weeks. These exemplary radiotherapy administration schedules are not intended, however, to limit the methods provided herein.

### II. Therapeutic methods

In the therapeutic methods provided herein, the TG02 citrate salt polymorphic form and othertherapeutic, e.g., anticancer, agent may be administered to a cancer patient under one or more of the following conditions: at different periodicities, at different durations, at different concentrations, by different administration routes, *etc.*

In some embodiments, the TG02 citrate salt polymorphic form is administered prior to the othertherapeutic agent, *e.g.,* 0.5, 1, 2, 3, 4, 5, 10, 12, or 18 hours, 1, 2, 3, 4, 5, or 6 days, or 1, 2, 3, or 4 weeks prior to the administration of the othertherapeutic agent.

In some embodiments, the TG02 citrate salt polymorphic form is administered after the othertherapeutic agent, *e.g.,* 0.5, 1, 2, 3, 4, 5, 10, 12, or 18 hours, 1, 2, 3, 4, 5, or 6 days, or 1, 2, 3, or 4 weeks after the administration of the othertherapeutic agent.

In some embodiments, the TG02 citrate salt polymorphic form and the othertherapeutic agent are administered concurrently but on different schedules. In other embodiments, the TG02 polymorphic form is administered once a day while the othertherapeutic agent is administered once a week, once every two weeks, once every three weeks, or once every four weeks.

The therapeutic methods provided herein comprise administering the TG02 citrate salt polymorphic form to a cancer patient in an amount which is effective to achieve its intended purpose. While individual needs vary, determination of optimal ranges of effective amounts of each component is within the skill of the art. Typically, the TG02 citrate salt polymorphic form may be administered in an amount from about 1 mg/kg to about 500 mg/kg, about 1 mg/kg to about 100 mg/kg, or about 1 mg/kg to about 50 mg/kg. The dosage of a composition can be at any dosage including, but not limited to, 30-600 mg/day. Particular doses include 50, 100, 200, 250, 300, 400, 500, and 600 mg/day. In one embodiment, the TG02 citrate salt polymorphic form is administed once a day on 3-7 consecutive days prior to the administration of the anti-PD-1 antibody, anti-PD-L1 antibody, or temozolomide. In another embodiment, 250 mg/day of the TG02 citrate salt polymorphic form is administered. In another embodiment, 250 mg/day of the TG02 citrate salt polymorphic form is administered twice weekly. In another embodiment, TG02 citrate salt polymorphic form administration continues on the day of the anti-PD-1 antibody, anti-PD-L1 antibody, or temozolomide and continues for additional days until disease progression or until TG02 citrate salt polymorphic form administration is no longer beneficial. These dosages are exemplary of the average case, but there can be individual instances in which higher or lower dosages are merited, and such are within the scope of this disclosure. In practice, the physician determines the actual dosing regimen that is most suitable for an individual patient, which can vary with the age, weight, and response of the particular patient.

The unit oral dose of the TG02 citrate salt polymorphic form may comprise from about 0.01 to about 1000 mg, e.g., about 10 to about 500 mg of the TG02 citrate salt polymorphic form. In one embodiment, the unit oral dose of the TG02 citrate salt polymorphic form is 10 mg, 20 mg, 30 mg, 40 mg, 50 mg, 60 mg, 70 mg 80 mg, 90 mg, 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, 250 mg, 260 mg, 270 mg, 280 mg, 290 mg, or 300 mg. The unit dose may be administered one or more times daily, e.g., as one or more tablets or capsules.

In addition to administering the TG02 citrate salt polymorphic form as a raw chemical, it may be administered as part of a pharmaceutical preparation or composition. In some embodiments, the pharmaceutical preparation or composition can include one or more pharmaceutically acceptable carriers, excipients, and/or auxiliaries. In some embodiments, the one or more carriers, excipients, and/or auxiliaries facilitate processing of the TG02 citrate salt polymorphic form into a preparation or composition which can be used pharmaceutically. The preparations, particularly those preparations which can be administered orally or topically and which can be used for one type of administration, such as tablets, dragees, slow release lozenges and capsules, mouth rinses and mouth washes, gels, liquid suspensions, hair rinses, hair gels, shampoos and also preparations which can be administered rectally, such as suppositories, as well as suitable solutions for administration by intravenous infusion, injection, topically or orally, contain from about 0.01 to 99 percent, in one embodiment from about 0.25 to 75 percent of active compound(s), together with the one or more carriers, excipients, and/or auxiliaries.

The pharmaceutical compositions of provided herein may be administered to any patient which may experience the beneficial effects of the TG02 citrate salt polymorphic form. Foremost among such patients are mammals, e.g., humans, although the methods and compositions provided herein are not intended to be so limited. Other patients include veterinary animals (cows, sheep, pigs, horses, dogs, cats and the like).

The pharmaceutical compositions provided herein are manufactured by means of conventional mixing, granulating, dragee-making, dissolving, or lyophilizing processes. Thus, pharmaceutical preparations for oral use can be obtained by combining the active compounds with solid excipients, optionally grinding the resulting mixture and processing the mixture of granules, after adding suitable auxiliaries, if desired or necessary, to obtain tablets or dragee cores.

The term "excipient" as used herein refers to any ingredient in a composition other than the TG02 citrate salt polymorphic form or other therapeutic agent. An excipient is typically an inert substance added to a composition to facilitate processing, handling, administration, etc. of the TG02 citrate salt polymorphic form. Useful excipients include, but are not limited to, adjuvants, antiadherents, binders, carriers, disintegrants, fillers, flavors, colors, diluents, lubricants, glidants, preservatives, sorbents, solvents, surfactants, and sweeteners. In one embodiment, the composition comprises at least one excipient selected from the group consisting of silicified microcrystalline cellulose, hypromellose 2910, crospvidone, and magnesium stearate. In one embodiment, the composition comprises silicified microcrystalline cellulose.

Conventional pharmaceutical excipients are well known to those of skill in the art. In particular, one of skill in the art will recognize that a wide variety of pharmaceutically acceptable excipients can be used in admixture with crystalline polymorphic forms of TG02 citrate salt , including those listed in the Handbook of Pharmaceutical Excipients, Pharmaceutical Press 4th Ed. (2003), and Remington: The Science and Practice of Pharmacy, Lippincott Williams & Wilkins, 21st ed. (2005).

Suitable excipients are, in particular, fillers such as saccharides, for example lactose or sucrose, mannitol or sorbitol, cellulose preparations and/or calcium phosphates, for example tricalcium phosphate or calcium hydrogen phosphate, as well as binders such as starch paste, using, for example, maize starch, wheat starch, rice starch, potato starch, gelatin, tragacanth, methyl cellulose, hydroxypropylmethylcellulose, sodium carboxymethylcellulose, and/or polyvinyl pyrrolidone. If desired, disintegrating agents may be added such as the above-mentioned starches and also carboxymethyl-starch, cross-linked polyvinyl pyrrolidone, agar, or alginic acid or a salt thereof, such as sodium alginate. Auxiliaries can be suitable flow-regulating agents and lubricants. Suitable auxiliaries include, for example, silica, talc, stearic acid or salts thereof, such as magnesium stearate or calcium stearate, and/or polyethylene glycol. Dragee cores are provided with suitable coatings which, if desired, are resistant to gastric juices. For this purpose, concentrated saccharide solutions may be used, which may optionally contain gum arabic, talc, polyvinyl pyrrolidone, polyethylene glycol and/or titanium dioxide, lacquer solutions and suitable organic solvents or solvent mixtures. In order to produce coatings resistant to gastric juices, solutions of suitable cellulose preparations such as acetylcellulose phthalate or hydroxypropylmethyl-cellulose phthalate, are used. Dye stuffs or pigments may be added to the tablets or dragee coatings, for example, for identification or in order to characterize combinations of active compound doses.

Other pharmaceutical preparations which can be used orally include push-fit capsules made of gelatin, as well as soft, sealed capsules made of gelatin and a plasticizer such as glycerol or sorbitol. The push-fit capsules can contain the active compounds in the form of granules which may be mixed with fillers such as lactose, binders such as starches, and/or lubricants such as talc or magnesium stearate and, optionally, stabilizers. In soft capsules, the active compounds are in one embodiment dissolved or suspended in suitable liquids, such as fatty oils, or liquid paraffin. In addition, stabilizers may be added.

Possible pharmaceutical preparations which can be used rectally include, for example, suppositories, which consist of a combination of one or more of the active compounds with a suppository base. Suitable suppository bases are, for example, natural or synthetic triglycerides, or paraffin hydrocarbons. In addition, it is also possible to use gelatin rectal capsules which consist of a combination of the active compounds with a base. Possible base materials include, for example, liquid triglycerides, polyethylene glycols, or paraffin hydrocarbons.

Suitable formulations for parenteral administration include aqueous solutions of the active compounds in water-soluble form, for example, water-soluble salts and alkaline solutions. In addition, suspensions of the active compounds as appropriate oily injection suspensions may be administered. Suitable lipophilic solvents or vehicles include fatty oils, for example, sesame oil, or synthetic fatty acid esters, for example, ethyl oleate or triglycerides or polyethylene glycol-400. Aqueous injection suspensions may contain substances which increase the viscosity of the suspension including, for example, sodium carboxymethyl cellulose, sorbitol, and/or dextran. Optionally, the suspension may also contain stabilizers.

Therapeutically effective amounts of the TG02 citrate salt polymorphic form and/or the anti-PD-1 antibody, anti-PD-L1 antibody, or temozolomide and/or the optional therapeutic agent formulated in accordance with standard pharmaceutical practices, are administered to a human patient in need thereof. Whether such a treatment is indicated depends on the individual case and is subject to medical assessment (diagnosis) that takes into consideration signs, symptoms, and/or malfunctions that are present, the risks of developing particular signs, symptoms and/or malfunctions, and other factors.

The TG02 citrate salt polymorphic form, the anti-PD-1 antibody, anti-PD-L1 antibody, or temozolomide and/or the optional therapeutic agent can be administered by any suitable route, for example by oral, buccal, inhalation, sublingual, rectal, vaginal, intracisternal or intrathecal through lumbar puncture, transurethral, nasal, percutaneous, i.e., transdermal, or parenteral (including intravenous, intramuscular, subcutaneous, intracoronary, intradermal, intramammary, intraperitoneal, intraarticular, intrathecal, retrobulbar, intrapulmonary injection and/or surgical implantation at a particular site) administration. Parenteral administration can be accomplished using a needle and syringe or using a high pressure technique.

Pharmaceutical compositions include those wherein the TG02 citrate salt polymorphic form, the anti-PD-1 antibody, anti-PD-L1 antibody, or temozolomide and/or the optional therapeutic agent are administered in an effective amount to achieve its intended purpose. The exact formulation, route of administration, and dosage is determined by an individual physician in view of the diagnosed condition or disease. Dosage amount and interval can be adjusted individually to provide levels of TG02, anti-PD-1 antibody, anti-PD-L1 antibody, or temozolomide, COX-2 inhibitor, and/or optional therapeutic agent that is sufficient to maintain therapeutic effects.

Toxicity and therapeutic efficacy of TG02, the anti-PD-1 antibody, anti-PD-L1 antibody, or temozolomide and/or the optional therapeutic agent can be determined by standard pharmaceutical procedures in cell cultures or experimental animals, e.g., for determining the maximum tolerated dose (MTD) of a compound, which defines as the highest dose that causes no toxicity in a patient. The dose ratio between the maximum tolerated dose and therapeutic effects (e.g. inhibiting of tumor growth) is the therapeutic index. The dosage can vary within this range depending upon the dosage form employed, and the route of administration utilized. Determination of a therapeutically effective amount is well within the capability of those skilled in the art, especially in light of the detailed disclosure provided herein.

A therapeutically effective amount of the TG02 citrate salt polymorphic form, anti-PD-1 antibody, anti-PD-L1 antibody, or temozolomide and/or optional therapeutic agent required for use in therapy varies with the nature of the condition being treated, the length of time that activity is desired, and the age and the condition of the patient, and ultimately is determined by the attendant physician. For example, dosage amounts and intervals can be adjusted individually to provide plasma levels of TG02 and anti-PD-1 antibody, anti-PD-L1 antibody, or temozolomide that are sufficient to maintain the desired therapeutic effects. The desired dose conveniently can be administered in a single dose, or as multiple doses administered at appropriate intervals, for example as one, two, three, four or more subdoses per day. Multiple doses often are desired, or required. For example, the TG02 citrate salt polymorphic form and anti-PD-1 antibody, anti-PD-L1 antibody, or temozolomide can be administered at a frequency of: one dose per day; four doses delivered as one dose per day at four-day intervals (q4d x 4); four doses delivered as one dose per day at three-day intervals (q3d x 4); one dose delivered per day at fiveday intervals (qd x 5); one dose per week for three weeks (qwk3); five daily doses, with two days rest, and another five daily doses (5/2/5); or, any dose regimen determined to be appropriate for the circumstance.

When the anti-PD-1 antibody is nivolumab, 3 mg/kg may be administered by intravenous infusion over 60 minutes every two weeks. When the anti-PD-1 antibody is pembrolizumab, 2 mg/kg may be administered by intravenous infusion over 30 minutes every two or three weeks. When the anti-PD-L1 antibody is avelumab, 10 mg/kg may be administered by intravenous infusion as frequently as every 2 weeks. Disis et al., J. Clin Oncol. 33 (2015) (suppl; abstr 5509). When the anti-PD-L1 antibody is MPDL3280A, 20 mg/kg may be administered by intravenous infusion every 3 weeks. Herbst *et al., Nature 515:*563-80 (2014). When the anti-CTLA-4 antibody is ipilumumab, 3 mg/kg may be administered by intravenous infusion over 90 minutes every 3 weeks. When the anti-CTLA-4 antibody is tremelimumab, 15 mg/kg may be administered by intravenous infusion every 12 weeks. Naido et al., British Journal of Cancer 111:2214-19 (2014); Drugs R D, 10: 123-32 (2010). When the anti-LAG3 antibody is GSK2831781, 1.5 to 5 mg/kg may be administered by intravenous infusion over 120 minutes every 2-4 weeks.

In one embodiment, the cancer patient has tumors with a biomarker, e.g., overexpression of MYC and/or MCL1, and receives 2 mg/kg pembrolizumab administered by intravenous infusion every three weeks and 30-600 mg of TG02 administered for 3-7 days prior to pembrolizumab administration, on the day of pembrolizumab administration, and thereafter until disease progression or until there is no therapeutic benefit.

In another embodiment, the cancer patient has tumors with a biomarker, e.g., overexpression of MYC and/or MCL1, and receives 3 mg/kg nivolumab administered by intravenous infusion every 2 weeks and 30-600 mg TG02 administered orally for 3-7 days prior to nivolumab administration, on the day of nivolumab administration, and thereafter until disease progression or until there is no therapeutic benefit.

In another embodiment, the cancer patient has tumors with a biomarker, e.g., overexpression of MYC and/or MCL1, and receives 3 mg/kg nivolumab administered by intravenous infusion every 2 weeks and 30-600 mg TG02 administered orally twice weekly prior to nivolumab administration, on the day of nivolumab administration, and thereafter until disease progression or until there is no therapeutic benefit.

In another embodiment, the treatment of the cancer patient with the anti-PD-1 antibody, anti-PD-L1 antibody, or temozolomide and the TG02 citrate salt polymorphic form induces anti-proliferative response faster than when the anti-PD-1 antibody, anti-PD-L1 antibody, or temozolomide is administered alone.

In another embodiment, the treatment of the cancer patient with a COX-2 inhibitor and the TG02 polymorphic form induces anti-proliferative response faster than when the COX-2 inhibitor is administered alone.

### III. Biomarkers

The term "biomarker" as used herein refers to any biological compound, such as a gene, a protein, a fragment of a protein, a peptide, a polypeptide, a nucleic acid, etc., that can be detected and/or quantified in a cancer patient *in vivo* or in a biological sample obtained from a cancer patient. A biomarker can be the entire intact molecule, or it can be a portion or fragment thereof. In one embodiment, the expression level of the biomarker is measured. The expression level of the biomarker can be measured, for example, by detecting the protein or RNA, e.g., mRNA, level of the biomarker. In some embodiments, portions or fragments of biomarkers can be detected or measured, for example, by an antibody or other specific binding agent. In some embodiments, a measurable aspect of the biomarker is associated with a given state of the patient, such as a particular stage of cancer. For biomarkers that are detected at the protein or RNA level, such measurable aspects may include, for example, the presence, absence, or concentration, i.e., expression level, of the biomarker in a cancer patient, or biological sample obtained from the cancer patient. For biomarkers that are detected at the nucleic acid level, such measurable aspects may include, for example, allelic versions of the biomarker or type, rate, and/or degree of mutation of the biomarker, also referred to herein as mutation status.

For biomarkers that are detected based on expression level of protein or RNA, expression level measured between different phenotypic statuses can be considered different, for example, if the mean or median expression level of the biomarker in the different groups is calculated to be statistically significant. Common tests for statistical significance include, among others, t-test, ANOVA, Kruskal-Wallis, Wilcoxon, Mann-Whitney, Significance Analysis of Microarrays, odds ratio, etc. Biomarkers, alone or in combination, provide measures of relative likelihood that a subject belongs to one phenotypic status or another. Therefore, they are useful, inter alia, as markers for disease and as indicators that particular therapeutic treatment regimens will likely result in beneficial patient outcomes.

Biomarkers include, but are not limited, the genes listed in Table 1. In one embodiment, the measurable aspect of the biomarker is its expression status. In one embodiment, the measurable aspect of the biomarker is its mutation status.

**Table 1**

| **Gene** | **Gene synonym** | **Gene description** |
|---|---|---|
| A2M | CPAMD5, FWP007, S863-7 | Alpha-2-macroglobulin |
| ABCB1 | ABC20, CD243, CLCS, GP170, MDR1, P-gp, PGY1 | ATP-binding cassette, sub-family B (MDR/TAP), member 1 |
| ABCC1 | GS-X, MRP, MRP1 | ATP-binding cassette, sub-family C (CFTR/MRP), member 1 |
| ABCC2 | CMOAT, cMRP, DJS, MRP2 | ATP-binding cassette, sub-family C (CFTR/MRP), member 2 |
| ABCC3 | cMOAT2, EST90757, MLP2, MOAT-D, MRP3 | ATP-binding cassette, sub-family C (CFTR/MRP), member 3 |
| ABCC5 | EST277145, MOAT-C, MRP5, SMRP | ATP-binding cassette, sub-family C (CFTR/MRP), member 5 |
| ABCC6 | ARA, EST349056, MLP1, MRP6, PXE, URG7 | ATP-binding cassette, sub-family C (CFTR/MRP), member 6 |
| ABCG2 | ABCP, BCRP, CD338, EST157481, MXR | ATP-binding cassette, sub-family G (WHITE), member 2 (Junior blood group) |
| ABL1 | ABL, c-ABL, JTK7, p150 | ABL proto-oncogene 1, non-receptor tyrosine kinase |
| ABL2 | ABLL, ARG | ABL proto-oncogene 2, non-receptor tyrosine kinase |
| ACAP1 | CENTB1, KIAA0050 | ArfGAP with coiled-coil, ankyrin repeat and PH domains 1 |
| ACLY | ACL, ATPCL, CLATP | ATP citrate lyase |
| ACPP | ACP-3, ACP3 | Acid phosphatase, prostate |
| ACVR1B | ActRIB, ACVRLK4, ALK4, SKR2 | Activin A receptor, type IB |
| ACVR2A | ACTRII, ACVR2 | Activin A receptor, type IIA |
| ACVR2B | ActR-IIB | Activin A receptor, type IIB |
| ADAM9 | CORD9, KIAA0021, MCMP, MDC9, Mltng | ADAM metallopeptidase domain 9 |
| ADAMTS1 | C3-C5, KIAA1346, METH1 | ADAM metallopeptidase with thrombospondin type 1 motif, 1 |
| ADAMTS14 | | ADAM metallopeptidase with thrombospondin type 1 motif, 14 |
| ADAMTS18 | ADAMTS21 | ADAM metallopeptidase with thrombospondin type 1 motif, 18 |
| ADAMTS20 | GON-1 | ADAM metallopeptidase with thrombospondin type 1 motif, 20 |
| ADAMTS3 | ADAMTS-4, KIAA0366 | ADAM metallopeptidase with thrombospondin type 1 motif, 3 |
| ADAMTS4 | ADAMTS-2, ADMP-1, KIAA0688 | ADAM metallopeptidase with thrombospondin type 1 motif, 4 |
| ADAMTS5 | ADAMTS11, ADMP-2 | ADAM metallopeptidase with thrombospondin type 1 motif, 5 |
| ADAMTS6 | ADAM-TS6 | ADAM metallopeptidase with thrombospondin type 1 motif, 6 |
| ADAMTS8 | ADAM-TS8, FLJ41712, METH2 | ADAM metallopeptidase with thrombospondin type 1 motif, 8 |
| ADAMTS9 | KIAA1312 | ADAM metallopeptidase with thrombospondin type 1 motif, 9 |
| ADM | AM | Adrenomedullin |
| ADRA1B | | Adrenoceptor alpha 1B |
| AFP | FETA, HPAFP | Alpha-fetoprotein |
| AGER | RAGE | Advanced glycosylation end product-specific receptor |
| AHR | bHLHe76 | Aryl hydrocarbon receptor |
| AHSG | A2HS, FETUA, HSGA | Alpha-2-HS-glycoprotein |
| AKAP12 | AKAP250, SSeCKS | A kinase (PRKA) anchor protein 12 |
| AKR1B1 | ALDR1, AR | Aldo-keto reductase family 1, member B1 (aldose reductase) |
| AKT1 | AKT, PKB, PRKBA, RAC | V-akt murine thymoma viral oncogene homolog 1 |
| AKT2 | | V-akt murine thymoma viral oncogene homolog 2 |
| AKT3 | PKBG, PRKBG, RAC-gamma | V-akt murine thymoma viral oncogene homolog 3 |
| ALB | | Albumin |
| ALCAM | CD166, MEMD | Activated leukocyte cell adhesion molecule |
| ALDOA | | Aldolase A, fructose-bisphosphate |
| ALDOB | | Aldolase B, fructose-bisphosphate |
| ALDOC | | Aldolase C, fructose-bisphosphate |
| ALPL | HOPS, TNSALP | Alkaline phosphatase, liver/bone/kidney |
| ALPP | | Alkaline phosphatase, placental |
| ANG | RNASE5 | Angiogenin, ribonuclease, RNase A family, 5 |
| ANGPT1 | Ang1, KIAA0003 | Angiopoietin 1 |
| ANGPT2 | Ang2 | Angiopoietin 2 |
| ANXA1 | ANX1, LPC1 | Annexin A1 |
| ANXA11 | ANX11 | Annexin A11 |
| ANXA2 | ANX2, ANX2L4, CAL1H, LIP2, LPC2D | Annexin A2 |
| ANXA4 | ANX4 | Annexin A4 |
| ANXA7 | ANX7 | Annexin A7 |
| AOC3 | HPAO, VAP-1, VAP1 | Amine oxidase, copper containing 3 |
| AP2B1 | ADTB2, CLAPB 1 | Adaptor-related protein complex 2, beta 1 subunit |
| APAF1 | APAF-1, CED4 | Apoptotic peptidase activating factor 1 |
| APEX1 | APE, APE-1, APEN, APEX, APX, HAP1, REF-1, REF1 | APEX nuclease (multifunctional DNA repair enzyme) 1 |
| APOA1 | | Apolipoprotein A-I |
| APOA2 | | Apolipoprotein A-II |
| APOC1 | | Apolipoprotein C-I |
| APOC3 | | Apolipoprotein C-III |
| APOD | | Apolipoprotein D |
| APOE | AD2 | Apolipoprotein E |
| APPBP2 | Hs.84084, KIAA0228, PAT1 | Amyloid beta precursor protein (cytoplasmic tail) binding protein 2 |
| AR | AIS, DHTR, HUMARA, NR3C4, SBMA, SMAX1 | Androgen receptor |
| AREG | AREGB, SDGF | Amphiregulin |
| ARG2 | | Arginase 2 |
| ARNT | bHLHe2, HIF-1beta | Aryl hydrocarbon receptor nuclear translocator |
| ASPH | BAH, CASQ2BP1, HAAH, JCTN | Aspartate beta-hydroxylase |
| ATM | ATA, ATC, ATD, ATDC, TEL1, TELO1 | ATM serine/threonine kinase |
| ATOH1 | bHLHa14, HATH1, MATH-1, Math1 | Atonal homolog 1 (Drosophila) |
| ATP7B | WND | ATPase, Cu++ transporting, beta polypeptide |
| AURKA | AIK, ARK1, AurA, BTAK, PPP1R47, STK15, STK6, STK7 | Aurora kinase A |
| AURKB | Aik2, AIM-1, ARK2, AurB, IPL1, PPP1R48, STK12, STK5 | Aurora kinase B |
| AZGP1 | ZA2G, ZAG | Alpha-2-glycoprotein 1, zinc-binding |
| B2M | | Beta-2-microglobulin |
| BAD | BBC2, BCL2L8 | BCL2-associated agonist of cell death |
| BAG1 | | BCL2-associated athanogene |
| BAI1 | | Brain-specific angiogenesis inhibitor 1 |
| BAX | BCL2L4 | BCL2-associated X protein |
| BCL11A | BCL11A-L, BCL11A-S, BCL11A-XL, CTIP1, EVI9, HBFQTL5, ZNF856 | B-cell CLL/lymphoma 11A (zinc finger protein) |
| BCL2 | Bcl-2, PPP1R50 | B-cell CLL/lymphoma 2 |
| BCL2A1 | ACC-1, ACC-2, BCL2L5, BFL1, GRS, HBPA1 | BCL2-related protein A1 |
| BCL2L1 | Bcl-X, bcl-xL, bcl-xS, BCL2L, BCLX, PPP1R52 | BCL2-like 1 |
| BCL2L2 | BCL-W, KIAA0271, PPP1R51 | BCL2-like 2 |
| BCL2L2-PABPN1 | | BCL2L2-PABPN1 readthrough |
| BCL3 | BCL4, D19S37 | B-cell CLL/lymphoma 3 |
| BCL6 | BCL5, BCL6A, LAZ3, ZBTB27, ZNF51 | B-cell CLL/lymphoma 6 |
| BDNF | | Brain-derived neurotrophic factor |
| BIRC2 | API1, c-IAP1, cIAP1, hiap-2, MIHB, RNF48 | Baculoviral IAP repeat containing 2 |
| BIRC3 | API2, c-IAP2, cIAP2, hiap-1, MALT2, MIHC, RNF49 | Baculoviral IAP repeat containing 3 |
| BIRC5 | API4, EPR-1, survivin | Baculoviral IAP repeat containing 5 |
| BIRC6 | BRUCE | Baculoviral IAP repeat containing 6 |
| BLK | MGC10442 | BLK proto-oncogene, Src family tyrosine kinase |
| BLMH | BH | Bleomycin hydrolase |
| BMI1 | PCGF4, RNF51 | BMI1 proto-oncogene, polycomb ring finger |
| BMP2 | BMP2A | Bone morphogenetic protein 2 |
| BMP4 | BMP2B | Bone morphogenetic protein 4 |
| BNIP3 | Nip3 | BCL2/adenovirus E1B 19kDa interacting protein 3 |
| BNIP3L | BNIP3a, Nix | BCL2/adenovirus E1B 19kDa interacting protein 3-like |
| BRCA1 | BRCC1, PPP1R53, RNF53 | Breast cancer 1, early onset |
| BRCA2 | BRCC2, FACD, FAD, FAD1, FANCD, FANCD1 | Breast cancer 2, early onset |
| BRMS1 | DKFZP564A063 | Breast cancer metastasis suppressor 1 |
| BTG2 | MGC126063, MGC126064, PC3, TIS21 | BTG family, member 2 |
| C18orf8 | HsT2591, MIC-1, MIC1 | Chromosome 18 open reading frame 8 |
| C1QBP | gC1Q-R, gC1qR, HABP1, p32, SF2p32 | Complement component 1, q subcomponent binding protein |
| C6 | | Complement component 6 |
| C7 | | Complement component 7 |
| CA8 | CALS, CARP | Carbonic anhydrase VIII |
| CALCA | CALC1 | Calcitonin-related polypeptide alpha |
| CALM1 | CALML2, CAMI, DD132, PHKD | Calmodulin 1 (phosphorylase kinase, delta) |
| CALM2 | CAMII, PHKD | Calmodulin 2 (phosphorylase kinase, delta) |
| CALM3 | PHKD | Calmodulin 3 (phosphorylase kinase, delta) |
| CALR | cC1qR, CRT, FLJ26680, RO, SSA | Calreticulin |
| CANX | CNX, IP90, P90 | Calnexin |
| CAPN6 | CalpM, CANPX, CAPNX | Calpain 6 |
| CASC3 | BTZ, MLN51 | Cancer susceptibility candidate 3 |
| CASP1 | ICE, IL1BC | Caspase 1, apoptosis-related cysteine peptidase |
| CASP10 | MCH4 | Caspase 10, apoptosis-related cysteine peptidase |
| CASP2 | ICH1, MGC2181, NEDD2, PPP1R57 | Caspase 2, apoptosis-related cysteine peptidase |
| CASP3 | apopain, CPP32, CPP32B, Yama | Caspase 3, apoptosis-related cysteine peptidase |
| CASP4 | ICE(rel)II, ICH-2, TX | Caspase 4, apoptosis-related cysteine peptidase |
| CASP5 | ICE(rel)III | Caspase 5, apoptosis-related cysteine peptidase |
| CASP6 | MCH2 | Caspase 6, apoptosis-related cysteine peptidase |
| CASP7 | CMH-1, ICE-LAP3, MCH3 | Caspase 7, apoptosis-related cysteine peptidase |
| CASP8 | Casp-8, FLICE, MACH, MCH5 | Caspase 8, apoptosis-related cysteine peptidase |
| CASP9 | APAF-3, ICE-LAP6, MCH6, PPP1R56 | Caspase 9, apoptosis-related cysteine peptidase |
| CAT | | Catalase |
| CAV1 | CAV | Caveolin 1, caveolae protein, 22kDa |
| CBL | c-Cbl, CBL2, RNF55 | Cbl proto-oncogene, E3 ubiquitin protein ligase |
| CCKBR | | Cholecystokinin B receptor |
| CCL11 | eotaxin, MGC22554, SCYA11 | Chemokine (C-C motif) ligand 11 |
| CCL13 | CKb10, MCP-4, MGC17134, NCC-1, SCYA13, SCYL1 | Chemokine (C-C motif) ligand 13 |
| CCL14 | CKb1, HCC-1, HCC-3, MCIF, NCC-2, SCYA14, SCYL2 | Chemokine (C-C motif) ligand 14 |
| CCL16 | CKb12, HCC-4, LCC-1, LEC, LMC, Mtn-1, NCC-4, SCYA16, SCYL4 | Chemokine (C-C motif) ligand 16 |
| CCL18 | AMAC-1, CKb7, DC-CK1, DCCK1, MIP-4, PARC, SCYA18 | Chemokine (C-C motif) ligand 18 (pulmonary and activation-regulated) |
| CCL19 | CKb11, ELC, exodus-3, MIP-3b, SCYA19 | Chemokine (C-C motif) ligand 19 |
| CCL2 | GDCF-2, HC11, MCAF, MCP-1, MCP1, MGC9434, SCYA2, SMC-CF | Chemokine (C-C motif) ligand 2 |
| CCL21 | 6Ckine, CKb9, ECL, exodus-2, SCYA21, SLC, TCA4 | Chemokine (C-C motif) ligand 21 |
| CCL23 | Ckb-8, CKb8, MIP-3, MPIF-1, SCYA23 | Chemokine (C-C motif) ligand 23 |
| CCL3 | G0S19-1, LD78ALPHA, MIP-1-alpha, SCYA3 | Chemokine (C-C motif) ligand 3 |
| CCL4 | Act-2, AT744.1, LAG1, MIP-1-beta, SCYA4 | Chemokine (C-C motif) ligand 4 |
| CCL5 | D17S136E, MGC17164, RANTES, SCYA5, SISd, TCP228 | Chemokine (C-C motif) ligand 5 |
| CCL7 | FIC, MARC, MCP-3, MCP3, NC28, SCYA6, SCYA7 | Chemokine (C-C motif) ligand 7 |
| CCL8 | HC14, MCP-2, SCYA8 | Chemokine (C-C motif) ligand 8 |
| CCNA1 | CT146 | Cyclin A1 |
| CCNA2 | CCN1, CCNA | Cyclin A2 |
| CCNB1 | CCNB | Cyclin B1 |
| CCNB2 | HsT17299 | Cyclin B2 |
| CCND1 | BCL1, D11S287E, PRAD1, U21B31 | Cyclin D1 |
| CCND2 | | Cyclin D2 |
| CCNE1 | CCNE | Cyclin E1 |
| CCNE2 | CYCE2 | Cyclin E2 |
| CCNG1 | CCNG | Cyclin G1 |
| CCNG2 | | Cyclin G2 |
| CCNH | CycH, p34, p37 | Cyclin H |
| CCR10 | GPR2 | Chemokine (C-C motif) receptor 10 |
| CCR7 | BLR2, CD197, CDw197, CMKBR7, EBI1 | Chemokine (C-C motif) receptor 7 |
| CD14 | | CD14 molecule |
| CD27 | S152, TNFRSF7, Tp55 | CD27 molecule |
| CD36 | FAT, GP3B, GP4, GPIV, SCARB3 | CD36 molecule (thrombospondin receptor) |
| CD38 | | CD38 molecule |
| CD40 | Bp50, p50, TNFRSF5 | CD40 molecule, TNF receptor superfamily member 5 |
| CD40LG | CD154, CD40L, gp39, hCD40L, HIGM1, IMD3, TNFSF5, TRAP | CD40 ligand |
| CD44 | CD44R, CSPG8, HCELL, IN, MC56, MDU2, MDU3, MIC4, Pgp1 | CD44 molecule (Indian blood group) |
| CD46 | MCP, MGC26544, MIC10, TLX, TRA2.10 | CD46 molecule, complement regulatory protein |
| CD52 | CDW52 | CD52 molecule |
| CD59 | 16.3A5, EJ16, EJ30, EL32, G344, MIC11, MIN1, MIN2, MIN3, MSK21, p18-20 | CD59 molecule, complement regulatory protein |
| CD70 | CD27L, CD27LG, TNFSF7 | CD70 molecule |
| CD74 | DHLAG | CD74 molecule, major histocompatibility complex, class II invariant chain |
| CD82 | IA4, KAI1, R2, ST6, TSPAN27 | CD82 molecule |
| CD9 | BA2, MIC3, MRP-1, P24, TSPAN29 | CD9 molecule |
| CDC16 | ANAPC6, APC6, CUT9 | Cell division cycle 16 |
| CDC20 | CDC20A, p55CDC | Cell division cycle 20 |
| CDC25A | | Cell division cycle 25A |
| CDC25B | | Cell division cycle 25B |
| CDC25C | CDC25, PPP1R60 | Cell division cycle 25C |
| CDC34 | E2-CDC34, UBC3, UBE2R1 | Cell division cycle 34 |
| CDC37 | P50CDC37 | Cell division cycle 37 |
| CDC6 | CDC18L | Cell division cycle 6 |
| CDH1 | CD324, UVO, uvomorulin | Cadherin 1, type 1, E-cadherin (epithelial) |
| CDH17 | cadherin, HPT-1 | Cadherin 17, LI cadherin (liver-intestine) |
| CDH5 | 7B4, CD144 | Cadherin 5, type 2 (vascular endothelium) |
| CDK1 | CDC2, CDC28A | Cyclin-dependent kinase 1 |
| CDK2 | | Cyclin-dependent kinase 2 |
| CDK4 | PSK-J3 | Cyclin-dependent kinase 4 |
| CDK6 | PLSTIRE | Cyclin-dependent kinase 6 |
| CDK7 | CAK, CAK1, CDKN7, MO15, STK1 | Cyclin-dependent kinase 7 |
| CDKN1A | CAP20, CDKN1, CIP1, P21, p21CIP1, p21Cip1/Waf1, SDI1, WAF1 | Cyclin-dependent kinase inhibitor 1A (p21, Cip1) |
| CDKN1C | BWCR, BWS, KIP2, P57 | Cyclin-dependent kinase inhibitor 1C (p57, Kip2) |
| CDKN2A | ARF, CDK4I, CDKN2, CMM2, INK4, INK4a, MLM, MTS1, p14, p14ARF, p16, p16INK4a, p19, p19Arf | Cyclin-dependent kinase inhibitor 2A |
| CEACAM5 | CD66e, CEA | Carcinoembryonic antigen-related cell adhesion molecule 5 |
| CEACAM6 | CD66c, NCA | Carcinoembryonic antigen-related cell adhesion molecule 6 (non-specific cross reacting antigen) |
| CENPF | hcp-1 | Centromere protein F, 350/400kDa |
| CFHR1 | CFHL, CFHL1, CFHL1P, CFHR1P, FHR1, H36-1, H36-2, HFL1, HFL2 | Complement factor H-related 1 |
| CFLAR | c-FLIP, CASH, CASP8AP1, Casper, CLARP, FLAME, FLIP, I-FLICE, MRIT | CASP8 and FADD-like apoptosis regulator |
| CFTR | ABC35, ABCC7, CF, CFTR/MRP, dJ760C5.1, MRP7, TNR-CFTR | Cystic fibrosis transmembrane conductance regulator (ATP-binding cassette sub-family C, member 7) |
| CGA | FSHA, GPHa, GPHA1, HCG, LHA, TSHA | Glycoprotein hormones, alpha polypeptide |
| CGB | CGB3 | Chorionic gonadotropin, beta polypeptide |
| CGB5 | HCG | Chorionic gonadotropin, beta polypeptide 5 |
| CGB7 | CG-beta-a | Chorionic gonadotropin, beta polypeptide 7 |
| CGB8 | | Chorionic gonadotropin, beta polypeptide 8 |
| CHD7 | CRG, FLJ20357, FLJ20361, KIAA1416 | Chromodomain helicase DNA binding protein 7 |
| CHEK1 | CHK1 | Checkpoint kinase 1 |
| CHEK2 | bA444G7, CDS1, CHK2, HuCds1, PP1425, RAD53 | Checkpoint kinase 2 |
| CHFR | FLJ10796, RNF196 | Checkpoint with forkhead and ring finger domains, E3 ubiquitin protein ligase |
| CHGA | | Chromogranin A (parathyroid secretory protein 1) |
| CHI3L1 | GP39, YKL40 | Chitinase 3-like 1 (cartilage glycoprotein-39) |
| CHP2 | | Calcineurin-like EF-hand protein 2 |
| CIB2 | DFNB48, KIP2, USH1J | Calcium and integrin binding family member 2 |
| CKB | CKBB | Creatine kinase, brain |
| CKS1B | CKS1, ckshs1 | CDC28 protein kinase regulatory subunit 1B |
| CKS2 | | CDC28 protein kinase regulatory subunit 2 |
| CLDN3 | C7orf1, CPE-R2, CPETR2, HRVP1, RVP1 | Claudin 3 |
| CLDN4 | CPE-R, CPETR, CPETR1, hCPE-R, WBSCR8 | Claudin 4 |
| CLDN7 | CEPTRL2, CPETRL2, Hs.84359 | Claudin 7 |
| CLEC3B | TN, TNA | C-type lectin domain family 3, member B |
| CLIC1 | NCC27, p64CLCP | Chloride intracellular channel 1 |
| CLIP1 | CLIP, CLIP-170, CLIP170, CYLN1, RSN | CAP-GLY domain containing linker protein 1 |
| CLSTN1 | CDHR12, CSTN1, KIAA0911 | Calsyntenin 1 |
| CLU | APOJ, CLI, CLU1, CLU2, KUB1, SGP-2, SP-40, TRPM-2 | Clusterin |
| CNN1 | Sm-Calp, SMCC | Calponin 1, basic, smooth muscle |
| CNTF | HCNTF | Ciliary neurotrophic factor |
| COL11A1 | CO11A1, COLL6, STL2 | Collagen, type XI, alpha 1 |
| COL17A1 | BP180, BPAG2 | Collagen, type XVII, alpha 1 |
| COL18A1 | KNO, KNO1, KS | Collagen, type XVIII, alpha 1 |
| COL1A1 | OI4 | Collagen, type I, alpha 1 |
| COL1A2 | OI4 | Collagen, type I, alpha 2 |
| COL4A2 | DKFZp686I14213, FLJ22259 | Collagen, type IV, alpha 2 |
| COL4A3 | | Collagen, type IV, alpha 3 (Goodpasture antigen) |
| COL4A4 | CA44 | Collagen, type IV, alpha 4 |
| COL4A5 | ASLN, ATS | Collagen, type IV, alpha 5 |
| COL6A1 | | Collagen, type VI, alpha 1 |
| COX17 | | COX17 cytochrome c oxidase copper chaperone |
| CP | | Ceruloplasmin (ferroxidase) |
| CRABP1 | CRABP, CRABP-I, CRABPI, RBP5 | Cellular retinoic acid binding protein 1 |
| CRADD | RAIDD | CASP2 and RIPK1 domain containing adaptor with death domain |
| CREBBP | CBP, KAT3A, RSTS, RTS | CREB binding protein |
| CRP | PTX1 | C-reactive protein, pentraxin-related |
| CRYAB | CRYA2, HSPB5 | Crystallin, alpha B |
| CSE1L | CAS, CSE1, XPO2 | CSE1 chromosome segregation 1-like (yeast) |
| CSF1 | M-CSF, MCSF, MGC31930 | Colony stimulating factor 1 (macrophage) |
| CSF1R | C-FMS, CD115, CSFR, FMS | Colony stimulating factor 1 receptor |
| CSF2 | GM-CSF, GMCSF | Colony stimulating factor 2 (granulocyte-macrophage) |
| CSF2RA | CD116, CSF2R | Colony stimulating factor 2 receptor, alpha, low-affinity (granulocyte-macrophage) |
| CSF3 | C17orf33, G-CSF, GCSF, MGC45931 | Colony stimulating factor 3 (granulocyte) |
| CSN1S1 | CASA, CSN1 | Casein alpha s1 |
| CSNK1E | CKIE, CKIepsilon, HCKIE | Casein kinase 1, epsilon |
| CSNK2A1 | | Casein kinase 2, alpha 1 polypeptide |
| CSNK2A2 | CSNK2A1 | Casein kinase 2, alpha prime polypeptide |
| CSNK2B | | Casein kinase 2, beta polypeptide |
| CST3 | | Cystatin C |
| CST6 | | Cystatin E/M |
| CSTA | STF1, STFA | Cystatin A (stefin A) |
| CSTB | CST6, EPM1, PME, STFB | Cystatin B (stefin B) |
| CTAG1A | ESO1, LAGE2A | Cancer/testis antigen 1A |
| CTAG1B | CT6.1, CTAG, CTAG1, ESO1, LAGE2A, LAGE2B, NY-ESO-1 | Cancer/testis antigen 1B |
| CTAG2 | CAMEL, CT6.2a, CT6.2b, ESO2, LAGE-1, LAGE-1a, LAGE-1b, LAGE1, MGC138724, MGC3803 | Cancer/testis antigen 2 |
| CTGF | CCN2, IGFBP8 | Connective tissue growth factor |
| CTNNB1 | armadillo, beta-catenin, CTNNB | Catenin (cadherin-associated protein), beta 1, 88kDa |
| CTNNBL1 | C20orf33, FLJ21108, NAP, NYD-SP19, P14, P14L | Catenin, beta like 1 |
| CTSB | | Cathepsin B |
| CTSD | CLN10, CPSD | Cathepsin D |
| CTSH | ACC-4, ACC-5, CPSB | Cathepsin H |
| CTSL | CTSL1, FLJ31037 | Cathepsin L |
| CUL2 | | Cullin 2 |
| CUL5 | VACM-1 | Cullin 5 |
| CXCL1 | FSP, GRO1, GROa, MGSA, MGSA-a, NAP-3, SCYB1 | Chemokine (C-X-C motif) ligand 1 (melanoma growth stimulating activity, alpha) |
| CXCL10 | C7, crg-2, gIP-10, IFI10, INP10, IP-10, mob-1, SCYB10 | Chemokine (C-X-C motif) ligand 10 |
| CXCL13 | ANGIE, ANGIE2, BCA-1, BLC, BLR1L, SCYB13 | Chemokine (C-X-C motif) ligand 13 |
| CXCL2 | CINC-2a, GRO2, GROb, MGSA-b, MIP-2a, SCYB2 | Chemokine (C-X-C motif) ligand 2 |
| CXCL5 | ENA-78, SCYB5 | Chemokine (C-X-C motif) ligand 5 |
| CXCL8 | 3-10C, AMCF-I, b-ENAP, GCP-1, GCP1, IL-8, IL8, K60, LECT, LUCT, LYNAP, MDNCF, MONAP, NAF, NAP-1, NAP1, SCYB8, TSG-1 | Chemokine (C-X-C motif) ligand 8 |
| CXCL9 | CMK, crg-10, Humig, MIG, SCYB9 | Chemokine (C-X-C motif) ligand 9 |
| CXCR1 | CD181, CDw128a, CKR-1, CMKAR1, IL8RA | Chemokine (C-X-C motif) receptor 1 |
| CXCR2 | CD182, CMKAR2, IL8RB | Chemokine (C-X-C motif) receptor 2 |
| CXCR4 | CD184, D2S201E, fusin, HM89, HSY3RR, LESTR, NPY3R, NPYR, NPYY3R | Chemokine (C-X-C motif) receptor 4 |
| CYB5R3 | DIA1 | Cytochrome b5 reductase 3 |
| CYP19A1 | ARO, ARO1, aromatase, CPV1, CYAR, CYP19, P-450AROM | Cytochrome P450, family 19, subfamily A, polypeptide 1 |
| CYP1A2 | CP12, P3-450 | Cytochrome P450, family 1, subfamily A, polypeptide 2 |
| CYP2C19 | CPCJ, CYP2C, P450IIC19 | Cytochrome P450, family 2, subfamily C, polypeptide 19 |
| CYP2E1 | CYP2E | Cytochrome P450, family 2, subfamily E, polypeptide 1 |
| CYP3A4 | CYP3A3 | Cytochrome P450, family 3, subfamily A, polypeptide 4 |
| CYP3A5 | CP35, P450PCN3, PCN3 | Cytochrome P450, family 3, subfamily A, polypeptide 5 |
| DAD1 | OST2 | Defender against cell death 1 |
| DAPK1 | DAPK | Death-associated protein kinase 1 |
| DAXX | DAP6 | Death-domain associated protein |
| DBI | ACBD1, ACBP | Diazepam binding inhibitor (GABA receptor modulator, acyl-CoA binding protein) |
| DCC | IGDCC1, NTN1R1 | DCC netrin 1 receptor |
| DCDC1 | | Doublecortin domain containing 1 |
| DCN | DSPG2, SLRR1B | Decorin |
| DDB2 | DDBB, FLJ34321, UV-DDB2 | Damage-specific DNA binding protein 2, 48kDa |
| DDIT3 | CHOP, CHOP10, GADD153 | DNA-damage-inducible transcript 3 |
| DEFA1 | DEF1, DEFA2, HNP-1, MRS | Defensin, alpha 1 |
| DEFA1B | | Defensin, alpha 1B |
| DEFA3 | DEF3, HNP-3 | Defensin, alpha 3, neutrophil-specific |
| DEK | D6S231E | DEK proto-oncogene |
| DES | CMD1I, CSM1, CSM2 | Desmin |
| DHFR | | Dihydrofolate reductase |
| DIAPH3 | AN, AUNA1, DRF3, FLJ34705, NSDAN | Diaphanous-related formin 3 |
| DLC1 | ARHGAP7, DLC-1, HP, p122-RhoGAP, STARD12 | DLC1 Rho GTPase activating protein |
| DNAJC2 | MPHOSPH11, MPP11, ZRF1, ZUO1, zuotin | DnaJ (Hsp40) homolog, subfamily C, member 2 |
| DST | BP240, BPA, BPAG1, CATX-15, FLJ13425, FLJ21489, FLJ30627, FLJ32235, KIAA0728, MACF2 | Dystonin |
| DUSP1 | CL100, HVH1, MKP-1, PTPN10 | Dual specificity phosphatase 1 |
| DUSP14 | MKP-L, MKP6 | Dual specificity phosphatase 14 |
| DUSP4 | HVH2, MKP-2, TYP | Dual specificity phosphatase 4 |
| DVL3 | KIAA0208 | Dishevelled segment polarity protein 3 |
| DYNLL1 | DLC1, DLC8, DNCL1, hdlc1, LC8, PIN | Dynein, light chain, LC8-type 1 |
| DYRK2 | | Dual-specificity tyrosine-(Y)-phosphorylation regulated kinase 2 |
| E2F1 | RBBP3, RBP3 | E2F transcription factor 1 |
| E2F3 | | E2F transcription factor 3 |
| E2F5 | | E2F transcription factor 5, p130-binding |
| EBAG9 | EB9, RCAS1 | Estrogen receptor binding site associated, antigen, 9 |
| EDN1 | ET1 | Endothelin 1 |
| EEF2 | EEF-2, EF2 | Eukaryotic translation elongation factor 2 |
| EFNA1 | ECKLG, EPLG1, LERK1, TNFAIP4 | Ephrin-A1 |
| EFNA2 | ELF-1, EPLG6, LERK6 | Ephrin-A2 |
| EFNA5 | AF1, EPLG7, LERK7 | Ephrin-A5 |
| EFNB1 | CFNS, Elk-L, EPLG2, LERK2 | Ephrin-B1 |
| EFNB2 | EPLG5, Htk-L, HTKL, LERK5, MGC126226, MGC126227, MGC126228 | Ephrin-B2 |
| EFNB3 | EPLG8, LERK-8 | Ephrin-B3 |
| EGF | | Epidermal growth factor |
| EGFR | ERBB, ERBB1 | Epidermal growth factor receptor |
| EGR1 | AT225, G0S30, KROX-24, NGFI-A, TIS8, ZIF-268, ZNF225 | Early growth response 1 |
| EI24 | EPG4, PIG8, TP53I8 | Etoposide induced 2.4 |
| EIF3H | eIF3-gamma, eIF3-p40, eIF3h, EIF3S3 | Eukaryotic translation initiation factor 3, subunit H |
| EIF4E | EIF4E1, EIF4EL1, EIF4F | Eukaryotic translation initiation factor 4E |
| EIF4EBP1 | 4E-BP1, PHAS-I | Eukaryotic translation initiation factor 4E binding protein 1 |
| EIF4G1 | EIF4F, EIF4G, p220, PARK18 | Eukaryotic translation initiation factor 4 gamma, 1 |
| EIF4H | KIAA0038, WBSCR1, WSCR1 | Eukaryotic translation initiation factor 4H |
| EIF5A | EIF-5A, EIF5A1, MGC104255, MGC99547 | Eukaryotic translation initiation factor 5A |
| ELANE | ELA2, HLE, HNE, NE | Elastase, neutrophil expressed |
| ELK3 | ERP, NET, SAP2 | ELK3, ETS-domain protein (SRF accessory protein 2) |
| ENC1 | ENC-1, KLHL37, NRPB, PIG10, TP53I10 | Ectodermal-neural cortex 1 (with BTB domain) |
| ENG | CD105, END, HHT1, ORW, ORW1 | Endoglin |
| ENO1 | ENO1L1, MBP-1, MPB1, PPH | Enolase 1, (alpha) |
| ENO2 | | Enolase 2 (gamma, neuronal) |
| ENPP2 | ATX, PD-IALPHA, PDNP2 | Ectonucleotide pyrophosphatase/phosphodiesterase 2 |
| EPAS1 | bHLHe73, HIF2A, HLF, MOP2, PASD2 | Endothelial PAS domain protein 1 |
| EPCAM | 17-1A, 323/A3, CD326, CO-17A, EGP-2, EGP34, EGP40, Ep-CAM, ESA, GA733-2, HEA125, KS1/4, KSA, Ly74, M4S1, MH99, MIC18, MK-1, MOC31, TACST-1, TACSTD1, TROP1 | Epithelial cell adhesion molecule |
| EPHA1 | EPH, EPHT, EPHT1 | EPH receptor A1 |
| EPHA2 | ECK | EPH receptor A2 |
| EPHA3 | ETK, ETK1, HEK, HEK4, TYRO4 | EPH receptor A3 |
| EPHA4 | Hek8, TYRO1 | EPH receptor A4 |
| EPHA7 | Hek11 | EPH receptor A7 |
| EPHA8 | EEK, Hek3 | EPH receptor A8 |
| EPHB2 | DRT, EPHT3, ERK, Hek5, Tyro5 | EPH receptor B2 |
| EPHB3 | ETK2, Hek2, Tyro6 | EPH receptor B3 |
| EPHB4 | HTK, Tyro11 | EPH receptor B4 |
| EPHX1 | EPHX | Epoxide hydrolase 1, microsomal (xenobiotic) |
| EPO | EP | Erythropoietin |
| EPOR | | Erythropoietin receptor |
| ERBB2 | CD340, HER-2, HER2, NEU, NGL | V-erb-b2 avian erythroblastic leukemia viral oncogene homolog 2 |
| ERBB3 | HER3, LCCS2 | V-erb-b2 avian erythroblastic leukemia viral oncogene homolog 3 |
| ERBB4 | ALS19 | V-erb-b2 avian erythroblastic leukemia viral oncogene homolog 4 |
| ERCC1 | RAD10 | Excision repair cross-complementation group 1 |
| ERCC2 | EM9, MAG, MGC102762, MGC126218, MGC126219, TFIIH, XPD | Excision repair cross-complementation group 2 |
| ERCC3 | BTF2, GTF2H, RAD25, TFIIH, XPB | Excision repair cross-complementation group 3 |
| ERCC4 | FANCQ, RAD1, XPF | Excision repair cross-complementation group 4 |
| ERCC5 | ERCM2, XPGC | Excision repair cross-complementation group 5 |
| ERCC6 | ARMD5, CKN2, CSB, RAD26 | Excision repair cross-complementation group 6 |
| ESR1 | Era, ESR, NR3A1 | Estrogen receptor 1 |
| ESR2 | Erb, NR3A2 | Estrogen receptor 2 (ER beta) |
| ETHE1 | HSCO, YF13H12 | Ethylmalonic encephalopathy 1 |
| ETV4 | E1A-F, E1AF, PEA3 | Ets variant 4 |
| ETV5 | ERM | Ets variant 5 |
| EXT1 | LGCR, LGS, ttv | Exostosin glycosyltransferase 1 |
| EZH2 | ENX-1, EZH1, KMT6, KMT6A | Enhancer of zeste 2 polycomb repressive complex 2 subunit |
| EZR | VIL2 | Ezrin |
| F13A1 | F13A | Coagulation factor XIII, A1 polypeptide |
| F13B | FXIIIB | Coagulation factor XIII, B polypeptide |
| F2 | | Coagulation factor II (thrombin) |
| F3 | CD142 | Coagulation factor III (thromboplastin, tissue factor) |
| FABP1 | L-FABP | Fatty acid binding protein 1, liver |
| FABP2 | I-FABP | Fatty acid binding protein 2, intestinal |
| FABP4 | A-FABP, aP2 | Fatty acid binding protein 4, adipocyte |
| FABP5 | E-FABP, KFABP, PA-FABP | Fatty acid binding protein 5 (psoriasisassociated) |
| FADD | GIG3, MORT1 | Fas (TNFRSF6)-associated via death domain |
| FAF1 | CGI-03, hFAF1, HFAF1s, UBXD12, UBXN3A | Fas (TNFRSF6) associated factor 1 |
| FAM129A | C1orf24, GIG39, NIBAN | Family with sequence similarity 129, member A |
| FAP | DPPIV | Fibroblast activation protein, alpha |
| FAS | APO-1, APT1, CD95, FAS1, TNFRSF6 | Fas cell surface death receptor |
| FASLG | APT1LG1, CD178, FasL, TNFSF6 | Fas ligand (TNF superfamily, member 6) |
| FASN | FAS, SDR27X1 | Fatty acid synthase |
| FBXO6 | FBG2, FBS2, FBX6, Fbx6b | F-box protein 6 |
| FCER2 | CD23, CD23A, CLEC4J, FCE2 | Fc fragment of IgE, low affinity II, receptor for (CD23) |
| FEN1 | FEN-1, MF1, RAD2 | Flap structure-specific endonuclease 1 |
| FES | FPS | FES proto-oncogene, tyrosine kinase |
| FGA | | Fibrinogen alpha chain |
| FGB | | Fibrinogen beta chain |
| FGF1 | AFGF, ECGF, ECGF-beta, ECGFA, ECGFB, FGF-alpha, FGFA, GLIO703, HBGF1 | Fibroblast growth factor 1 (acidic) |
| FGF17 | FGF-13 | Fibroblast growth factor 17 |
| FGF18 | FGF-18, ZFGF5 | Fibroblast growth factor 18 |
| FGF19 | | Fibroblast growth factor 19 |
| FGF2 | FGFB | Fibroblast growth factor 2 (basic) |
| FGF23 | | Fibroblast growth factor 23 |
| FGF3 | HBGF-3, INT2 | Fibroblast growth factor 3 |
| FGF4 | HBGF-4, HST, HST-1, HSTF1, K-FGF, KFGF | Fibroblast growth factor 4 |
| FGF6 | | Fibroblast growth factor 6 |
| FGF7 | KGF | Fibroblast growth factor 7 |
| FGF8 | AIGF | Fibroblast growth factor 8 (androgen-induced) |
| FGF9 | | Fibroblast growth factor 9 |
| FGFR1 | BFGFR, CD331, CEK, FLG, FLT2, H2, H3, H4, H5, KAL2, N-SAM | Fibroblast growth factor receptor 1 |
| FGFR2 | BEK, CD332, CEK3, CFD1, ECT1, JWS, K-SAM, KGFR, TK14, TK25 | Fibroblast growth factor receptor 2 |
| FGFR3 | ACH, CD333, CEK2, JTK4 | Fibroblast growth factor receptor 3 |
| FGFR4 | CD334, JTK2 | Fibroblast growth factor receptor 4 |
| FGG | | Fibrinogen gamma chain |
| FHIT | AP3Aase, FRA3B | Fragile histidine triad |
| FIGF | VEGF-D, VEGFD | C-fos induced growth factor (vascular endothelial growth factor D) |
| FKBP5 | FKBP51, FKBP54, P54, PPIase, Ptg-10 | FK506 binding protein 5 |
| FKBP8 | FKBP38, FKBPr38 | FK506 binding protein 8, 38kDa |
| FLT1 | FLT, VEGFR1 | Fms-related tyrosine kinase 1 |
| FLT4 | PCL, VEGFR3 | Fms-related tyrosine kinase 4 |
| FMO5 | | Flavin containing monooxy genase 5 |
| FN1 | CIG, FINC, GFND2, LETS, MSF | Fibronectin 1 |
| FOLH1 | FOLH, GCP2, GCPII, NAALAD1, NAALAdase, PSM, PSMA | Folate hydrolase (prostate-specific membrane antigen) 1 |
| FOS | AP-1, c-fos | FBJ murine osteosarcoma viral oncogene homolog |
| FOSL1 | fra-1 | FOS-like antigen 1 |
| FOXJ1 | FKHL13, HFH-4, HFH4 | Forkhead box J1 |
| FOXM1 | FKHL16, HFH-11, HNF-3, INS-1, MPHOSPH2, MPP2, TGT3, trident | Forkhead box M1 |
| FOXO1 | FKH1, FKHR, FOXO1A | Forkhead box O1 |
| FOXO3 | AF6q21, FKHRL1, FOXO2, FOXO3A | Forkhead box O3 |
| FOXQ1 | HFH1 | Forkhead box Q1 |
| FSCN1 | FLJ38511, p55, SNL | Fascin actin-bundling protein 1 |
| FSHB | | Follicle stimulating hormone, beta polypeptide |
| FST | FS | Follistatin |
| FTH1 | FHC, FTH, FTHL6, PIG15, PLIF | Ferritin, heavy polypeptide 1 |
| FTL | MGC71996, NBIA3 | Ferritin, light polypeptide |
| FZD1 | DKFZp564G072 | Frizzled class receptor 1 |
| FZD2 | | Frizzled class receptor 2 |
| G6PD | G6PD1 | Glucose-6-phosphate dehydrogenase |
| GADD45A | DDIT1, GADD45 | Growth arrest and DNA-damage-inducible, alpha |
| GADD45G | CR6, DDIT2, GADD45gamma, GRP17 | Growth arrest and DNA-damage-inducible, gamma |
| GAS1 | | Growth arrest-specific 1 |
| GAST | GAS | Gastrin |
| GATA3 | HDR | GATA binding protein 3 |
| GCLM | GLCLR | Glutamate-cysteine ligase, modifier subunit |
| GDF15 | MIC-1, MIC1, NAG-1, PDF, PLAB, PTGFB | Growth differentiation factor 15 |
| GDNF | ATF1, ATF2, HFB1-GDNF | Glial cell derived neurotrophic factor |
| GH1 | GH, GH-N, GHN, hGH-N | Growth hormone 1 |
| GH2 | GH-V , GH2, GHL, GHV, hGH-V | Growth hormone 2 |
| GJA1 | CX43, GJAL, ODD, ODDD, ODOD, SDTY3 | Gap junction protein, alpha 1, 43kDa |
| GJB5 | CX31.1 | Gap junction protein, beta 5, 31.1kDa |
| GLO1 | GLOD1 | Glyoxalase I |
| GMNN | Gem | Geminin, DNA replication inhibitor |
| GNAS | GNAS1, GNASXL, GPSA, NESP, NESP55, SCG6 | GNAS complex locus |
| GPA33 | A33 | Glycoprotein A33 (transmembrane) |
| GPC3 | DGSX, OCI-5, SDYS, SGB, SGBS, SGBS1 | Glypican 3 |
| GPI | AMF, NLK | Glucose-6-phosphate isomerase |
| GPX1 | | Glutathione peroxidase 1 |
| GPX2 | GSHPX-GI | Glutathione peroxidase 2 (gastrointestinal) |
| GRB10 | | Growth factor receptor-bound protein 10 |
| GRB2 | NCKAP2 | Growth factor receptor-bound protein 2 |
| GRB7 | | Growth factor receptor-bound protein 7 |
| GSK3A | | Glycogen synthase kinase 3 alpha |
| GSN | DKFZp313L0718 | Gelsolin |
| GSR | | Glutathione reductase |
| GSTM1 | GST1, H-B, MU | Glutathione S-transferase mu 1 |
| GSTM3 | GST5 | Glutathione S-transferase mu 3 (brain) |
| GSTP1 | FAEES3, GST3, GSTP | Glutathione S-transferase pi 1 |
| HDAC10 | DKFZP761B039 | Histone deacetylase 10 |
| HDAC2 | RPD3, YAF1 | Histone deacetylase 2 |
| HDAC5 | FLJ90614, KIAA0600, NY-CO-9 | Histone deacetylase 5 |
| HGF | DFNB39, F-TCF, HGFB, HPTA, SF | Hepatocyte growth factor (hepapoietin A; scatter factor) |
| HGFAC | HGFA, HGFAP | HGF activator |
| HIF1A | bHLHe78, HIF-1alpha, HIF1, MOP1, PASD8 | Hypoxia inducible factor 1, alpha subunit (basic helix-loop-helix transcription factor) |
| HIP1R | FLJ14000, HIP12, HIP3, ILWEQ, KIAA0655 | Huntingtin interacting protein 1 related |
| HIST1H2AC | H2AFL | Histone cluster 1, H2ac |
| HK1 | | Hexokinase 1 |
| HK2 | | Hexokinase 2 |
| HLA-G | | Major histocompatibility complex, class I, G |
| HMGA1 | HMGIY | High mobility group AT-hook 1 |
| HMGA2 | BABL, HMGIC, LIPO | High mobility group AT-hook 2 |
| HMOX1 | bK286B10, HO-1 | Heme oxygenase (decycling) 1 |
| HOXA5 | HOX1, HOX1C | Homeobox A5 |
| HOXA9 | HOX1, HOX1G | Homeobox A9 |
| HP | | Haptoglobin |
| HPGD | SDR36C1 | Hydroxyprostaglandin dehydrogenase 15-(NAD) |
| HPN | TMPRSS1 | Hepsin |
| HRAS | HRAS1 | Harvey rat sarcoma viral oncogene homolog |
| HSF1 | HSTF1 | Heat shock transcription factor 1 |
| HSP90AA1 | FLJ31884, Hsp89, Hsp90, HSP90N, HSPC1, HSPCA | Heat shock protein 90kDa alpha (cytosolic), class A member 1 |
| HSP90AB1 | HSPC2, HSPCB | Heat shock protein 90kDa alpha (cytosolic), class B member 1 |
| HSP90B1 | GP96, GRP94, TRA1 | Heat shock protein 90kDa beta (Grp94), member 1 |
| HSPA1A | HSP70-1, HSPA1 | Heat shock 70kDa protein 1A |
| HSPA1B | HSP70-2 | Heat shock 70kDa protein 1B |
| HSPA1L | HSP70-HOM, hum70t | Heat shock 70kDa protein 1-like |
| HSPA2 | | Heat shock 70kDa protein 2 |
| HSPA4 | HS24/P52, HSPH2 | Heat shock 70kDa protein 4 |
| HSPA8 | HSC70, HSC71, HSP73, HSPA10 | Heat shock 70kDa protein 8 |
| HSPB1 | Hs.76067, Hsp25, HSP27, HSP28 | Heat shock 27kDa protein 1 |
| HSPD1 | GROEL, HSP60, SPG13 | Heat shock 60kDa protein 1 (chaperonin) |
| HSPE1 | CPN10, GROES | Heat shock 10kDa protein 1 |
| HSPH1 | HSP105A, HSP105B, KIAA0201, NY-CO-25 | Heat shock 1 05kDa/110kDa protein 1 |
| IBSP | BSP, BSP-II, SP-II | Integrin-binding sialoprotein |
| ICAM1 | BB2, CD54 | Intercellular adhesion molecule 1 |
| ID1 | bHLHb24, dJ857M17.1.2 | Inhibitor of DNA binding 1, dominant negative helix-loop-helix protein |
| ID2 | bHLHb26, GIG8 | Inhibitor of DNA binding 2, dominant negative helix-loop-helix protein |
| ID3 | bHLHb25, HEIR-1 | Inhibitor of DNA binding 3, dominant negative helix-loop-helix protein |
| IDO1 | IDO, INDO | Indoleamine 2,3-dioxygenase 1 |
| IFNA1 | IFL, IFN, IFN-ALPHA, IFN-alphaD, IFNA13, IFNA@ | Interferon, alpha 1 |
| IFNA13 | | Interferon, alpha 13 |
| IFNAR1 | IFNAR, IFRC | Interferon (alpha, beta and omega) receptor 1 |
| IFNAR2 | IFNABR | Interferon (alpha, beta and omega) receptor 2 |
| IFNB1 | IFB, IFF, IFNB | Interferon, beta 1, fibroblast |
| IFNG | | Interferon, gamma |
| IGF1 | IGF-I, IGF1A, IGFI | Insulin-like growth factor 1 (somatomedin C) |
| IGF1R | CD221, IGFIR, IGFR, JTK13, MGC18216 | Insulin-like growth factor 1 receptor |
| IGF2 | C11orf43, FLJ44734, IGF-II | Insulin-like growth factor 2 |
| IGF2R | CD222, CIMPR, M6P-R, MPR1, MPRI | Insulin-like growth factor 2 receptor |
| IGFBP2 | IBP2 | Insulin-like growth factor binding protein 2, 36kDa |
| IGFBP3 | BP-53, IBP3 | Insulin-like growth factor binding protein 3 |
| IL10 | CSIF, IL-10, IL10A, TGIF | Interleukin 10 |
| IL11 | AGIF, IL-11 | Interleukin 11 |
| IL12A | CLMF, IL-12A, NFSK, NKSF1, p35 | Interleukin 12A |
| IL13 | ALRH, BHR1, IL-13, MGC116786, MGC116788, MGC116789, P600 | Interleukin 13 |
| IL13RA2 | CD213a2, CT19, IL-13R, IL13BP | Interleukin 13 receptor, alpha 2 |
| IL15 | IL-15, MGC9721 | Interleukin 15 |
| IL16 | FLJ16806, FLJ42735, HsT19289, IL-16, LCF, prIL-16 | Interleukin 16 |
| IL17A | CTLA8, IL-17, IL-17A, IL17 | Interleukin 17A |
| IL 17B | IL-17B, IL-20, MGC138900, MGC138901, NIRF, ZCYTO7 | Interleukin 17B |
| IL18 | IGIF, IL-18, IL-1g, IL1F4 | Interleukin 18 |
| IL1A | IL-1A, IL1, IL1-ALPHA, IL1F1 | Interleukin 1, alpha |
| IL1B | IL-1B, IL1-BETA, IL1F2 | Interleukin 1, beta |
| IL1R1 | CD121A, D2S1473, IL1R, IL1RA | Interleukin 1 receptor, type I |
| IL1R2 | CD121b, IL1RB | Interleukin 1 receptor, type II |
| IL1RN | ICIL-1RA, IL-1RN, IL1F3, IL1RA, IRAP, MGC10430 | Interleukin 1 receptor antagonist |
| IL2 | IL-2, TCGF | Interleukin 2 |
| IL24 | C49A, FISP, IL-24, IL10B, mda-7, Mob-5, ST16 | Interleukin 24 |
| IL2RA | CD25, IDDM10, IL2R | Interleukin 2 receptor, alpha |
| IL2RB | CD122, IL15RB | Interleukin 2 receptor, beta |
| IL2RG | CD132, CIDX, IMD4, SCIDX1 | Interleukin 2 receptor, gamma |
| IL4 | BCGF-1, BCGF1, BSF1, IL-4, MGC79402 | Interleukin 4 |
| IL4R | CD124 | Interleukin 4 receptor |
| IL5 | EDF, IL-5, TRF | Interleukin 5 |
| IL6 | BSF2, HGF, HSF, IFNB2, IL-6 | Interleukin 6 |
| IL6R | CD126 | Interleukin 6 receptor |
| IL6ST | CD130, GP130 | Interleukin 6 signal transducer |
| IL7 | IL-7 | Interleukin 7 |
| IL9 | HP40, IL-9, P40 | Interleukin 9 |
| ILF3 | DRBP76, MPHOSPH4, MPP4, NF90, NFAR-1 | Interleukin enhancer binding factor 3, 90kDa |
| ILK | | Integrin-linked kinase |
| INHBA | | Inhibin, beta A |
| INHBB | | Inhibin, beta B |
| INS | IDDM1, IDDM2 | Insulin |
| IRF1 | MAR | Interferon regulatory factor 1 |
| IRF4 | LSIRF, MUM1 | Interferon regulatory factor 4 |
| ITGA1 | CD49a, VLA1 | Integrin, alpha 1 |
| ITGA2 | CD49B | Integrin, alpha 2 (CD49B, alpha 2 subunit of VLA-2 receptor) |
| ITGA2B | CD41, CD41B, GP2B, PPP1R93 | Integrin, alpha 2b (platelet glycoprotein IIb of IIb/IIIa complex, antigen CD41) |
| ITGA3 | CD49c, GAP-B3, MSK18, VCA-2, VLA3a | Integrin, alpha 3 (antigen CD49C, alpha 3 subunit of VLA-3 receptor) |
| ITGA4 | CD49D | Integrin, alpha 4 (antigen CD49D, alpha 4 subunit of VLA-4 receptor) |
| ITGA5 | CD49e, FNRA | Integrin, alpha 5 (fibronectin receptor, alpha polypeptide) |
| ITGA6 | CD49f | Integrin, alpha 6 |
| ITGAM | CD11B, CR3A, MAC-1 | Integrin, alpha M (complement component 3 receptor 3 subunit) |
| ITGAV | CD51, MSK8, VNRA, VTNR | Integrin, alpha V |
| ITGB1 | CD29, FNRB, GPIIA, MDF2, MSK12 | Integrin, beta 1 (fibronectin receptor, beta polypeptide, antigen CD29 includes MDF2, MSK12) |
| ITGB3 | CD61, GP3A, GPIIIa | Integrin, beta 3 (platelet glycoprotein IIIa, antigen CD61) |
| ITGB4 | CD104 | Integrin, beta 4 |
| ITGB5 | | Integrin, beta 5 |
| ITGB6 | | Integrin, beta 6 |
| ITGB8 | | Integrin, beta 8 |
| ITIH4 | H4P, IHRP, ITIHL1 | Inter-alpha-trypsin inhibitor heavy chain family, member 4 |
| JKAMP | C14orf100, CDA06, HSPC213, HSPC327, JAMP | JNK1/MAPK8-associated membrane protein |
| JTB | hJT | Jumping translocation breakpoint |
| JUN | AP-1, c-Jun | Jun proto-oncogene |
| JUND | AP-1 | Jun D proto-oncogene |
| JUP | CTNNG, DP3, DPIII, PDGB, PKGB | Junction plakoglobin |
| KAT2B | GCN5, GCN5L, P/CAF, PCAF | K(lysine) acetyltransferase 2B |
| KDR | CD309, FLK1, VEGFR, VEGFR2 | Kinase insert domain receptor (a type III receptor tyrosine kinase) |
| KIF2A | HK2, KIF2 | Kinesin heavy chain member 2A |
| KIF2C | CT139, KNSL6, MCAK | Kinesin family member 2C |
| KISS1 | | KiSS-1 metastasis-suppressor |
| KIT | C-Kit, CD117, PBT, SCFR | V-kit Hardy-Zuckerman 4 feline sarcoma viral oncogene homolog |
| KITLG | FPH2, Kitl, KL-1, MGF, SCF, SF | KIT ligand |
| KLF4 | EZF, GKLF | Kruppel-like factor 4 (gut) |
| KLF5 | BTEB2, CKLF, IKLF | Kruppel-like factor 5 (intestinal) |
| KLK10 | NES1, PRSSL1 | Kallikrein-related peptidase 10 |
| KLK11 | PRSS20, TLSP | Kallikrein-related peptidase 11 |
| KLK13 | KLK-L4 | Kallikrein-related peptidase 13 |
| KLK14 | KLK-L6 | Kallikrein-related peptidase 14 |
| KLK15 | ACO, HSRNASPH, prostinogen | Kallikrein-related peptidase 15 |
| KLK2 | | Kallikrein-related peptidase 2 |
| KLK3 | APS, PSA | Kallikrein-related peptidase 3 |
| KLK4 | EMSP, EMSP1, KLK-L1, PRSS17, PSTS | Kallikrein-related peptidase 4 |
| KLK5 | KLK-L2, SCTE | Kallikrein-related peptidase 5 |
| KLK6 | Bssp, Klk7, neurosin, PRSS18, PRSS9 | Kallikrein-related peptidase 6 |
| KLK7 | PRSS6, SCCE | Kallikrein-related peptidase 7 |
| KLK8 | HNP, neuropsin, ovasin, PRSS19, TADG14 | Kallikrein-related peptidase 8 |
| KLRK1 | CD314, D12S2489E, KLR, NKG2-D, NKG2D | Killer cell lectin-like receptor subfamily K, member 1 |
| KRAS | KRAS1, KRAS2 | Kirsten rat sarcoma viral oncogene homolog |
| KRT13 | CK13, K13, MGC161462, MGC3781 | Keratin 13 |
| KRT14 | EBS3, EBS4 | Keratin 14 |
| KRT15 | CK15, K15, K1CO | Keratin 15 |
| KRT17 | PCHC1 | Keratin 17 |
| KRT18 | | Keratin 18 |
| KRT19 | CK19, K19, K1CS, MGC15366 | Keratin 19 |
| KRT4 | CK4, CYK4, K4 | Keratin 4 |
| KRT8 | CARD2, CK8, CYK8, K2C8, K8, KO | Keratin 8 |
| LALBA | LYZL7 | Lactalbumin, alpha- |
| LAMB1 | CLM | Laminin, beta 1 |
| LAMC1 | LAMB2 | Laminin, gamma 1 (formerly LAMB2) |
| LCN1 | MGC71975, PMFA, TLC, TP, VEGP | Lipocalin 1 |
| LDHA | | Lactate dehydrogenase A |
| LEP | OB, OBS | Leptin |
| LGALS3 | GALIG, LGALS2, MAC-2 | Lectin, galactoside-binding, soluble, 3 |
| LGALS3BP | 90K, BTBD17B, CyCAP, gp90, M2BP, MAC-2-BP, TANGO10B | Lectin, galactoside-binding, soluble, 3 binding protein |
| LGALS4 | GAL4 | Lectin, galactoside-binding, soluble, 4 |
| LGI1 | EPITEMPIN, EPT, ETL1, IB1099 | Leucine-rich, glioma inactivated 1 |
| LGMN | LGMN1, PRSC1 | Legumain |
| LHB | CGB4, hLHB, LSH-B | Luteinizing hormone beta polypeptide |
| LHX1 | LIM-1, LIM1 | LIM homeobox 1 |
| LIF | CDF, DIA, HILDA | Leukemia inhibitory factor |
| LIG4 | | Ligase IV, DNA, ATP-dependent |
| LIMK1 | LIMK | LIM domain kinase 1 |
| LMNA | CMD1A, HGPS, LGMD1B, LMN1, LMNL1, PRO1 | Lamin A/C |
| LRP1B | LRP-DIT, LRPDIT | Low density lipoprotein receptorrelated protein 1B |
| LRP6 | ADCAD2 | Low density lipoprotein receptorrelated protein 6 |
| LTA | LT, TNFB, TNFSF1 | Lymphotoxin alpha |
| LTA4H | | Leukotriene A4 hydrolase |
| LTB | p33, TNFC, TNFSF3 | Lymphotoxin beta (TNF superfamily, member 3) |
| LTBR | D12S370, TNF-R-III, TNFCR, TNFR-RP, TNFR2-RP, TNFRSF3 | Lymphotoxin beta receptor (TNFR superfamily, member 3) |
| LTF | HLF2 | Lactotransferrin |
| MAD2L1 | HSMAD2, MAD2 | MAD2 mitotic arrest deficient-like 1 (yeast) |
| MAD2L2 | MAD2B, POLZ2, REV7 | MAD2 mitotic arrest deficient-like 2 (yeast) |
| MAGEA3 | CT1.3, HIP8, HYPD, MAGE3, MGC14613 | Melanoma antigen family A, 3 |
| MAGEA4 | CT1.4, MAGE-41, MAGE-X2, MAGE4, MAGE4A, MAGE4B, MGC21336 | Melanoma antigen family A, 4 |
| MAGEA6 | CT1.6, MAGE6 | Melanoma antigen family A, 6 |
| MAGEB5 | CT3.3, MAGE-B5 | Melanoma antigen family B, 5 |
| MAGEB6 | CT3.4, FLJ40242, MAGE-B6, MAGEB6A | Melanoma antigen family B, 6 |
| MAGEC1 | CT7, CT7.1, MAGE-C1, MGC39366 | Melanoma antigen family C, 1 |
| MAGEC2 | CT10, MAGE-C2, MAGEE1 | Melanoma antigen family C, 2 |
| MAGEC3 | CT7.2, HCA2, MAGE-C3 | Melanoma antigen family C, 3 |
| MAGED1 | DLXIN-1, NRAGE | Melanoma antigen family D, 1 |
| MAGED2 | 11B6, BCG1, HCA10, JCL-1, MAGE-D2, MAGED, MGC8386 | Melanoma antigen family D, 2 |
| MAGI1 | AIP3, BAIAP1, BAP1, MAGI-1, TNRC19, WWP3 | Membrane associated guanylate kinase, WW and PDZ domain containing 1 |
| MAP2K1 | MAPKK1, MEK1, PRKMK1 | Mitogen-activated protein kinase kinase 1 |
| MAP2K2 | MEK2, PRKMK2 | Mitogen-activated protein kinase kinase 2 |
| MAP2K4 | JNKK1, MEK4, MKK4, PRKMK4, SERK1 | Mitogen-activated protein kinase kinase 4 |
| MAPK1 | ERK, ERK2, MAPK2, p41mapk, PRKM1, PRKM2 | Mitogen-activated protein kinase 1 |
| MAPK14 | CSBP1, CSBP2, CSPB1, Mxi2, p38, PRKM14, PRKM15 | Mitogen-activated protein kinase 14 |
| MAPK3 | ERK1, p44erk1, p44mapk, PRKM3 | Mitogen-activated protein kinase 3 |
| MAPK7 | BMK1, ERK5, PRKM7 | Mitogen-activated protein kinase 7 |
| MAPK8 | JNK, JNK1, PRKM8, SAPK1 | Mitogen-activated protein kinase 8 |
| MAPKAPK2 | | Mitogen-activated protein kinase-activated protein kinase 2 |
| MBD1 | CXXC3, PCM1 | Methyl-CpG binding domain protein 1 |
| MBD2 | | Methyl-CpG binding domain protein 2 |
| MBD4 | MED1 | Methyl-CpG binding domain protein 4 |
| MCL1 | BCL2L3, Mcl-1 | Myeloid cell leukemia 1 |
| MCM2 | BM28, CCNL1, cdc19, CDCL1, D3S3194, KIAA0030 | Minichromosome maintenance complex component 2 |
| MCM3 | | Minichromosome maintenance complex component 3 |
| MCM5 | CDC46 | Minichromosome maintenance complex component 5 |
| MCM7 | CDC47, MCM2, PPP1R104 | Minichromosome maintenance complex component 7 |
| MDH1 | | Malate dehydrogenase 1, NAD (soluble) |
| MDK | FLJ27379, MK, NEGF2 | Midkine (neurite growth-promoting factor 2) |
| MDM2 | HDM2, MGC5370 | MDM2 proto-oncogene, E3 ubiquitin protein ligase |
| MECP2 | MRX16, MRX79, RTT | Methyl CpG binding protein 2 |
| MED1 | CRSP1, CRSP200, DRIP230, PBP, PPARBP, PPARGBP, RB18A, TRAP220, TRIP2 | Mediator complex subunit 1 |
| MET | HGFR, RCCP2 | MET proto-oncogene, receptor tyrosine kinase |
| MFGE8 | BA46, EDIL1, hP47, HsT19888, MFG-E8, OAcGD3S, SED1, SPAG10 | Milk fat globule-EGF factor 8 protein |
| MGMT | | O-6-methylguanine-DNA methyltransferase |
| MIA | CD-RAP | Melanoma inhibitory activity |
| MIF | GIF, GLIF | Macrophage migration inhibitory factor (glycosylation-inhibiting factor) |
| MKI67 | MIB-, PPP1R105 | Marker of proliferation Ki-67 |
| MLH1 | COCA2, FCC2, HNPCC, HNPCC2 | MutL homolog 1 |
| MLLT11 | AF1Q | Myeloid/lymphoid or mixed-lineage leukemia (trithorax homolog, Drosophila); translocated to, 11 |
| MME | CALLA, CD10, NEP | Membrane metallo-endopeptidase |
| MMP1 | CLG | Matrix metallopeptidase 1 (interstitial collagenase) |
| MMP10 | STMY2 | Matrix metallopeptidase 10 (stromelysin 2) |
| MMP11 | STMY3 | Matrix metallopeptidase 11 (stromelysin 3) |
| MMP12 | HME | Matrix metallopeptidase 12 (macrophage elastase) |
| MMP13 | CLG3 | Matrix metallopeptidase 13 (collagenase 3) |
| MMP14 | MT1-MMP | Matrix metallopeptidase 14 (membrane-inserted) |
| MMP15 | MT2-MMP, MTMMP2, SMCP-2 | Matrix metallopeptidase 15 (membrane-inserted) |
| MMP16 | C8orf57, DKFZp761D112, MT3-MMP | Matrix metallopeptidase 16 (membrane-inserted) |
| MMP2 | CLG4, CLG4A, TBE-1 | Matrix metallopeptidase 2 (gelatinase A, 72kDa gelatinase, 72kDa type IV collagenase) |
| MMP3 | STMY, STMY1 | Matrix metallopeptidase 3 (stromelysin 1, progelatinase) |
| MMP7 | MPSL1, PUMP-1 | Matrix metallopeptidase 7 (matrilysin, uterine) |
| MMP8 | CLG1 | Matrix metallopeptidase 8 (neutrophil collagenase) |
| MMP9 | CLG4B | Matrix metallopeptidase 9 (gelatinase B, 92kDa gelatinase, 92kDa type IV collagenase) |
| MPO | | Myeloperoxidase |
| MRE11A | ATLD, MRE11 | MRE11 meiotic recombination 11 homolog A (S. cerevisiae) |
| MSH6 | GTBP | MutS homolog 6 |
| MSLN | CAK1, MPF | Mesothelin |
| MSMB | IGBF, MSP, MSPB, PN44, PRPS, PSP, PSP-94, PSP57, PSP94 | Microseminoprotein, beta- |
| MSR1 | CD204, SCARA1 | Macrophage scavenger receptor 1 |
| MT1A | MT1, MT1S | Metallothionein 1A |
| MT1G | MT1, MT1K | Metallothionein 1G |
| MTA1 | | Metastasis associated 1 |
| MUC1 | ADMCKD, ADMCKD1, CD227, MCD, MCKD, MCKD1, PEM, PUM | Mucin 1, cell surface associated |
| MUTYH | MYH | MutY homolog |
| MVP | LRP, VAULT1 | Major vault protein |
| MXI1 | bHLHc11, MAD2, MXD2, MXI | MAX interactor 1, dimerization protein |
| MYBL2 | B-MYB, BMYB | V-myb avian myeloblastosis viral oncogene homolog-like 2 |
| MYC | bHLHe39, c-Myc, MYCC | V-myc avian myelocytomatosis viral oncogene homolog |
| MYOCD | MYCD | Myocardin |
| MYOD1 | bHLHc1, MYF3, MYOD, PUM | Myogenic differentiation 1 |
| MYOG | bHLHc3, MYF4 | Myogenin (myogenic factor 4) |
| NAGA | D22S674 | N-acetylealactosaminidase, alpha- |
| NAIP | BIRC1, NLRB1 | NLR family, apoptosis inhibitory protein |
| NAMPT | PBEF, PBEF1 | Nicotinamide phosphoribosyltransferase |
| NAT2 | AAC2 | N-acetyltransferase 2 (arylamine N-acetyltransferase) |
| NCAM1 | CD56, NCAM | Neural cell adhesion molecule 1 |
| NCOA3 | ACTR, AIB1, bHLHe42, CAGH16, KAT13B, p/CIP, RAC3, SRC-3, SRC3, TNRC16, TRAM-1 | Nuclear receptor coactivator 3 |
| NDRG1 | CAP43, DRG1, NDR1, RTP, TDD5 | N-myc downstream regulated 1 |
| NEDD8 | Nedd-8 | Neural precursor cell expressed, developmentally down-regulated 8 |
| NEO1 | HsT17534, IGDCC2, NGN, NTN1R2 | Neogenin 1 |
| NFKB1 | KBF1, NF-kappaB, NF-kB1, NFkappaB, NFKB-p50, p105, p50 | Nuclear factor of kappa light polypeptide gene enhancer in B-cells 1 |
| NFKB2 | LYT-10, NF-kB2, p105, p52 | Nuclear factor of kappa light polypeptide gene enhancer in B-cells 2 (p49/p100) |
| NFKBIA | IkappaBalpha, IKBA, MAD-3, NFKBI | Nuclear factor of kappa light polypeptide gene enhancer in B-cells inhibitor, alpha |
| NFKBIE | IKBE | Nuclear factor of kappa light polypeptide gene enhancer in B-cells inhibitor, epsilon |
| NGF | NGFB | Nerve growth factor (beta polypeptide) |
| NGFR | CD271, p75NTR, TNFRSF16 | Nerve growth factor receptor |
| NKX3-1 | BAPX2, NKX3.1, NKX3A | NK3 homeobox 1 |
| NME1 | NDPKA, NM23, NM23-H1 | NME/NM23 nucleoside diphosphate kinase 1 |
| NME2 | NDPKB, NM23-H2 | NME/NM23 nucleoside diphosphate kinase 2 |
| NOS1 | nNOS, NOS | Nitric oxide synthase 1 (neuronal) |
| NOS2 | HEP-NOS, iNOS, NOS, NOS2A | Nitric oxide synthase 2, inducible |
| NOS3 | ECNOS, eNOS | Nitric oxide synthase 3 (endothelial cell) |
| NOTCH1 | TAN1 | Notch 1 |
| NOTCH2 | | Notch 2 |
| NOTCH3 | CADASIL, CASIL | Notch 3 |
| NQO1 | DHQU, DIA4, DTD, NMOR1, QR1 | NAD(P)H dehydrogenase, quinone 1 |
| NR0B1 | AHC, AHCH, DAX1, DSS, NR0B1 | Nuclear receptor subfamily 0, group B, member 1 |
| NRG1 | GGF, HGL, HRG, NDF, NRG1-IT2 | Neuregulin 1 |
| NRG2 | Don-1, HRG2, NTAK | Neuregulin 2 |
| NRG3 | | Neuregulin 3 |
| NRP1 | CD304, NRP, VEGF165R | Neuropilin 1 |
| NRP2 | VEGF165R2 | Neuropilin 2 |
| NTF3 | NGF2 | Neurotrophin 3 |
| NTF4 | GLC1O, NT-4/5, NTF5 | Neurotrophin 4 |
| NTHL1 | NTH1, OCTS3 | Nth endonuclease III-like 1 (E. coli) |
| NTN1 | NTN1L | Netrin 1 |
| NTRK1 | MTC, TRK, TRKA | Neurotrophic tyrosine kinase, receptor, type 1 |
| NTRK2 | TRKB | Neurotrophic tyrosine kinase, receptor, type 2 |
| NTRK3 | TRKC | Neurotrophic tyrosine kinase, receptor, type 3 |
| NUDT1 | MTH1 | Nudix (nucleoside diphosphate linked moiety X)-type motif 1 |
| NUMB | C14orf41 | Numb homolog (Drosophila) |
| OGG1 | HMMH, HOGG1, MUTM, OGH1 | 8-oxoguanine DNA glycosylase |
| OR51E2 | PSGR | Olfactory receptor, family 51, subfamily E, member 2 |
| ORM1 | | Orosomucoid 1 |
| OSM | MGC20461 | Oncostatin M |
| PAGE4 | CT16.7, GAGEC1, PAGE-4 | P antigen family, member 4 (prostate associated) |
| PAPPA | ASBABP2, DIPLA1, IGFBP-4ase, PAPA, PAPPA, PAPPA1 | Pregnancy-associated plasma protein A, pappalysin 1 |
| PARP1 | ADPRT, PARP, PPOL | Poly (ADP-ribose) polymerase 1 |
| PARVB | CGI-56 | Parvin, beta |
| PAX5 | BSAP | Paired box 5 |
| PAX8 | | Paired box 8 |
| PCNA | | Proliferating cell nuclear antigen |
| PDGFA | PDGF-A, PDGF1 | Platelet-derived growth factor alpha polypeptide |
| PDGFB | SIS, SSV | Platelet-derived growth factor beta polypeptide |
| PDGFRA | CD140a, PDGFR2 | Platelet-derived growth factor receptor, alpha polypeptide |
| PDGFRB | CD140b, JTK12, PDGFR, PDGFR1 | Platelet-derived growth factor receptor, beta polypeptide |
| PDZD4 | FLJ34125, KIAA1444, LU1, PDZK4, PDZRN4L | PDZ domain containing 4 |
| PF4 | CXCL4, SCYB4 | Platelet factor 4 |
| PGC | | Progastricsin (pepsinogen C) |
| PGF | D12S1900, PGFL, PLGF, PlGF-2, SHGC-10760 | Placental growth factor |
| PGR | NR3C3, PR | Progesterone receptor |
| PHF20 | C20orf104, dJ1121G12.1, TDRD20A | PHD finger protein 20 |
| PIGR | | Polymeric immunoglobulin receptor |
| PIK3CA | PI3K | Phosphatidylinositol-4,5-bisphosphate 3-kinase, catalytic subunit alpha |
| PIK3R1 | GRB1, p85, p85-ALPHA | Phosphoinositide-3-kinase, regulatory subunit 1 (alpha) |
| PIK3R2 | p85, P85B | Phosphoinositide-3-kinase, regulatory subunit 2 (beta) |
| PIK3R3 | p55 | Phosphoinositide-3-kinase, regulatory subunit 3 (gamma) |
| PIM1 | PIM | Pim-1 proto-oncogene, serine/threonine kinase |
| PIM2 | | Pim-2 proto-oncogene, serine/threonine kinase |
| PIM3 | | Pim-3 proto-oncogene, serine/threonine kinase |
| PIN1 | dod | Peptidylprolyl cis/trans isomerase, NIMA-interacting 1 |
| PIP4K2B | PIP5K2B, PIP5KIIB, PIP5KIIbeta | Phosphatidylinositol-5-phosphate 4-kinase, type II, beta |
| PKM | OIP3, PK3, PKM2, THBP1 | Pyruvate kinase, muscle |
| PLAT | | Plasminogen activator, tissue |
| PLAU | UPA, URK | Plasminogen activator, urokinase |
| PLAUR | CD87, UPAR, URKR | Plasminogen activator, urokinase receptor |
| PLG | | Plasminogen |
| PLK1 | PLK | Polo-like kinase 1 |
| PLP1 | GPM6C, PLP, SPG2 | Proteolipid protein 1 |
| PMEPA1 | STAG1, TMEPAI | Prostate transmembrane protein, androgen induced 1 |
| PML | MYL, RNF71, TRIM19 | Promyelocytic leukemia |
| PMP22 | GAS-3, HNPP, Sp110 | Peripheral myelin protein 22 |
| PNMT | PENT | Phenylethanolamine N-methyltransferase |
| POMC | ACTH, CLIP, LPH, MSH, NPP, POC | Proopiomelanocortin |
| PON1 | ESA, PON | Paraoxonase 1 |
| POSTN | OSF-2, periostin, PN | Periostin, osteoblast specific factor |
| POU2F2 | OCT2, OTF2 | POU class 2 homeobox 2 |
| PPA2 | FLJ20459 | Pyrophosphatase (inorganic) 2 |
| PPARG | NR1C3, PPARG1, PPARG2, PPARgamma | Peroxisome proliferator-activated receptor gamma |
| PPARGC1A | PGC1, PGC1A, PPARGC1 | Peroxisome proliferator-activated receptor gamma, coactivator 1 alpha |
| PPM1D | PP2C-DELTA, Wip1 | Protein phosphatase, Mg2+/Mn2+ dependent, 1D |
| PPP1R15A | GADD34 | Protein phosphatase 1, regulatory subunit 15A |
| PPY | PNP | Pancreatic polypeptide |
| PRDM13 | | PR domain containing 13 |
| PRDM16 | KIAA1675, MEL1, MGC166915, PFM13 | PR domain containing 16 |
| PRDX2 | MGC4104, NKEFB, PRP, PRX2, PRXII, TDPX1, TSA | Peroxiredoxin 2 |
| PRDX4 | AOE37-2 | Peroxiredoxin 4 |
| PRKCA | PKCA | Protein kinase C, alpha |
| PRKCB | PKCB, PRKCB1, PRKCB2 | Protein kinase C, beta |
| PRKCE | | Protein kinase C, epsilon |
| PRKCH | PKC-L, PKCL, PRKCL | Protein kinase C, eta |
| PRKCI | DXS1179E, PKCI | Protein kinase C, iota |
| PRKCQ | | Protein kinase C, theta |
| PRKDC | DNA-PKcs, DNAPK, DNPK1, HYRC, HYRC1, p350, XRCC7 | Protein kinase, DNA-activated, catalytic polypeptide |
| PRL | | Prolactin |
| PROC | | Protein C (inactivator of coagulation factors Va and VIIIa) |
| PRSS1 | TRY1 | Protease, serine, 1 (trypsin 1) |
| PSCA | | Prostate stem cell antigen |
| PSMD4 | AF, AF-1, Rpn10, S5A | Proteasome (prosome, macropain) 26S subunit, non-ATPase, 4 |
| PTCH1 | BCNS, NBCCS, PTCH | Patched 1 |
| PTCH2 | | Patched 2 |
| PTGS1 | COX1, PGHS-1, PTGHS | Prostaglandin-endoperoxide synthase 1 (prostaglandin G/H synthase and cyclooxygenase) |
| PTGS2 | COX2 | Prostaglandin-endoperoxide synthase 2 (prostaglandin G/H synthase and cyclooxygenase) |
| PTH | PTH1 | Parathyroid hormone |
| PTHLH | HHM, PLP, PTHR, PTHRP | Parathyroid hormone-like hormone |
| PTK2 | FADK, FAK, FAK1, PPP1R71 | Protein tyrosine kinase 2 |
| PTN | HBGF8, HBNF, NEGF1 | Pleiotrophin |
| PTPRO | GLEPP1, NPHS6, PTP-oc, PTP-U2, PTPU2 | Protein tyrosine phosphatase, receptor type, O |
| PTTG1 | EAP1, HPTTG, PTTG, securin, TUTR1 | Pituitary tumor-transforming 1 |
| PURA | PUR-ALPHA, PUR1, PURALPHA | Purine-rich element binding protein A |
| PZP | CPAMD6 | Pregnancy-zone protein |
| RAB11FIP3 | eferin, KIAA0665, Rab11-FIP3 | RAB11 family interacting protein 3 (class II) |
| RAB18 | | RAB18, member RAS oncogene family |
| RAB25 | CATX-8 | RAB25, member RAS oncogene family |
| RAC1 | p21-Rac1, Rac-1, TC-25 | Ras-related C3 botulinum toxin substrate 1 (rho family, small GTP binding protein Rac1) |
| RAD23A | HHR23A, MGC111083 | RAD23 homolog A (S. cerevisiae) |
| RAD23B | HHR23B, HR23B, P58 | RAD23 homolog B (S. cerevisiae) |
| RAD51 | BRCC5, HsRad51, HsT16930, RAD51A, RECA | RAD51 recombinase |
| RAD51D | HsTRAD, R51H3, RAD51L3, Trad | RAD51 paralog D |
| RAD52 | | RAD52 homolog (S. cerevisiae) |
| RAD54B | RDH54 | RAD54 homolog B (S. cerevisiae) |
| RAF1 | c-Raf, CRAF, Raf-1 | Raf-1 proto-oncogene, serine/threonine kinase |
| RARA | NR1B1, RAR | Retinoic acid receptor, alpha |
| RARB | HAP, NR1B2, RRB2 | Retinoic acid receptor, beta |
| RARG | NR1B3, RARC | Retinoic acid receptor, gamma |
| RASA1 | CM-AVM, GAP, p120GAP, p120RASGAP, RASA | RAS p21 protein activator (GTPase activating protein) 1 |
| RB1 | OSRC, PPP1R130, RB | Retinoblastoma 1 |
| RBBP4 | lin-53, NURF55, RbAp48 | Retinoblastoma binding protein 4 |
| RBL1 | cp107, p107, PRB1 | Retinoblastoma-like 1 |
| RBL2 | p130, Rb2 | Retinoblastoma-like 2 |
| RBM6 | 3G2, DEF-3, DEF3, g16, NY-LU-12 | RNA binding motif protein 6 |
| RBP4 | | Retinol binding protein 4, plasma |
| REL | c-Rel, I-Rel | V-rel avian reticuloendotheliosis viral oncogene homolog |
| RELA | NFKB3, p65 | V-rel avian reticuloendotheliosis viral oncogene homolog A |
| RELB | REL-B | V-rel avian reticuloendotheliosis viral oncogene homolog B |
| RET | CDHF12, CDHR16, HSCR1, MEN2A, MEN2B, MTC1, PTC, RET51 | Ret proto-oncogene |
| RHOA | ARH12, ARHA, Rho12, RhoA, RHOH12 | Ras homolog family member A |
| RHOB | ARH6, ARHB, MST081, RhoB, RHOH6 | Ras homolog family member B |
| RHOC | ARH9, ARHC, RhoC | Ras homolog family member C |
| RPA2 | | Replication protein A2, 32kDa |
| RPL27 | L27 | Ribosomal protein L27 |
| RPS3 | FLJ26283, FLJ27450, MGC87870, S3 | Ribosomal protein S3 |
| RPS6KA1 | HU-1, RSK, RSK1 | Ribosomal protein S6 kinase, 90kDa, polypeptide 1 |
| RPS6KA3 | CLS, HU-3, MRX19, RSK, RSK2 | Ribosomal protein S6 kinase, 90kDa, polypeptide 3 |
| RXRA | NR2B1 | Retinoid X receptor, alpha |
| RXRB | H-2RIIBP, NR2B2, RCoR-1 | Retinoid X receptor, beta |
| RXRG | NR2B3 | Retinoid X receptor, gamma |
| S100A1 | S100-alpha, S100A | S100 calcium binding protein A1 |
| S100A2 | CAN19, S100L | S100 calcium binding protein A2 |
| S100A4 | 18A2, 42A, CAPL, FSP1, MTS1, P9KA, PEL98 | S100 calcium binding protein A4 |
| S100A6 | 2A9, CABP, CACY, PRA | S100 calcium binding protein A6 |
| S100A7 | PSOR1, S100A7c | S100 calcium binding protein A7 |
| S100A8 | 60B8AG, CAGA, CFAG, CGLA, MRP8, P8 | S100 calcium binding protein A8 |
| S100A9 | 60B8AG, CAGB, CFAG, CGLB, LIAG, MAC387, MIF, MRP14, NIF, P14 | S100 calcium binding protein A9 |
| S100B | S100beta | S100 calcium binding protein B |
| S1PR1 | CD363, D1S3362, edg-1, EDG1 | Sphingosine-1-phosphate receptor 1 |
| SAA1 | PIG4, SAA, TP53I4 | Serum amyloid A1 |
| SAA2 | | Serum amyloid A2 |
| SART1 | Ara1, SNRNP110, Snu66 | Squamous cell carcinoma antigen recognized by T cells |
| SCGB1A1 | CC10, CC16, CCSP, UGB | Secretoglobin, family 1A, member 1 (uteroglobin) |
| SCGB1D2 | LIPB, LPHB | Secretoglobin, family 1D, member 2 |
| SCGB2A1 | LPHC, MGB2, MGC71973, UGB3 | Secretoglobin, family 2A, member 1 |
| SCGB2A2 | MGB1, MGC71974, UGB2 | Secretoglobin, family 2A, member 2 |
| SDC1 | CD138, SDC, SYND1, syndecan | Syndecan 1 |
| SELE | CD62E, ELAM, ELAM1, ESEL | Selectin E |
| SELL | CD62L, hLHRc, LAM-1, LAM1, Leu-8, LNHR, LSEL, Lyam-1, LYAM1, PLNHR | Selectin L |
| SELP | CD62, CD62P, GMP140, GRMP, PADGEM, PSEL | Selectin P (granule membrane protein 140kDa, antigen CD62) |
| SEMA3B | LUCA-1, SemA, sema5, SEMAA, semaV | Sema domain, immunoglobulin domain (Ig), short basic domain, secreted, (semaphorin) 3B |
| 2-Sep | DIFF6, hNedd5, KIAA0158, NEDD5, Pnutl3 | Septin 2 |
| SERPINA1 | A1A, A1AT, AAT, alpha-1-antitrypsin, alpha1AT, PI, PI1 | Serpin peptidase inhibitor, clade A (alpha-1 antiproteinase, antitrypsin), member 1 |
| SERPINA3 | AACT, ACT, alpha-1-antichymotrypsin | Serpin peptidase inhibitor, clade A (alpha-1 antiproteinase, antitrypsin), member 3 |
| SERPINA5 | PAI3, PCI, PLANH3, PROCI | Serpin peptidase inhibitor, clade A (alpha-1 antiproteinase, antitrypsin), member 5 |
| SERPINB2 | HsT1201, PAI2, PLANH2 | Serpin peptidase inhibitor, clade B (ovalbumin), member 2 |
| SERPINB3 | HsT1196, SCC, SCCA1, T4-A | Serpin peptidase inhibitor, clade B (ovalbumin), member 3 |
| SERPINB4 | LEUPIN, PI11, SCCA-2, SCCA1, SCCA2 | Serpin peptidase inhibitor, clade B (ovalbumin), member 4 |
| SERPINE1 | PAI, PAI1, PLANH1 | Serpin peptidase inhibitor, clade E (nexin, plasminogen activator inhibitor type 1), member 1 |
| SERPINF1 | EPC-1, PEDF, PIG35 | Serpin peptidase inhibitor, clade F (alpha-2 antiplasmin, pigment epithelium derived factor), member 1 |
| SFN | YWHAS | Stratifin |
| SHBG | ABP, MGC126834, MGC138391, TEBG | Sex hormone-binding globulin |
| SIRT2 | SIR2L | Sirtuin 2 |
| SKP2 | FBL1, FBXL1, p45 | S-phase kinase-associated protein 2, E3 ubiquitin protein ligase |
| SLC19A1 | FOLT | Solute carrier family 19 (folate transporter), member 1 |
| SLC2A1 | DYT18, GLUT, GLUT1, HTLVR | Solute carrier family 2 (facilitated glucose transporter), member 1 |
| SLC3A2 | 4F2, 4F2HC, 4T2HC, CD98, CD98HC, MDU1, NACAE | Solute carrier family 3 (amino acid transporter heavy chain), member 2 |
| SLPI | ALK1, ALP, BLPI, HUSI, HUSI-I, WAP4, WFDC4 | Secretory leukocyte peptidase inhibitor |
| SMAD1 | JV4-1, MADH1, MADR1 | SMAD family member 1 |
| SMAD2 | JV18-1, MADH2, MADR2 | SMAD family member 2 |
| SMAD3 | HsT17436, JV15-2, MADH3 | SMAD family member 3 |
| SMAD4 | DPC4, MADH4 | SMAD family member 4 |
| SMYD3 | KMT3E, ZMYND1, ZNFN3A1 | SET and MYND domain containing 3 |
| SOD1 | ALS, ALS1, IPOA | Superoxide dismutase 1, soluble |
| SOD2 | | Superoxide dismutase 2, mitochondrial |
| SOX1 | | SRY (sex determining region Y)-box 1 |
| SOX9 | CMD1, CMPD1, SRA1 | SRY (sex determinino region Y)-box 9 |
| SP1 | | Sp1 transcription factor |
| SPARC | ON | Secreted protein, acidic, cysteine-rich (osteonectin) |
| SPARCL1 | MAST9 | SPARC-like 1 (hevin) |
| SPINK1 | PCTT, PSTI, Spink3, TATI | Serine peptidase inhibitor, Kazal type 1 |
| SPINT1 | HAI, MANSC2 | Serine peptidase inhibitor, Kunitz type 1 |
| SPINT2 | HAI-2, Kop | Serine peptidase inhibitor, Kunitz type, 2 |
| SPP1 | BNSP, BSPI, ETA-1, OPN | Secreted phosphoprotein 1 |
| SPRR1B | GADD33, SPRR1 | Small proline-rich protein 1B |
| SPRR3 | | Small proline-rich protein 3 |
| SPRY1 | hSPRY1 | Sprouty homolog 1, antagonist of FGF signaling (Drosophila) |
| SRC | ASV, c-src, SRC1 | SRC proto-oncogene, non-receptor tyrosine kinase |
| SRD5A1 | | Steroid-5-alpha-reductase, alpha polypeptide 1 (3-oxo-5 alpha-steroid delta 4-dehydrogenase alpha 1) |
| SRD5A2 | | Steroid-5-alpha-reductase, alpha polypeptide 2 (3-oxo-5 alpha-steroid delta 4-dehydrogenase alpha 2) |
| SST | SMST | Somatostatin |
| SSX2 | CT5.2a, HD21, HOM-MEL-40, MGC119055, MGC15364, MGC3884, SSX | Synovial sarcoma, X breakpoint 2 |
| SSX2B | CT5.2b | Synovial sarcoma, X breakpoint 2B |
| ST14 | HAI, MT-SP1, PRSS14, SNC19, TMPRSS14 | Suppression of tumorigenicity 14 (colon carcinoma) |
| STARD3 | es64, MLN64 | StAR-related lipid transfer (START) domain containing 3 |
| STAT4 | | Signal transducer and activator of transcription 4 |
| STAT5A | MGF, STAT5 | Signal transducer and activator of transcription 5A |
| STEAP1 | PRSS24, STEAP | Six transmembrane epithelial antigen of the prostate 1 |
| STMN1 | C1orf215, FLJ32206, Lag, LAP18, OP18, PP17, PP19, PR22, SMN | Stathmin 1 |
| STRAP | MAWD, pt-wd, UNRIP | Serine/threonine kinase receptor associated protein |
| STT3A | ITM1, MGC9042, STT3-A, TMC | STT3A, subunit of the oligosaccharyltransferase complex (catalytic) |
| SULT1E1 | EST, STE | Sulfotransferase family 1E, estrogen-preferring, member 1 |
| TAGLN | DKFZp686P11 128, SM22, SMCC, TAGLN1, WS3-10 | Transgelin |
| TDRD6 | bA446F17.4, CT41.2, NY-CO-45, SPATA36 | Tudor domain containing 6 |
| TEK | CD202b, TIE-2, TIE2, VMCM, VMCM1 | TEK tyrosine kinase, endothelial |
| TERT | EST2, hEST2, TCS1, TP2, TRT | Telomerase reverse transcriptase |
| TF | PRO1557, PRO2086 | Transferrin |
| TFAP2B | AP2-B | Transcription factor AP-2 beta (activating enhancer binding protein 2 beta) |
| TFDP1 | Dp-1, DP1, DRTF1 | Transcription factor Dp-1 |
| TFDP2 | Dp-2 | Transcription factor Dp-2 (E2F dimerization partner 2) |
| TFF1 | BCEI, D21S21, HP1.A, HPS2, pNR-2, pS2 | Trefoil factor 1 |
| TFF2 | SML1 | Trefoil factor 2 |
| TFF3 | HITF, ITF | Trefoil factor 3 (intestinal) |
| TFRC | CD71, p90, TFR1 | Transferrin receptor |
| TG | AITD3, TGN | Thyroglobulin |
| TGFA | | Transforming growth factor, alpha |
| TGFB1 | CED, DPD1, TGFB, TGFbeta | Transforming growth factor, beta 1 |
| TGFB2 | | Transforming growth factor, beta 2 |
| TGFB3 | ARVD, ARVD1 | Transforming growth factor, beta 3 |
| TGFBR3 | betaglycan, BGCAN | Transforming growth factor, beta receptor III |
| TGM4 | TGP | Transglutaminase 4 |
| TGM7 | TGMZ | Transglutaminase 7 |
| THBS1 | THBS, THBS-1, TSP, TSP-1, TSP1 | Thrombospondin 1 |
| THBS2 | TSP2 | Thrombospondin 2 |
| THBS4 | | Thrombospondin 4 |
| THPO | MGDF, MPLLG, TPO | Thrombopoietin |
| THRA | AR7, EAR-7.1/EAR-7.2, ERBA, ERBA1, NR1A1, THRA1, THRA2, THRA3 | Thyroid hormone receptor, alpha |
| THRB | ERBA-BETA, ERBA2, GRTH, NR1A2, PRTH, THR1, THRB1, THRB2 | Thyroid hormone receptor, beta |
| TIE1 | JTK14, TIE | Tyrosine kinase with immunoglobulin-like and EGF-like domains 1 |
| TIMP1 | CLGI, EPO, TIMP | TIMP metallopeptidase inhibitor 1 |
| TIMP2 | CSC-21K | TIMP metallopeptidase inhibitor 2 |
| TIMP3 | SFD | TIMP metallopeptidase inhibitor 3 |
| TK1 | | Thymidine kinase 1, soluble |
| TMF1 | ARA160, TMF | TATA element modulatory factor 1 |
| TMPRSS2 | PRSS10 | Transmembrane protease, serine 2 |
| TMPRSS3 | DFNB10, DFNB8 | Transmembrane protease, serine 3 |
| TNC | DFNA56, HXB, MGC167029, TN | Tenascin C |
| TNF | DIF, TNF-alpha, TNFA, TNFSF2 | Tumor necrosis factor |
| TNFAIP2 | B94, EXOC3L3 | Tumor necrosis factor, alpha-induced protein 2 |
| TNFAIP3 | A20, OTUD7C | Tumor necrosis factor, alpha-induced protein 3 |
| TNFRSF10A | Apo2, CD261, DR4, TRAILR-1 | Tumor necrosis factor receptor superfamily, member 10a |
| TNFRSF10B | CD262, DR5, KILLER, TRAIL-R2, TRICK2A, TRICKB | Tumor necrosis factor receptor superfamily, member 10b |
| TNFRSF10C | CD263, DcR1, LIT, TRAILR3, TRID | Tumor necrosis factor receptor superfamily, member 10c, decoy without an intracellular domain |
| TNFRSF10D | CD264, DcR2, TRAILR4, TRUNDD | Tumor necrosis factor receptor superfamily, member 10d, decoy with truncated death domain |
| TNFRSF11B | OCIF, OPG, TR1 | Tumor necrosis factor receptor superfamily, member 11b |
| TNFRSF12A | CD266, FN14, TweakR | Tumor necrosis factor receptor superfamily, member 12A |
| TNFRSF14 | ATAR, CD270, HVEA, HVEM, LIGHTR, TR2 | Tumor necrosis factor receptor superfamily, member 14 |
| TNFRSF1A | CD120a, TNF-R, TNF-R-I, TNF-R55, TNFAR, TNFR1, TNFR60 | Tumor necrosis factor receptor superfamily, member 1A |
| TNFRSF1B | CD120b, p75, TNF-R-II, TNF-R75, TNFBR, TNFR2, TNFR80 | Tumor necrosis factor receptor superfamily, member 1B |
| TNFRSF4 | ACT35, CD134, OX40, TXGP1L | Tumor necrosis factor receptor superfamily, member 4 |
| TNFRSF8 | CD30, D1S166E, KI-1 | Tumor necrosis factor receptor superfamily, member 8 |
| TNFRSF9 | 4-1BB, CD137, ILA | Tumor necrosis factor receptor superfamily, member 9 |
| TNFSF10 | Apo-2L, CD253, TL2, TRAIL | Tumor necrosis factor (ligand) superfamily, member 10 |
| TNFSF11 | CD254, ODF, OPGL, RANKL, TRANCE | Tumor necrosis factor (ligand) superfamily, member 11 |
| TNFSF13 | APRIL, CD256 | Tumor necrosis factor (ligand) superfamily, member 13 |
| TNFSF13B | BAFF, BLYS, CD257, TALL-1, TALL1, THANK, TNFSF20 | Tumor necrosis factor (ligand) superfamily, member 13b |
| TNFSF4 | CD252, gp34, OX-40L, TXGP1 | Tumor necrosis factor (ligand) superfamily, member 4 |
| TNFSF8 | CD153, CD30LG | Tumor necrosis factor (ligand) superfamily, member 8 |
| TNK2 | ACK, ACK1, p21cdc42Hs | Tyrosine kinase, non-receptor, 2 |
| TOP2A | TOP2 | Topoisomerase (DNA) II alpha 170kDa |
| TP53 | LFS1, p53 | Tumor protein p53 |
| TP53BP2 | 53BP2, ASPP2, PPP1R13A | Tumor protein p53 binding protein 2 |
| TPD52 | D52, hD52, N8L | Tumor protein D52 |
| TPI1 | | Triosephosphate isomerase 1 |
| TPM1 | C15orf13, CMH3 | Tropomyosin 1 (alpha) |
| TPM2 | AMCD1, DA1, NEM4 | Tropomyosin 2 (beta) |
| TPX2 | C20orf1, C20orf2, DIL-2, p100 | TPX2, microtubule-associated |
| TRAF1 | EBI6 | TNF receptor-associated factor 1 |
| TRAF2 | TRAP3 | TNF receptor-associated factor 2 |
| TRAF4 | CART1, MLN62, RNF83 | TNF receptor-associated factor 4 |
| TRIM25 | EFP, RNF147, ZNF147 | Tripartite motif containing 25 |
| TRIP4 | HsT17391, ZC2HC5 | Thyroid hormone receptor interactor 4 |
| TRO | KIAA1114, MAGE-D3, MAGED3 | Trophinin |
| TSG101 | TSG10, VPS23 | Tumor susceptibility 101 |
| TSPAN8 | CO-029, TM4SF3 | Tetraspanin 8 |
| TSPO | BZRP, DBI, IBP, MBR, mDRC, PBR, pk18, PKBS | Translocator protein (18kDa) |
| TTR | CTS, CTS1, HsT2651, PALB | Transthyretin |
| TUSC2 | C3orf11, FUS1, PAP, PDAP2 | Tumor suppressor candidate 2 |
| TWIST1 | ACS3, bHLHa38, BPES2, BPES3, CRS, CRS1, H-twist, SCS, TWIST | Twist family bHLH transcription factor 1 |
| TXLNA | DKFZp451J0118 | Taxilin alpha |
| TYMP | ECGF1, MNGIE | Thymidine phosphorylase |
| TYMS | HsT422, TMS, TS, Tsase | Thymidylate synthetase |
| TYRO3 | Brt, Dtk, RSE, Sky, Tif | TYRO3 protein tyrosine kinase |
| UBA1 | A1S9T, CFAP124, GXP1, POC20, UBE1, UBE1X | Ubiquitin-like modifier activating enzyme 1 |
| UBE2C | UBCH10 | Ubiquitin-conjugating enzyme E2C |
| UBE2I | UBC9 | Ubiquitin-conjugating enzyme E2I |
| UBE2N | MGC8489, UBC13, UbcH-ben | Ubiquitin-conjugating enzyme E2N |
| UGT1A10 | UGT1J | UDP glucuronosyltransferase 1 family, polypeptide A10 |
| UGT1A3 | UGT1C | UDP glucuronosyltransferase 1 family, polypeptide A3 |
| UGT1A4 | HUG-BR2, UGT1D | UDP glucuronosyltransferase 1 family, polypeptide A4 |
| UGT1A8 | UGT1H | UDP glucuronosyltransferase 1 family, polypeptide A8 |
| UGT1A9 | HLUGP4, LUGP4, UGT1AI | UDP glucuronosyltransferase 1 family, polypeptide A9 |
| USH1C | AIE-75, DFNB18, harmonin, NY-CO-37, NY-CO-38, PDZ-73, PDZ73, PDZD7C | Usher syndrome 1C (autosomal recessive, severe) |
| VAMP3 | CEB | Vesicle-associated membrane protein 3 |
| VCAM1 | CD106 | Vascular cell adhesion molecule 1 |
| VEGFA | VEGF, VEGF-A, VPF | Vascular endothelial growth factor A |
| VEGFB | VEGFL, VRF | Vascular endothelial growth factor B |
| VEGFC | VRP | Vascular endothelial growth factor C |
| VHL | VHL1 | Von Hippel-Lindau tumor suppressor, E3 ubiquitin protein ligase |
| VIL1 | D2S1471, VIL | Villin 1 |
| VIP | | Vasoactive intestinal peptide |
| VTN | VN | Vitronectin |
| VWF | F8VWF | Von Willebrand factor |
| WEE1 | | WEE1 G2 checkpoint kinase |
| WFDC2 | dJ461P17.6, EDDM4, HE4, WAP5 | WAP four-disulfide core domain 2 |
| WISP1 | CCN4 | WNT1 inducible signaling pathway protein 1 |
| WNT1 | INT1 | Wingless-type MMTV integration site family, member 1 |
| WNT2 | INTIL1, IRP | Wingless-type MMTV integration site family member 2 |
| WRN | RECQ3, RECQL2 | Werner syndrome, RecQ helicase-like |
| WT1 | AWT1, GUD, WAGR, WIT-2 | Wilms tumor 1 |
| XBP1 | XBP2 | X-box binding protein 1 |
| XIAP | API3, BIRC4, hILP | X-linked inhibitor of apoptosis |
| XPA | XP1, XPAC | Xeroderma pigmentosum, complementation group A |
| XPC | RAD4, XPCC | Xeroderma pigmentosum, complementation group C |
| XRCC2 | | X-ray repair complementing defective repair in Chinese hamster cells 2 |
| XRCC3 | | X-ray repair complementing defective repair in Chinese hamster cells 3 |
| XRCC4 | | X-ray repair complementing defective repair in Chinese hamster cells 4 |
| XRCC5 | KARP-1, KU80, Ku86, KUB2 | X-ray repair complementing defective repair in Chinese hamster cells 5 (double-strand-break rejoining) |
| XRCC6 | D22S671, D22S731, G22P1, KU70, ML8 | X-ray repair complementing defective repair in Chinese hamster cells 6 |
| YBX1 | BP-8, CSDA2, CSDB, DBPB, MDR-NF1, NSEP-1, NSEP1, YB-1, YB1 | Y box binding protein 1 |
| YWHAB | YWHAA | Tyrosine 3-monooxygenase/tryptophan 5-monooxygenase activation protein, beta |
| YWHAE | FLJ45465 | Tyrosine 3-monooxygenase/tryptophan 5-monooxygenase activation protein, epsilon |
| YWHAH | YWHA1 | Tyrosine 3-monooxygenase/tryptophan 5-monooxygenase activation protein, eta |
| ZBTB16 | PLZF, ZNF145 | Zinc finger and BTB domain containing 16 |
| ZMAT3 | FLJ12296, MGC10613, PAG608, WIG-1, WIG1 | Zinc finger, matrin-type 3 |

In one embodiment, the biomarker is MYC. In one embodiment, the measurable aspect of MYC is its expression status. In one embodiment, the biomarker is overexpression of MYC.

Thus, in certain aspects of the disclosure, the biomarker is MYC which is differentially present in a subject of one phenotypic status, e.g., a patient having cancer, e.g., hepatocellular carcinoma (HCC), glioblastomas (GBM), lung cancer, breast cancer, head and neck cancer, prostate cancer, melanoma, or colorectal cancer, as compared with another phenotypic status, e.g., a normal undiseased subject or a patient having cancer without overexpression MYC.

In one embodiment, the biomarker is MCL1. In one embodiment, the measurable aspect of MCL1 is its expression status. In one embodiment, the biomarker is overexpression of MCL1.

A biomarker is differentially present between different phenotypic status groups if the mean or median expression or mutation levels of the biomarker is calculated to be different, i.e., higher or lower, between the groups. Thus, biomarkers provide an indication that a subject, e.g., a cancer patient, belongs to one phenotypic status or another.

Thus, in certain aspects of the disclosure, the biomarker is MCL1 which is differentially present, i.e., overexpressed, in a subject of one phenotypic status, e.g., a patient having cancer, e.g., hepatocellular carcinoma (HCC), glioblastomas (GBM), lung cancer, breast cancer, head and neck cancer, prostate cancer, melanoma, colorectal cancer, medulloblastoma, or general brain tumors, as compared with another phenotypic status, e.g., an undiseased patient or a cancer patient without overexpression MCL1.

In addition to individual biological compounds, e.g., MYC or MCL1, the term "biomarker" as used herein is meant to include groups, sets, or arrays of multiple biological compounds. For example, the combination of MYC and MCL1 may comprise a biomarker. The term "biomarker" may comprise one, two, three, four, five, six, seven, eight, nine, ten, fifteen, twenty, twenty five, thirty, or more, biological compounds.

The determination of the expression level or mutation status of a biomarker in a patient can be performed using any of the many methods known in the art. Any method known in the art for quantitating specific proteins and/or detecting MYC and/or MCL1 expression, or the expression or mutation levels of any other biomarker in a patient or a biological sample may be used in the methods of the disclosure. Examples include, but are not limited to, PCR (polymerase chain reaction), or RT-PCR, Northern blot, Western blot, ELISA (enzyme linked immunosorbent assay), RIA (radioimmunoassay), gene chip analysis of RNA expression, immunohistochemistry or immunofluorescence. *See, e.g.,* Slagle et al. Cancer 83:1401 (1998). Certain embodiments of the disclosure include methods wherein biomarker RNA expression (transcription) is determined. Other embodiments of the disclosure include methods wherein protein expression in the biological sample is determined. See, for example, Harlow et al., Antibodies: A Laboratory Manual, Cold Spring Harbor Laboratory, Cold Spring Harbor, NY, (1988) and Ausubel et al., Current Protocols in Molecular Biology, John Wiley & Sons, New York 3rd Edition, (1995). For northern blot or RT-PCR analysis, RNA is isolated from the tumor tissue sample using RNAse free techniques. Such techniques are commonly known in the art.

In one embodiment of the disclosure, a biological sample is obtained from the patient and cells in the biopsy are assayed for determination of biomarker expression or mutation status.

In one embodiment of the disclosure, PET imaging is used to determine biomarker expression.

In another embodiment of the disclosure, Northern blot analysis of biomarker transcription in a tumor cell sample is performed. Northern analysis is a standard method for detection and/or quantitation of mRNA levels in a sample. Initially, RNA is isolated from a sample to be assayed using Northern blot analysis. In the analysis, the RNA samples are first separated by size via electrophoresis in an agarose gel under denaturing conditions. The RNA is then transferred to a membrane, crosslinked and hybridized with a labeled probe. Typically, Northern hybridization involves polymerizing radiolabeled or nonisotopically labeled DNA, in vitro, or generation of oligonucleotides as hybridization probes. Typically, the membrane holding the RNA sample is prehybridized or blocked prior to probe hybridization to prevent the probe from coating the membrane and, thus, to reduce non-specific background signal. After hybridization, typically, unhybridized probe is removed by washing in several changes of buffer. Stringency of the wash and hybridization conditions can be designed, selected and implemented by any practitioner of ordinary skill in the art. Detection is accomplished using detectably labeled probes and a suitable detection method. Radiolabeled and non-radiolabled probes and their use are well known in the art. The presence and or relative levels of expression of the biomarker being assayed can be quantified using, for example, densitometry.

In another embodiment of the disclosure, biomarker expression and/or mutation status is determined using RT-PCR. RT-PCR allows detection of the progress of a PCR amplification of a target gene in real time. Design of the primers and probes required to detect expression and/or mutation status of a biomarker of the disclosure is within the skill of a practitioner of ordinary skill in the art. RT-PCR can be used to determine the level of RNA encoding a biomarker of the disclosure in a tumor tissue sample. In an embodiment of the disclosure, RNA from the biological sample is isolated, under RNAse free conditions, than converted to DNA by treatment with reverse transcriptase. Methods for reverse transcriptase conversion of RNA to DNA are well known in the art. A description of PCR is provided in the following references: Mullis et al., Cold Spring Harbor Symp. Quant. Biol. 51:263 (1986); EP 50,424; EP 84,796; EP 258,017; EP 237,362; EP 201,184; U.S. Patent Nos. 4,683,202; 4,582,788; 4,683,194.

RT-PCR probes depend on the 5'-3' nuclease activity of the DNA polymerase used for PCR to hydrolyze an oligonucleotide that is hybridized to the target amplicon (biomarker gene). RT-PCR probes are oligonucleotides that have a fluorescent reporter dye attached to the 5, end and a quencher moiety coupled to the 3' end (or vice versa). These probes are designed to hybridize to an internal region of a PCR product. In the unhybridized state, the proximity of the fluor and the quench molecules prevents the detection of fluorescent signal from the probe. During PCR amplification, when the polymerase replicates a template on which an RT-PCR probe is bound, the 5'-3' nuclease activity of the polymerase cleaves the probe. This decouples the fluorescent and quenching dyes and FRET no longer occurs. Thus, fluorescence increases in each cycle, in a manner proportional to the amount of probe cleavage. Fluorescence signal emitted from the reaction can be measured or followed over time using equipment which is commercially available using routine and conventional techniques.

In another embodiment of the disclosure, expression of proteins encoded by biomarkers are detected by western blot analysis. A western blot (also known as an immunoblot) is a method for protein detection in a given sample of tissue homogenate or extract. It uses gel electrophoresis to separate denatured proteins by mass. The proteins are then transferred out of the gel and onto a membrane (*e.g*., nitrocellulose or polyvinylidene fluoride (PVDF)), where they are detected using a primary antibody that specifically bind to the protein. The bound antibody can then detected by a secondary antibody that is conjugated with a detectable label (*e.g*., biotin, horseradish peroxidase or alkaline phosphatase). Detection of the secondary label signal indicates the presence of the protein.

In another embodiment of the disclosure, the expression of a protein encoded by a biomarker is detected by enzyme-linked immunosorbent assay (ELISA). In one embodiment of the disclosure, "sandwich ELISA" comprises coating a plate with a capture antibody; adding sample wherein any antigen present binds to the capture antibody; adding a detecting antibody which also binds the antigen; adding an enzyme-linked secondary antibody which binds to detecting antibody; and adding substrate which is converted by an enzyme on the secondary antibody to a detectable form. Detection of the signal from the secondary antibody indicates presence of the biomarker antigen protein.

In another embodiment of the disclosure, the expression of a biomarker is evaluated by use of a gene chip or microarray. Such techniques are within ordinary skill held in the art.

### V. Definitions

The term "TG02" as used herein refers to (16E)-14-methyl-20-oxa-5,7,14,26-tetraazatetracyclo[19.3.1.1(2,6).1(8,12)]heptacosa-1(25),2(26),3,5,8(27),9,11,16,21,23-decaene.

The term "TG02 free base" or "TG02 FB" refers to the free base of (16E)-14-methyl-20-oxa-5,7,14,26-tetraazatetracyclo[19.3.1.1(2,6).1(8,12)]heptacosa-1(25),2(26),3,5,8(27),9,11,16,21,23-decaene.

The term "TG02 acid addition salt" or "TG02 salt" refers to a pharmaceutically acceptable acid addition salt of (16E)-14-methyl-20-oxa-5,7,14,26-tetraazatetracyclo[19.3.1.1(2,6).1(8,12)]heptacosa-1(25),2(26),3,5,8(27),9,11,16,21,23-decaene. Examples of acids which can be employed to form pharmaceutically acceptable acid addition salts include inorganic acids such as nitric, boric, hydrochloric, hydrobromic, sulfuric, and phosphoric, and organic acids such as oxalic, maleic, succinic, and citric. Nonlimiting examples of salts of TG02 include, but are not limited to, the hydrochloride, hydrobromide, hydroiodide, sulfate, bisulfate, 2-hydroxyethansulfonate, phosphate, hydrogen phosphate, acetate, adipate, alginate, aspartate, benzoate, bisulfate, butyrate, camphorate, camphorsulfonate, digluconate, glycerolphsphate, hemisulfate, heptanoate, hexanoate, formate, succinate, fumarate, maleate, ascorbate, isethionate, salicylate, methanesulfonate, mesitylenesulfonate, naphthylenesulfonate, nicotinate, 2-naphthalenesulfonate, oxalate, pamoate, pectinate, persulfate, 3-phenylproprionate, picrate, pivalate, propionate, trichloroacetate, trifluoroacetate, phosphate, glutamate, bicarbonate, paratoluenesulfonate, undecanoate, lactate, citrate, tartrate, gluconate, methanesulfonate, ethanedisulfonate, benzene sulfonate, and p-toluenesulfonate salts.

The term "TG02 citrate" or "TG02 CA" refers to the citrate salt of (16E)-14-methyl-20-oxa-5,7,14,26-tetraazatetracyclo[19.3.1.1(2,6).1(8,12)]heptacosa-1(25),2(26),3,5,8(27),9,11,16,21,23-decaene. This is also known as (16E)-14-methyl-20-oxa-5,7,14,26-tetraazatetracyclo[19.3.1.1(2,6).1(8,12)]heptacosa-1(25),2(26),3,5,8(27),9,11,16,21,23-decaene - citric acid.

The term "biological sample" as used herein refers any tissue or fluid from a patient that is suitable for detecting a biomarker, such as MYC and/or MCL1 expression status. Examples of useful biological samples include, but are not limited to, biopsied tissues and/or cells, *e.g.,* solid tumor, lymph gland, inflamed tissue, tissue and/or cells involved in a condition or disease, blood, plasma, serous fluid, cerebrospinal fluid, saliva, urine, lymph, cerebral spinal fluid, and the like. Other suitable biological samples will be familiar to those of ordinary skill in the relevant arts. A biological sample can be analyzed for biomarker expression and/or mutation using any technique known in the art and can be obtained using techniques that are well within the scope of ordinary knowledge of a clinical practioner. In one embodiment of the disclosure, the biological sample comprises blood cells.

The terms "a", "an", "the", and similar referents in the context of describing the disclosure (especially in the context of the claims) are to be construed to cover both the singular and the plural, unless otherwise indicated. Recitation of ranges of values herein merely are intended to serve as a shorthand method of referring individually to each separate value falling within the range, unless otherwise indicated herein, and each separate value is incorporated into the specification as if it were individually recited herein. The use of any and all examples, or exemplary language, e.g., "such as," provided herein, is intended to better illustrate the disclosure and is not a limitation on the scope of the disclosure unless otherwise claimed. No language in the specification should be construed as indicating any non-claimed element as essential to the practice of the disclosure.

The term "about," as used herein, includes the recited number ± 10%. Thus, "about 10" means 9 to 11.

As used herein, the term "substantially pure" with reference to a particular TG02 polymorphic form means that the polymorphic form comprises about 10% or less, *e.g.,* about 9%, about 8%, about 7%, about 6%, about 5%, about 4%, about 3%, about 2%, or about 1%, or less, by weight, of any other physical, e.g., crystalline and/or amorphous, forms of TG02.

As used herein, the term "pure" with reference to a particular TG02 polymorphic form means that the polymorphic form comprises about 1% or less, *e.g*., about 0.9%, about 0.8%, about 0.7%, about 0.6%, about 0.5%, about 0.4%, about 0.3%, about 0.2%, or about 0.1%, or less, by weight, of any other physical forms of TG02. In one embodiment, a "pure" polymorphic form contains no PXRD-detectable amount of any other physical forms of TG02.

As used herein, the term "amorphous" refers to a solid form of TG02 that lacks the long-range order characteristic of a crystal, *i.e.,* the solid is non-crystalline.

As used herein, the term "essentially the same" with reference to PXRD peak positions and relative intensities means that peak position and intensity variability are taken into account when comparing PXRD diffractograms. PXRD peak positions can show inter-apparatus variability as much as ± 0.2° and be "essentially the same." Relative peak intensities can also show inter-apparatus variability due to degree of crystallinity, orientation, prepared sample surface, and other factors known to those skilled in the art, and should be taken as qualitative measures only.

As used herein, the term "micronization" refers to a process or method by which the size of a population of particles is reduced, typically to the micron scale.

As used herein, the term "micron" or "µm" refer to "micrometer," which is 1 x 10⁻⁶ meter.

As used herein, the term "average particle size distribution" or "D₅₀" is the diameter where 50 mass-% of the particles have a larger equivalent diameter, and the other 50 mass-% have a smaller equivalent diameter as determined by laser diffraction using Malvern Master Sizer Microplus equipment or its equivalent.

As used herein, the terms "treat," "treating," "treatment," and the like refer to eliminating, reducing, or ameliorating a disease or condition, and/or symptoms associated therewith. Although not precluded, treating a disease or condition does not require that the disease, condition, or symptoms associated therewith be completely eliminated. However, in one embodiment, administration of a TG02 polymorphic form and/or an immune checkpoint inhibitor leads to complete remission of the cancer.

The term "therapeutically effective amount," as used herein, refers to that amount of the therapeutic agent sufficient to result in amelioration of one or more symptoms of a disorder, or prevent advancement of a disorder, or cause regression of the disorder. For example, with respect to the treatment of cancer, in one embodiment, a therapeutically effective amount will refer to the amount of a therapeutic agent that causes a therapeutic response, e.g., normalization of blood counts, decrease in the rate of tumor growth, decrease in tumor mass, decrease in the number of metastases, increase in time to tumor progression, and/or increase patient survival time by at least about 2%. In other embodiments, a therapeutically effective amount will refer to the amount of a therapeutic agent that causes a therapeutic response of at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or at least about 100%, or more.

The term "pharmaceutically acceptable carrier" or "pharmaceutically acceptable vehicle" encompasses any of the standard pharmaceutical carriers, solvents, surfactants, or vehicles. Suitable pharmaceutically acceptable vehicles include aqueous vehicles and nonaqueous vehicles. Standard pharmaceutical carriers and their formulations are described in Remington's Pharmaceutical Sciences, Mack Publishing Co., Easton, PA, 19th ed. 1995.

The term "container" means any receptacle and closure therefore suitable for storing, shipping, dispensing, and/or handling a pharmaceutical product.

The term "insert" means information accompanying a pharmaceutical product that provides a description of how to administer the product, along with the safety and efficacy data required to allow the physician, pharmacist, and patient to make an informed decision regarding use of the product. The package insert generally is regarded as the "label" for a pharmaceutical product.

"Concurrent administration," "administered in combination," "simultaneous administration," and similar phrases mean that two or more agents are administered concurrently to the subject being treated. By "concurrently," it is meant that each agent is administered either simultaneously or sequentially in any order at different points in time. However, if not administered simultaneously, it is meant that they are administered to an individual in a sequence and sufficiently close in time so as to provide the desired therapeutic effect and act in concert. For example, the TG02 citrate salt polymorphic form can be administered at the same time or sequentially in any order at different points in time as the anti-PD-1 antibody, anti-PD-L1 antibody, or temozolomide and/or the optional therapeutic agent. The TG02 citrate salt polymorphic form and the anti-PD-1 antibody, anti-PD-L1 antibody, or temozolomide and/or the optional therapeutic agent can be administered separately, in any appropriate form and by any suitable route. When the TG02 citrate salt polymorphic form and the anti-PD-1 antibody, anti-PD-L1 antibody, or temozolomide and/or the optional therapeutic agent are not administered concurrently, it is understood that they can be administered in any order to a patient in need thereof. For example, the TG02 citrate salt polymorphic form can be administered prior to (e.g., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (e.g., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the anti-PD-1 antibody, anti-PD-L1 antibody, or temozolomide, to an individual in need thereof. In various embodiments, the TG02 citrate salt polymorphic form and the anti-PD-1 antibody, anti-PD-L1 antibody, or temozolomide are administered 1 minute apart, 10 minutes apart, 30 minutes apart, less than 1 hour apart, 1 hour apart, 1 hour to 2 hours apart, 2 hours to 3 hours apart, 3 hours to 4 hours apart, 4 hours to 5 hours apart, 5 hours to 6 hours apart, 6 hours to 7 hours apart, 7 hours to 8 hours apart, 8 hours to 9 hours apart, 9 hours to 10 hours apart, 10 hours to 11 hours apart, 11 hours to 12 hours apart, no more than 24 hours apart or no more than 48 hours apart. In one embodiment, the components of the combination therapies are administered at about 1 minute to about 24 hours apart. In one embodiment, the TG02 citrate salt polymorphic form is administered 3-7 days prior to the day the anti-PD-1 antibody, anti-PD-L1 antibody, or temozolomide is administered. In another embodiment, the TG02 citrate salt polymorphic form is also administered on the day the anti-PD-1 antibody, anti-PD-L1 antibody, or temozolomide is administered and continues to be administered until disease progression or TG02 therapy is no longer beneficial.

### EXAMPLES

### General Instrument and Methodology Details

### X-ray Powder Diffraction

X-ray Powder Diffraction (XRPD or PXRD) diffractograms were collected on a Bruker AXS C2 GADDS, Bruker AXS DB Advance, PANalytical Empyrean, or similar diffractometer using Cu Kα radiation.

### Bruker AXS C2 GADDS diffractometer

XRPD diffractograms were collected on a Bruker AXS C2 GADDS diffractometer using Cu Kα radiation (40 kV, 40 rnA), an automated XYZ stage, a laser video microscope for auto-sample positioning and a Vantec-500 2-dimensional area detector. X-ray optics consists of a single Gobel multilayer mirror coupled with a pinhole collimator of 0.3 mm.

The beam divergence, i.e. the effective size of the X-ray beam on the sample, was approximately 4 mm. A Θ-Θ continuous scan mode was employed with a sample detector distance of 20 ecm which gives an effective 2Θ range of 1.5° - 32.5°. Typically, the sample was exposed to the X-ray beam for 120 seconds. The software used for data collection and analysis was GADDS for Win7/XP and Diffrac *Plus* EVA, respectively.

Samples run under ambient conditions were prepared as flat plate specimens using powder as received without grinding. Samples were prepared and analysed on either a glass slide or glass frit. Samples were lightly pressed onto a glass slide to obtain a flat surface for analysis. A glass frit filter block was used to isolate and analyse solids from suspensions by adding a small amount of suspension directly to the glass frit before filtration under a light vacuum.

For variable temperature (VT) experiments samples were mounted on an Anton Paar DHS 900 hot stage at ambient conditions. The sample was then heated to the appropriate temperature at 20 °C/min and subsequently held isothermally for 1 minute before data collection was initiated. Samples were prepared and analysed on a silicon wafer mounted to the hot stage using a heat-conducting compound.

### Bruker AXS DB Advance diffractometer

XRPD diffractograms were collected on a Bruker D8 diffractometer using Cu Kα radiation (40 kV, 40 rnA) and a 8-28 goniometer fitted with aGe monochromator. The incident beam passes through a 2.0 mm divergence slit followed by a 0.2 mm anti-scatter slit and knife edge. The diffracted beam passes through an 8.0 mm receiving slit with 2.5° Soller slits followed by the Lynxeye Detector. The software used for data collection and analysis was Diffrac *Plus* XRD Commander and Diffrac *Plus* EVA respectively.

Samples were run under ambient conditions as flat plate spectmens using powder as received. The sample was prepared on a polished, zero-background (510) silicon wafer by gently pressing onto the flat surface or packed into a cut cavity. The sample was rotated in its own plane.

The details of the standard data collection method are: Angular range: 2 to 42° 2Θ; Step size: 0.05° 2 Θ; and Collection time: 0.5 s/step (total collection time: 6.40 min).

### PANalytical Empyrean diffractometer

XRPD diffractograms were collected on a PANalytical Empyrean diffractometer using Cu Kα radiation (45 kV, 40 rnA) in transmission geometry. A 0.5° slit, 4 mm mask and 0.04 rad Soller slits with a focusing mirror were used on the incident beam. A PIXcel^{3D} detector, placed on the diffracted beam, was fitted with a receiving slit and 0.04 rad Soller slits. The software used for data collection was X'Pert Data Collector using X'Pert Operator Interface. The data were analysed and presented using Diffrac *Plus* EVA or HighScore Plus.

Samples were prepared and analysed in either a metal or Millipore 96 well-plate in transmission mode. X-ray transparent film was used between the metal sheets on the metal well-plate and powders (approximately 1 - 2 mg) were used as received. The Millipore plate was used to isolate and analyse solids from suspensions by adding a small amount of suspension directly to the plate before filtration under a light vacuum.

The scan mode for the metal plate used the gonic scan axis, whereas a 28 scan was utilised for the Millipore plate.

The details of the standard screening data collection method are: Angular range: 2.5 to 32.0° 2Θ; Step size: 0.0130° 2Θ; and Collection time: 12.75 s/step (total collection time of 2.07 min).

The software used for data collection was X'Pert Data Collector and the data were analysed and presented using Diffrac *Plus* EVA or Highscore Plus.

Samples were prepared and analysed in an Anton Paar chromed sample holder.

### Nuclear Magnetic Resonance (NMR)

¹H NMR spectra were collected on a Bruker 400 MHz instrument equipped with an auto-sampler and controlled by a DRX400 console. Samples were prepared in DMSO-d6 solvent, unless otherwise stated. Automated experiments were acquired using ICON-NMR configuration within Topspin software, using standard Brukerloaded experiments (¹H, ¹³C {¹H}, DEPT135). Off-line analysis was performed using ACD Spectrus Processor.

### Differential Scanning Calorimetry (DSC)

DSC data were collected on a TA Instruments Q2000 equipped with a 50 position auto-sampler. Typically, 0.5 - 1.5 mg of each sample, in a pin-holed aluminium pan, was heated at either 2 °C/min or 10 °C/min from 25 °C to 250 °C. A purge of dry nitrogen at 50 ml/min was maintained over the sample. The instrument control software was Advantage for Q Series and Thermal Advantage and the data were analysed using Universal Analysis or TRIOS.

### Thermo-Gravimetric Analysis (TGA)

TGA data were collected on a TA Instruments Q500 TGA, equipped with a 16 position auto-sampler. Typically, 3 - 6 mg of each sample was loaded onto a pre-tared aluminium DSC pan and heated at 10 °C/min from ambient temperature to 350 °C. A nitrogen purge at 60 ml/min was maintained over the sample.

The instrument control software was Advantage for Q Series and Thermal Advantage and the data were analysed using Universal Analysis or TRIOS.

### Polarised Light Microscopy (PLM)

Samples were analysed on a Leica LM/DM polarised light microscope with a digital video camera for image capture. A small amount of each sample was placed on a glass slide, with or without immersion oil, and covered with a glass slip. The sample was viewed with appropriate magnification and partially polarised light, coupled to a λ falsecolour filter. Images were captured using StudioCapture or Image ProPlus software.

### Gravimetric Vapour Sorption (GVS)

Sorption isotherms were obtained using a SMS DVS Intrinsic moisture sorption analyser, controlled by DVS Intrinsic Control software. The sample temperature was maintained at 25 °C by the instrument controls. The humidity was controlled by mixing streams of dry and wet nitrogen, with a total flow rate of 200 ml/min. The relative humidity was measured by a calibrated Rotronic probe (dynamic range of 1.0 - 100 %RH), located near the sample. The weight change, (mass relaxation) of the sample as a function of % RH was constantly monitored by a microbalance (accuracy ±0.005 mg).

Typically, 5 - 30 mg of sample was placed in a tared mesh stainless steel basket under ambient conditions. The sample was loaded and unloaded at 40 % RH and 25 °C (typical room conditions). A moisture sorption isotherm was performed as outlined below (2 scans per complete cycle). The standard isotherm was performed at 25 °C at 10 % RH intervals over a 0 - 90 % RH range. Typically, a double cycle (4 scans) was carried out. Data analysis was carried out within Microsoft Excel using the DVS Analysis Suite.

The sample was recovered after completion of the isotherm and re-analysed by XRPD.

### Water Determination by Karl Fischer Titration (KF)

The water content of each sample was measured on a Metrohm 874 Oven Sample Processor at 150 °C with 851 Titrano Coulometer using Hydranal Coulomat AG oven reagent and nitrogen purge. Weighed solid samples were introduced into a sealed sample vial. Approximately 10 mg of sample was used per titration and duplicate determinations were made. An average of these results is presented unless otherwise stated. Data collection and analysis were performed using Tiamo software.

### Thermodynamic Aqueous Solubility

Aqueous solubility was determined by suspending sufficient compound in relevant media to give a maximum final concentration of ≥10 mg/ml of the parent freeform of the compound. The suspension was equilibrated at 25 °C, on a Heidolph plate shaker set to 750 rpm for 24 hours. The pH of the saturated solution was then measured and the suspension filtered through a glass fibre C filter (particle retention 1.2 µm) and diluted appropriately. Quantitation was by HPLC with reference to a standard solution of approximately 0.15 mg/ml in DMSO. Different volumes of the standard, diluted and undiluted sample solutions were injected. The solubility was calculated using the peak areas determined by integration of the peak found at the same retention time as the principal peak in the standard injection.

### Light Stability Trials

Solid and liquid samples were exposed to accelerated stress conditions using an Atlas CPS+ light box. Samples were prepared for analysis in duplicate in clear glass vials with closed lids for the liquid samples and open vials for the solid samples. Two of the vials were exposed to light conditions and the other vial was wrapped in aluminium foil to act as reference material. The sample thickness of solid samples was no more than ~3 mm.

Exposure to light was effected by a combination of a single quartz glass filter with two window glass filters to reduce the effects of UV light upon the test samples. Temperature effects on the samples were reduced by attaching a chiller unit to the light box. Analysis of the samples was performed by HPLC

### COMPARATIVE EXAMPLE 1

### Preparation of TG02 free base polymorphic forms

### Comparative Example: Form I (FB)

Preparation: NaHCO₃ (aq) was added to a mixture containing TG02·2HCl and DCM adjusting pH to 8. The separated organic layer was concentrated till near dryness to give TG02 Form I (FB).

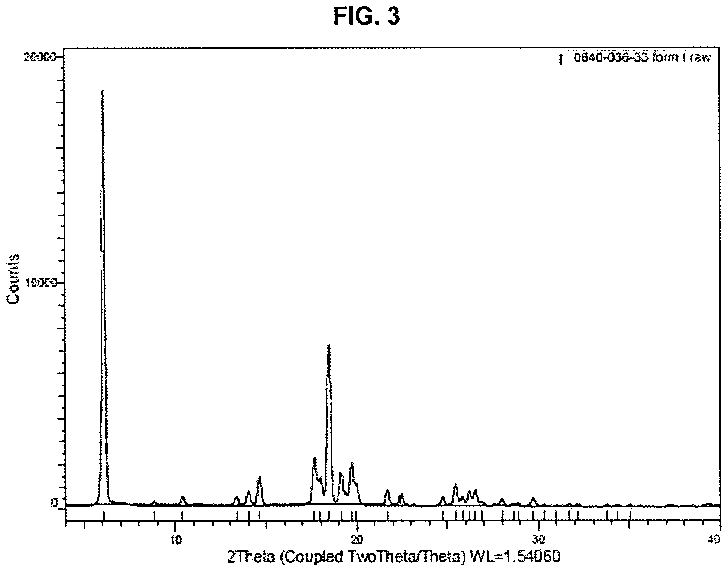

Characterization: The PXRD of TG02 Form I (FB) is shown in Fig. 3. Table 3 lists the peak positions, d values, and relative peak intensities of TG02 Form I (FB).

**Table 3**

| **Index** | **Angle (2-Theta °)** | **d Value (Å)** | **Relative Intensity** |
|---|---|---|---|
| 1 | 6.077 | 14.53102 | 100.0 % |
| 2 | 8.840 | 9.99553 | 0.8 % |
| 3 | 10.404 | 8.49596 | 2.2 % |
| 4 | 13.368 | 6.61832 | 2.0 % |
| 5 | 14.031 | 6.30672 | 3.7% |
| 6 | 14.628 | 6.05083 | 7.0 % |
| 7 | 17.675 | 5.01390 | 11.6 % |
| 8 | 17.994 | 4.92580 | 6.5 % |
| 9 | 18.475 | 4.79867 | 38.5 % |
| 10 | 19.135 | 4.63446 | 7.2 % |
| 11 | 19.727 | 4.49685 | 10.1 % |
| 12 | 19.913 | 4.45515 | 5.4 % |
| 13 | 21.698 | 4.09257 | 3.9 % |
| 14 | 22.460 | 3.95543 | 3.1 % |
| 15 | 24.749 | 3.59448 | 1.9 % |
| 16 | 25.456 | 3.49626 | 5.3 % |
| 17 | 25.833 | 3.44601 | 2.2 % |
| 18 | 26.209 | 3.39751 | 3.6 % |
| 19 | 26.527 | 3.35744 | 4.0 % |
| 20 | 26.882 | 3.31396 | 0.8 % |
| 21 | 28.004 | 3.18361 | 1.6 % |
| 22 | 28.625 | 3.11602 | 0.5 % |
| 23 | 28.857 | 3.09142 | 0.7 % |
| 24 | 29.725 | 3.00315 | 1.9 % |
| 25 | 30.305 | 2.94692 | 0.4 % |
| 26 | 31.009 | 2.88160 | 0.3 % |
| 27 | 31.689 | 2.82135 | 0.8 % |
| 28 | 32.160 | 2.78109 | 0.6 % |
| 29 | 33.741 | 2.65431 | 0.5 % |
| 30 | 34.293 | 2.61283 | 0.4 % |
| 31 | 35.029 | 2.55957 | 0.5 % |

### Comparative Example: Form II (FB)

Preparation: K₂CO₃ (aq) was added to a solution containing TG02·HCl and MeOH at 40-60°C adjusting pH to 8-9. The product was filtered and dried to give TG02 Form II (FB).

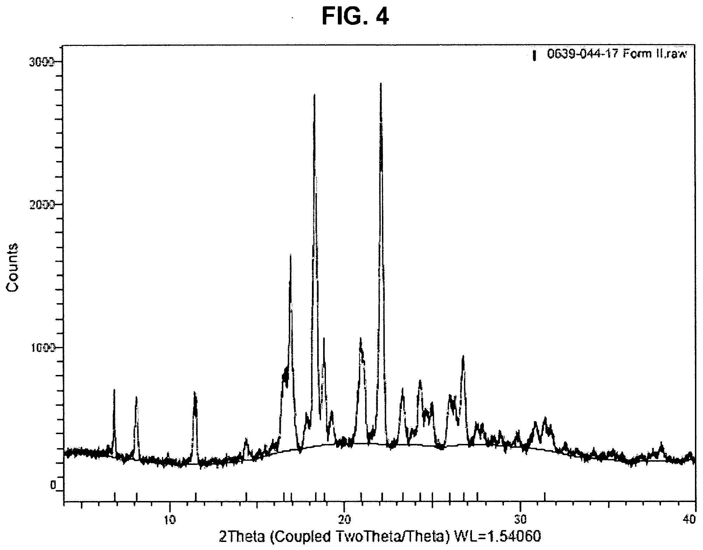

Characterization: The PXRD of TG02 Form II (FB) is shown in Fig. 4. Table 4 lists the peak positions, d values, and relative peak intensities of TG02 Form II (FB).

**Table 4**

| **Index** | **Angle (2-Theta °)** | **d Value (Å)** | **Relative Intensity** |
|---|---|---|---|
| 1 | 6.025 | 14.65812 | 1.4 % |
| 2 | 6.954 | 12.70150 | 7.9 % |
| 3 | 8.238 | 10.72461 | 100.0 % |
| 4 | 10.036 | 8.80626 | 0.7 % |
| 5 | 11.607 | 7.61760 | 25.9 % |
| 6 | 14.563 | 6.07746 | 1.0 % |
| 7 | 15.299 | 5.78667 | 0.5 % |
| 8 | 16.683 | 5.30984 | 22.1 % |
| 9 | 17.153 | 5.16540 | 26.0 % |
| 10 | 18.064 | 4.90672 | 1.6 % |
| 11 | 18.546 | 4.78043 | 10.1 % |
| 12 | 19.073 | 4.64946 | 40.0 % |
| 13 | 21.013 | 4.22436 | 2.5 % |
| 14 | 21.294 | 4.16916 | 4.6 % |
| 15 | 22.342 | 3.97608 | 12.7 % |
| 16 | 23.516 | 3.78001 | 4.5 % |
| 17 | 24.029 | 3.70051 | 0.5 % |
| 18 | 24.518 | 3.62784 | 1.5 % |
| 19 | 25.204 | 3.53068 | 4.6 % |
| 20 | 26.225 | 3.39544 | 2.7 % |
| 21 | 26.509 | 3.35968 | 3.0 % |
| 22 | 26.954 | 3.30524 | 2.5 % |
| 23 | 27.212 | 3.27451 | 1.2 % |
| 24 | 27.755 | 3.21161 | 1.5 % |
| 25 | 28.047 | 3.17886 | 1.2 % |
| 26 | 29.133 | 3.06275 | 1.2 % |
| 27 | 31.644 | 2.82522 | 1.5 % |
| 28 | 32.026 | 2.79241 | 1.8 % |
| 29 | 33.634 | 2.66252 | 0.6 % |
| 30 | 38.906 | 2.31296 | 0.4 % |

### Comparative Example: Form III (FB)

Preparation: A solution of TG02 free base in DCM was swapped with toluene. After cooling to 20-30°C, the product was filtered and dried to give TG02 Form III (FB).

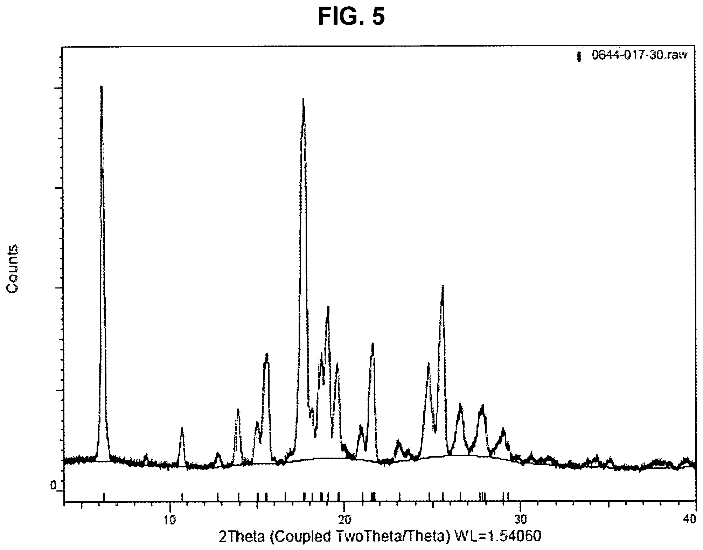

Characterization: The PXRD of TG02 Form III (FB) is shown in Fig. 5. Table 5 lists the peak positions, d values, and relative peak intensities of TG02 Form III (FB).

**Table 5**

| **Index** | **Angle (2-Theta °)** | **d Value (Å)** | **Relative Intensity** |
|---|---|---|---|
| 1 | 6.236 | 14.16204 | 100.0 % |
| 2 | 10.734 | 8.23523 | 10.4 % |
| 3 | 12.791 | 6.91506 | 3.7 % |
| 4 | 13.957 | 6.34010 | 14.8 % |
| 5 | 14.987 | 5.90674 | 9.4 % |
| 6 | 15.053 | 5.88077 | 10.9 % |
| 7 | 15.486 | 5.71739 | 26.2 % |
| 8 | 15.599 | 5.67617 | 29.6 % |
| 9 | 16.650 | 5.32029 | 1.3 % |
| 10 | 17.674 | 5.01413 | 89.8 % |
| 11 | 17.769 | 4.98765 | 93.6 % |
| 12 | 18.162 | 4.88050 | 13.2 % |
| 13 | 18.649 | 4.75417 | 25.4 % |
| 14 | 18.726 | 4.73479 | 27.5 % |
| 15 | 19.056 | 4.65350 | 37.8 % |
| 16 | 19.082 | 4.64721 | 38.9 % |
| 17 | 19.676 | 4.50833 | 22.2 % |
| 18 | 19.619 | 4.52115 | 23.3 % |
| 19 | 21.718 | 4.08873 | 22.6 % |
| 20 | 21.000 | 4.22691 | 7.8 % |
| 21 | 21.536 | 4.12288 | 26.5 % |
| 22 | 21.594 | 4.11207 | 29.9 % |
| 23 | 21.631 | 4.10514 | 30.9 % |
| 24 | 23.109 | 3.84567 | 4.7 % |
| 25 | 24.800 | 3.58719 | 25.1 % |
| 26 | 25.596 | 3.47737 | 44.3 % |
| 27 | 26.589 | 3.34973 | 13.0 % |
| 28 | 27.675 | 3.22071 | 11.4 % |
| 29 | 27.857 | 3.20014 | 11.8 % |
| 30 | 27.981 | 3.18625 | 9.7 % |
| 31 | 29.046 | 3.07175 | 7.8 % |
| 32 | 29.288 | 3.04691 | 4.0 % |

### Comparative Example: Form IV (FB)

Preparation: A warm solution containing TG02 free base and DMF was cooled to 20-30°C. The product was filtered and dried to give TG02 Form IV (FB).

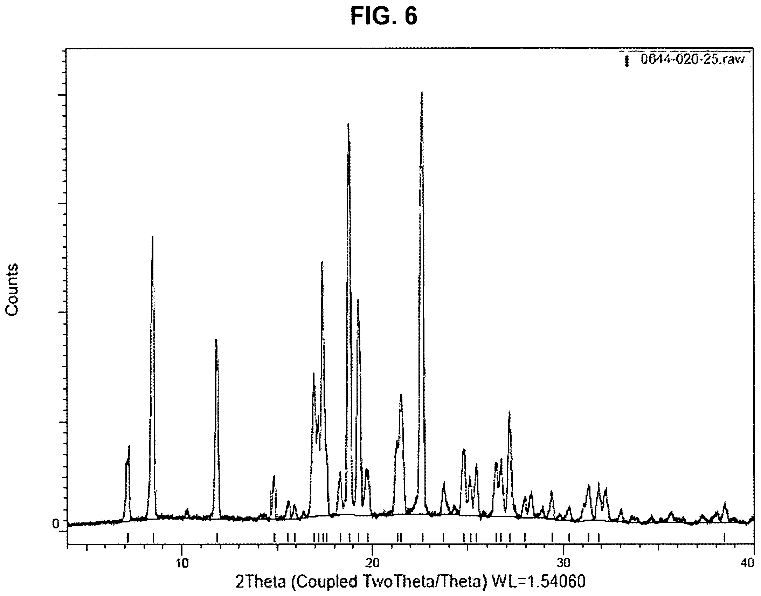

Characterization: The PXRD of TG02 Form IV (FB) is shown in Fig. 6. Table 6 lists the peak positions, d values, and relative peak intensities of TG02 Form IV (FB).

**Table 6**

| **Index** | **Angle (2-Theta °)** | **d Value (Å)** | **Relative Intensity** |
|---|---|---|---|
| 1 | 7.143 | 12.36611 | 14.9 % |
| 2 | 7.184 | 12.29422 | 13.7 % |
| 3 | 8.484 | 10.41370 | 67.9 % |
| 4 | 11.850 | 7.46220 | 42.0 % |
| 5 | 14.826 | 5.97050 | 9.4 % |
| 6 | 15.597 | 5.67675 | 4.0 % |
| 7 | 15.933 | 5.55779 | 3.3 % |
| 8 | 16.957 | 5.22453 | 31.4 % |
| 9 | 17.169 | 5.16040 | 21.5 % |
| 10 | 17.409 | 5.08997 | 61.1 % |
| 11 | 17.573 | 5.04269 | 17.0 % |
| 12 | 18.311 | 4.84106 | 10.5 % |
| 13 | 18.807 | 4.71465 | 93.2 % |
| 14 | 19.299 | 4.59553 | 48.3 % |
| 15 | 19.773 | 4.48636 | 11.0 % |
| 16 | 21.337 | 4.16085 | 16.2 % |
| 17 | 21.519 | 4.12623 | 27.5 % |
| 18 | 22.616 | 3.92843 | 100.0 % |
| 19 | 23.749 | 3.74353 | 7.5 % |
| 20 | 24.791 | 3.58845 | 15.6 % |
| 21 | 25.126 | 3.54135 | 8.3 % |
| 22 | 25.448 | 3.49736 | 11.3 % |
| 23 | 26.468 | 3.36482 | 12.1 % |
| 24 | 26.729 | 3.33250 | 13.9 % |
| 25 | 27.180 | 3.27825 | 23.7 % |
| 26 | 27.970 | 3.18744 | 4.7 % |
| 27 | 29.384 | 3.03719 | 6.3 % |
| 28 | 30.310 | 2.94650 | 3.3 % |
| 29 | 31.344 | 2.85159 | 8.2 % |
| 30 | 31.867 | 2.80594 | 9.4 % |
| 31 | 38.475 | 2.33792 | 4.7 % |

### Comparative Example: Form V (FB)

Preparation: A warm solution containing TG02 free base and DMSO/acetone or NMP/acetone or DMF/EtOAc was cooled to 20-30°C. The product was filtered and dried to give TG02 Form V (FB).

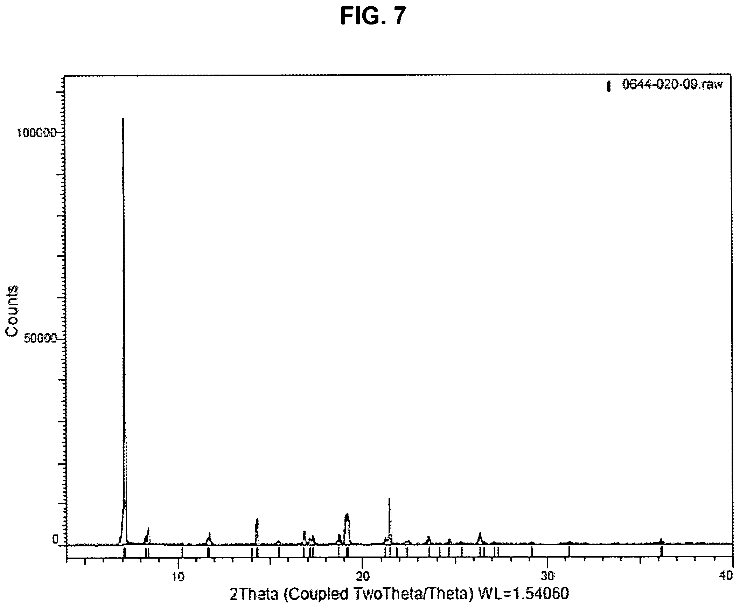

Characterization: The PXRD of TG02 Form V (FB) is shown in Fig. 7. Table 7 lists the peak positions, d values, and relative peak intensities of TG02 Form V (FB).

**Table 7**

| **Index** | **Angle (2-Theta °)** | **d Value (Å)** | **Relative Intensity** |
|---|---|---|---|
| 1 | 7.087 | 12.46270 | 12.1 % |
| 2 | 7.151 | 12.35255 | 100.0 % |
| 3 | 8.271 | 10.68141 | 2.0 % |
| 4 | 8.416 | 10.49824 | 4.0 % |
| 5 | 10.245 | 8.62705 | 0.4 % |
| 6 | 11.657 | 7.58525 | 1.3 % |
| 7 | 11.739 | 7.53278 | 2.9 % |
| 8 | 14.053 | 6.29687 | 0.3 % |
| 9 | 14.299 | 6.18933 | 6.1 % |
| 10 | 15.478 | 5.72033 | 0.7 % |
| 11 | 16.858 | 5.25516 | 2.7 % |
| 12 | 17.163 | 5.16220 | 1.2 % |
| 13 | 17.336 | 5.11121 | 2.1 % |
| 14 | 18.751 | 4.72848 | 1.8 % |
| 15 | 19.114 | 4.63953 | 5.9 % |
| 16 | 19.185 | 4.62256 | 7.4 % |
| 17 | 21.259 | 4.17594 | 0.9 % |
| 18 | 21.495 | 4.13071 | 9.7 % |
| 19 | 21.867 | 4.06121 | 0.2 % |
| 20 | 22.414 | 3.96346 | 0.5 % |
| 21 | 23.607 | 3.76576 | 1.4 % |
| 22 | 24.185 | 3.67699 | 0.2 % |
| 23 | 24.711 | 3.59998 | 1.1 % |
| 24 | 25.351 | 3.51045 | 0.2 % |
| 25 | 26.345 | 3.38018 | 2.7 % |
| 26 | 26.558 | 3.35357 | 0.3 % |
| 27 | 27.092 | 3.28875 | 0.6 % |
| 28 | 27.334 | 3.26010 | 0.2 % |
| 29 | 29.159 | 3.06012 | 0.4 % |
| 30 | 31.202 | 2.86423 | 0.6 % |
| 31 | 36.149 | 2.48278 | 1.2 % |
| 32 | 36.238 | 2.47691 | 0.6 % |

### COMPARATIVE EXAMPLE 2

### Preparation of TG02 HCl polymorphic forms

### Comparative Example: Form VI (HCl)

Preparation: A solution containing TG02·HCl (10 g), EtOH (184 mL) and water (16 mL) was heated to reflux for 1 h. The mixture was cooled to 0-5 °C and stirred for 1 h. The mixture was filtered, and the filter cake was washed with 90% EtOH (aq) and dried to give TG02 Form VI (HCl).

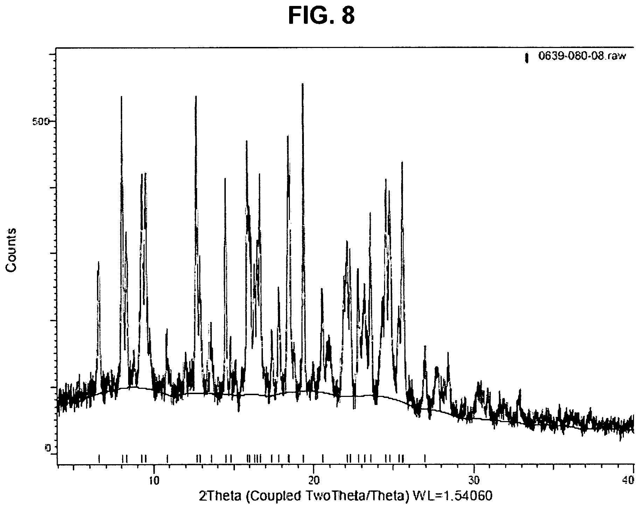

Characterization: The PXRD of TG02 Form VI (HCl) is shown in Fig. 8. Table 8 lists the peak positions, d values, and relative peak intensities of TG02 Form VI (HCl).

**Table 8**

| **Index** | **Angle (2-Theta °)** | **d Value (Å)** | **Relative Intensity** |
|---|---|---|---|
| 1 | 6.593 | 13.39617 | 41.8 % |
| 2 | 8.055 | 10.96766 | 90.8 % |
| 3 | 8.309 | 10.63284 | 45.6 % |
| 4 | 9.300 | 9.50212 | 65.4 % |
| 5 | 9.527 | 9.27630 | 69.2 % |
| 6 | 10.843 | 8.15308 | 16.7 % |
| 7 | 12.695 | 6.96730 | 96.1 % |
| 8 | 12.917 | 6.84816 | 40.2 % |
| 9 | 13.594 | 6.50861 | 19.1 % |
| 10 | 14.505 | 6.10155 | 70.5 % |
| 11 | 14.799 | 5.98113 | 18.4 % |
| 12 | 15.868 | 5.58066 | 83.0 % |
| 13 | 15.979 | 5.54199 | 58.7 % |
| 14 | 16.289 | 5.43722 | 41.3 % |
| 15 | 16.491 | 5.37121 | 47.4 % |
| 16 | 16.664 | 5.31561 | 71.7 % |
| 17 | 17.409 | 5.09002 | 21.2 % |
| 18 | 17.845 | 4.96641 | 34.5 % |
| 19 | 18.460 | 4.80252 | 74.6 % |
| 20 | 19.392 | 4.57378 | 100.0 % |
| 21 | 20.553 | 4.31777 | 32.1 % |
| 22 | 22.103 | 4.01853 | 49.9 % |
| 23 | 22.290 | 3.98509 | 42.9 % |
| 24 | 22.832 | 3.89183 | 31.6 % |
| 25 | 23.197 | 3.83142 | 30.9 % |
| 26 | 23.565 | 3.77237 | 56.2 % |
| 27 | 24.552 | 3.62281 | 67.2 % |
| 28 | 24.796 | 3.58772 | 63.8 % |
| 29 | 25.353 | 3.51019 | 30.5 % |
| 30 | 25.604 | 3.47630 | 63.7 % |
| 31 | 26.981 | 3.30197 | 18.5 % |

### Comparative Example: Form VII (HCl)

Preparation Method A: A solution containing TG02·2HCl (20 g), pyridine (60 mL) and H₂O (120 mL) was heated at 80°C for 1-3 h. The mixture was cooled to 20-30°C and stirred for 2 h. The mixture was filtered, and the filter cake was washed with H₂O and dried to give TG02 Form VII (HCl).

Preparation Method B: A solution containing TG02·2HCl (50 g) pyridine (150 mL) and EtOAc (300 mL ) was heated at 80°C for 2-3 h. The mixture was cooled to 20-30°C and stirred for 2 h. The mixture was filtered, and the filter cake was washed with H2O and dried to give TG02 Form VII (HCl).

Preparation Method C: A solution containing TG02·HCl (5.7 g), EtOH (74.5 mL) and H₂O (8 mL) was heated at reflux for 1 h. The mixture was cooled to 0-5°C and stirred for 1 h. The mixture was filtered, and the filter cake was washed with EtOH and dried to give TG02 Form VII (HCl).

Preparation Method D: A solution containing TG02·HCl (9.3 g) EtOH (284 mL) and H₂O (22 mL) was heated at reflux for 1 h. The mixture was cooled to 0-5°C and stirred for 2h. The mixture was filtered, and the filter cake was washed with EtOH and dried to give TG02 Form VII (HCl).

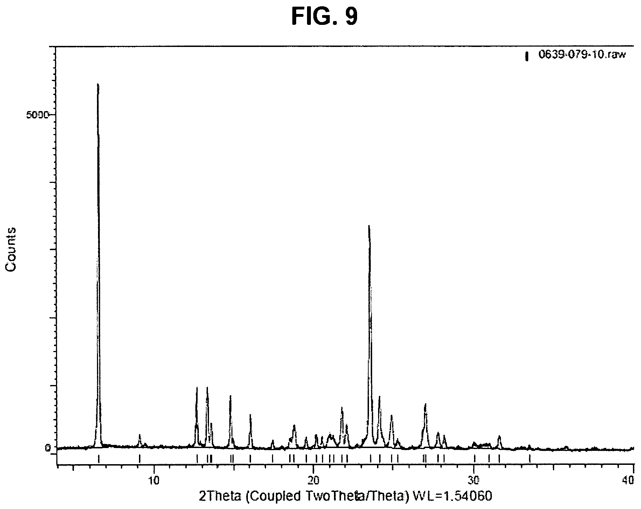

Characterization: The PXRD of TG02 Form VII (HCl) is shown in Fig. 9. Table 9 lists the peak positions, d values, and relative peak intensities of TG02 Form VII (HCl).

**Table 9**

| **Index** | **Angle (2-Theta °)** | **d Value (Å)** | **Relative Intensity** |
|---|---|---|---|
| 1 | 6.601 | 13.37949 | 100.0 % |
| 2 | 9.152 | 9.65532 | 3.4 % |
| 3 | 12.691 | 6.96956 | 15.3 % |
| 4 | 13.364 | 6.62027 | 16.9 % |
| 5 | 13.598 | 6.50683 | 6.3 % |
| 6 | 14.802 | 5.98006 | 14.5 % |
| 7 | 14.952 | 5.92026 | 2.1 % |
| 8 | 16.061 | 5.51407 | 8.5 % |
| 9 | 17.457 | 5.07590 | 2.4 % |
| 10 | 18.555 | 4.77810 | 2.9 % |
| 11 | 18.809 | 4.71417 | 6.5 % |
| 12 | 19.548 | 4.53753 | 2.6% |
| 13 | 20.191 | 4.39445 | 3.3 % |
| 14 | 20.549 | 4.31868 | 3.0% |
| 15 | 21.259 | 4.17601 | 2.7 % |
| 16 | 21.025 | 4.22208 | 3.8 % |
| 17 | 21.785 | 4.07639 | 10.2 % |
| 18 | 22.084 | 4.02178 | 5.5 % |
| 19 | 23.554 | 3.77407 | 61.2 % |
| 20 | 24.135 | 3.68456 | 14.4 % |
| 21 | 24.914 | 3.57101 | 8.1 % |
| 22 | 25.287 | 3.51924 | 1.9 % |
| 23 | 26.904 | 3.31123 | 5.1 % |
| 24 | 27.007 | 3.29892 | 11.7 % |
| 25 | 27.792 | 3.20747 | 4.4 % |
| 26 | 28.179 | 3.16424 | 3.6 % |
| 27 | 30.091 | 2.96742 | 1.2 % |
| 28 | 31.007 | 2.88184 | 1.2 % |
| 29 | 31.632 | 2.82632 | 3.7 % |
| 30 | 33.498 | 2.67297 | 0.8 % |

### Comparative Example: Form VIII (HCl)

Preparation: A solution containing TG02·HCl (77.1 g), EtOH (2340 mL) and H₂O (185 mL) was heated at reflux for 0.5 h. The mixture was cooled to 0-5°C and stirred for 2 h. The mixture was filtered, and the filter cake was washed with EtOH and dried to give TG02 Form VIII (HCl). TG02 Form VIII (HCl) gradually converted to TG02 Form VI (HCl) over time.

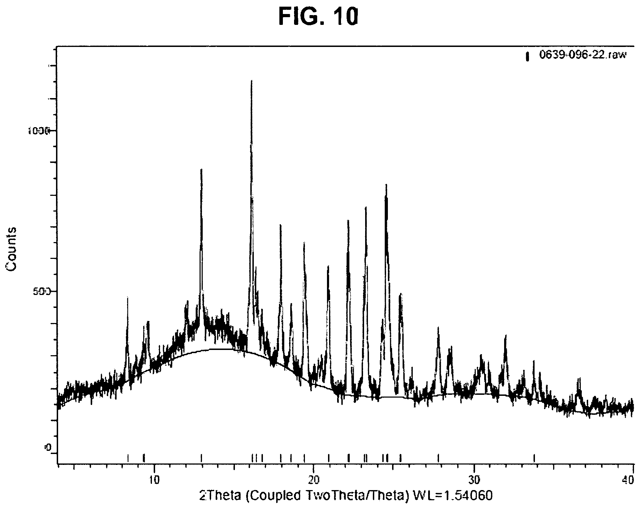

Characterization: The PXRD of TG02 HCl Form VIII is shown in Fig. 10. Table 10 lists the peak positions, d values, and relative peak intensities of TG02 Form VII (HCl).

**Table 10**

| **Index** | **Angle (2-Theta °)** | **d Value (Å)** | **Relative Intensity** |
|---|---|---|---|
| 1 | 8.351 | 10.57951 | 29.3 % |
| 2 | 9.402 | 9.39909 | 12.9 % |
| 3 | 12.994 | 6.80778 | 61.4 % |
| 4 | 16.147 | 5.48485 | 100.0 % |
| 5 | 16.386 | 5.40540 | 31.3 % |
| 6 | 16.807 | 5.27097 | 17.7 % |
| 7 | 17.977 | 4.93024 | 51.8 % |
| 8 | 18.624 | 4.76041 | 24.8 % |
| 9 | 19.441 | 4.56226 | 51.2 % |
| 10 | 20.933 | 4.24024 | 46.9 % |
| 11 | 22.152 | 4.00961 | 36.1 % |
| 12 | 22.211 | 3.99909 | 62.3 % |
| 13 | 23.190 | 3.83254 | 41.3 % |
| 14 | 23.305 | 3.81384 | 73.4 % |
| 15 | 24.305 | 3.65916 | 27.5 % |
| 16 | 24.317 | 3.65736 | 23.9 % |
| 17 | 24.586 | 3.61800 | 72.3 % |
| 18 | 24.679 | 3.60450 | 63.2 % |
| 19 | 25.407 | 3.50293 | 33.0 % |
| 20 | 25.513 | 3.48852 | 39.5 % |
| 21 | 27.804 | 3.20607 | 23.7 % |
| 22 | 33.775 | 2.65168 | 11.3 % |

### EXAMPLE 3

### Preparation of TG02 Form X (citrate)

Preparation: Method A: A 12.2% w/v solution of TG02 free base was prepared in DMSO/ethanol (94/6 v/v) by heating to about 70°C to dissolve the TG02 free base. A separate solution of citric acid in ethanol (10% w/v) containing a 2% molar excess of citric acid relative to TG02 free base was prepared. The volume of the citric acid/ethanol solution was about 64% relative to the TG02 free base solution. The citric acid/ethanol solution (at about 70°C) was transferred to the TG02 free base solution to form TG02 citrate, and the solution was stirred for at least 30 minutes. Warm ethanol (1.5 volume equivalents to previous citric acid/TG02 free base solution) was added and the solution was stirred for at least one hour at about 70°C. The solution was cooled to about 5°C. TG02 citrate crystallized upon cooling. TG02 citrate was collected by filtration, washed with ethanol and dried to give TG02 Form X (citrate) in 88-93% yield.

Method B: A homogeneous mixture of TG02 Form X (citrate), TG02 Citrate Pattern 1 of US 9,120,815, and TG02 Citrate Pattern 2 of US 9,120,815 was treated with solvent (20 vol.) and agitated for 4 days at different temperatures (5 °C, 25 °C, and 50 °C). The recovered solids were air dried and analysed by XRPD. As summarized in Table 11, several solvents resulted in the recovery of TG02 Form X (citrate).

**Table 11**

| **Solvent** | **Temperature** | **TG02 (Citrate) Polymorph¹** |
|---|---|---|
| IPA | 5 °C | Form X |
| Acetone | | Form X |
| TBME | | Pattern 2 + Form X |
| EtOAc | | Pattern 2 + Form X |
| H₂O | | Pattern 2 |
| THF:H₂O (9:1) | | Pattern 2 |
| DCM:H₂O (9:1) | | Pattern 2 + Form X |
| Acetone:H₂O (9:1) | | Pattern 2 |
| IPA | 25 °C | Form X |
| Acetone | | Form X |
| TBME | | Pattern 2 + Form X |
| EtOAc | | Form X |
| H₂O | | Pattern 2 |
| THF:H₂O (9:1) | | Pattern 2 |
| DCM:H₂O (9:1) | | Pattern 2 |
| Acetone:H₂O (9:1) | | Pattern 2 |
| IPA | 50 °C | Form X |
| Acetone | | Form X |
| TBME | | Form X |
| EtOAc | | Form X |
| H₂O | | Pattern 2 |
| THF:H₂O (9:1) | | Pattern 2 |
| DCM:H₂O (9:1) | | Form X |
| Acetone:H₂O (9:1) | | Pattern 2 |

| | | |
|---|---|---|
| ¹ *See* Example 4 for discussion of Pattern 2 | | |

Characterization: Form X (citrate) is a non-solvated, non-hygroscopic crystalline form of TG02 citrate which remained unchanged by XRPD following storage at elevated temperatures and relative humidity levels (40 °C / 75 % RH, 25 °C / 97 % RH and 60 °C / ambient RH) after 28 days. Proton NMR was consistent with the proposed structure. However, as the methylene protons of the citric acid overlapped with the D₆-DMSO reference peak, the stoichiometric equivalence of citrate acid is slightly offset. Thermal gravimetric analysis revealed the sample lost 29.8 % w/w between 190 - 230 °C, possibly due to the dissociation of the salt. Differential scanning calorimetry thermal events included a single broad endotherm at 204.5 °C (366.9 J/g). Analysis of the thermal events monitored at two different heating rates (2 °C and 10 °C) revealed a significant change in both melt temperature and enthalpy (192.1 °C, 336.2 J/g and 205.3 °C, 378.2 J/g). This finding suggests the observed endotherm is not a pure melt (thermodynamic) but also consists of a kinetic component possibly the dissociation of the citrate. HPLC purity analysis resulted in a purity reading of 97.6 %. GVS analysis showed the material to be non-hygroscopic, with an uptake of 0.13 % between 0 - 90 % RH, with a maximum difference of 0.04 % hysteresis between 40 - 60 % RH. The sample remained unchanged by XRPD following GVS analysis. Thermodynamic solubility determination produced a reading of 0.64 mg/ml solubility in aqueous media.

The XRPD of Form X (citrate) is shown in Fig. 11. Table 12 lists the peak positions, d values, and relative peak intensities of TG02 Form X (citrate).

**Table 12**

| **Index** | **Angle (2-Theta °)** | **d value (Å)** | **Relative Intensity** |
|---|---|---|---|
| 1 | 8.6 | 10.3 | 10.7 % |
| 2 | 9.4 | 9.4 | 12.8 % |
| 3 | 11.9 | 7.4 | 13.0 % |
| 4 | 12.5 | 7.1 | 8.6 % |
| 5 | 14.3 | 6.2 | 9.8 % |
| 6 | 15.2 | 5.8 | 33.4 % |
| 7 | 15.5 | 5.7 | 47.7 % |
| 8 | 16.1 | 5.5 | 14.6 % |
| 9 | 16.4 | 5.4 | 6.9 % |
| 10 | 17.0 | 5.2 | 22.3 % |
| 11 | 17.4 | 5.1 | 18.4 % |
| 12 | 17.9 | 5.0 | 7.6 % |
| 13 | 19.0 | 4.7 | 8.6% |
| 14 | 19.6 | 4.5 | 13.2 % |
| 15 | 20.3 | 4.4 | 9.0 % |
| 16 | 20.6 | 4.3 | 9.1 % |
| 17 | 21.2 | 4.2 | 5.3 % |
| 18 | 21.7 | 4.1 | 33.5 % |
| 19 | 22.1 | 4.0 | 31.4 % |
| 20 | 23.0 | 3.9 | 100.0 % |
| 21 | 23.5 | 3.8 | 5.6 % |
| 22 | 23.9 | 3.7 | 7.4 % |
| 23 | 24.2 | 3.7 | 5.1 % |
| 24 | 24.8 | 3.6 | 9.1 % |
| 25 | 26.2 | 3.4 | 21.9 % |
| 26 | 27.3 | 3.3 | 7.1 % |
| 27 | 28.0 | 3.2 | 8.3 % |
| 28 | 29.9 | 3.0 | 21.1 % |

### EXAMPLE 4

### TG02 Citrate Patterns of US 9,120,815

TG02 Citrate Pattern 1 ("Pattern 1") of US 9,120,815 is a non-solvated, slightly hygroscopic crystalline form of TG02 citrate, which remained unchanged by XRPD following storage at elevated temperatures and relative humidity (40 °C / 75 % RH, 25 °C / 97 % RH and 60 °C / ambient RH) for 28 days. Proton NMR analysis was consistent with the proposed structure, although there was overlap of the methylene protons of the citrate and the D₆-DMSO reference peak. Thermal gravimetric analysis showed a weight loss of 29.6 % w/w between 180 - 240 °C, possibly due to loss of citrate. Differential scanning calorimetry revealed a single endothermic event at 196.6 °C (327.7 J/g). Comparing DSC profiles of the material at two different heating rates (2 °C and 10 °C) showed a large difference in onset temperature and enthalpy. This observation suggest the endotherm is likely due to both melt of the material (thermodynamic) and loss of the citrate (kinetic). HPLC purity analysis resulted in a purity reading of 97.7 %. GVS analysis showed the material to be slightly-hygroscopic, with an uptake of 1.0 % between 0 - 90 % RH and a maximum hysteresis of 0.2 % between 40 - 50 % RH. The sample remained unchanged by XRPD following GVS analysis. Thermodynamic solubility determination produced a reading of 0.33 mg/ml solubility in aqueous media. XRPD following thermodynamic solubility analysis revealed a form change from TG02 Citrate Pattern 1 to TG02 Citrate Pattern 2 (both of US 9,120,815). The XRPD of Pattern 1 is shown in Fig. 1.

TG02 Citrate Pattern 2 ("Pattern 2") of US 9,120,815 defined as a hydrated, hygroscopic crystalline form of TG02 citrate, which remained unchanged by XRPD following storage at elevated temperatures and relative humidity (40 °C / 75 % RH and 25 °C / 97 % RH) for 28 days. However, storage at 60 °C / ambient RH resulted in additional peaks being present in the XRPD diffractogram after 7 days. These additional peaks remained throughout the 28 days storage period. This may be caused by dehydration of the hydrate. Proton NMR analysis was consistent with the proposed structure, although there was overlap of the methylene protons of the citrate and the D₆-DMSO reference peak. Thermal gravimetric analysis showed two weight losses: a weight loss of 3.1 % w/w between 25 - 115 °C, possibly due to loss of water, followed by a weight loss of 29.5 % w/w between 180 - 240 °C, possibly due to the dissociation of citrate. Differential scanning calorimetry consisted of a broad, asymmetrical endotherm between 25 - 115 °C (70.2 J/g) followed by a large endotherm at 186.0 °C (313.4 J/g), possibly due to the sample melt and dissolution of the salt. HPLC purity analysis resulted in a purity reading of 97.7 %. GVS analysis showed the material to be hygroscopic, with an uptake of 3.84 % between 0 - 90 % RH, with a maximum hysteresis of 1.4 % between 10 - 30 % RH. The sample remained unchanged by XRPD following GVS analysis. Water content was determined by Karl Fischer analysis to be 3.3 %, equating to 1 molar equivalent of water. Thermodynamic solubility determination produced a reading of 0.23 mg/ml solubility in aqueous media and remained unchanged by XRPD. The XRPD of Pattern 2 is shown in Fig. 2.

### EXAMPLE 5

### Photostability

The photostability of Pattern 1 and Pattern 2 of US 9,120,815, and Form X (citrate) was investigated to determine whether light exposure results in substance changes.

A thin layer (≤ 3 mm) of Pattern 1, Pattern 2, and Form X (citrate) was placed in a clear glass HPLC vial. These samples were prepared in duplicate; one set to be kept in the dark (wrapped in aluminium foil) and the other exposed to light. Images of the HPLC vials were taken before and after light exposure. Samples were exposed to irradiation (765 W/m2) for 6.9 h, equating to one weeks Miami sunshine.

No visual change in appearance, XRPD, or purity of each sample was noted.

### EXAMPLE 6

### Pharmaceutical Compositions

Compatibility experiments were conducted to select excipients for TG02 Form X (citrate) for use in gelatin capsules. Binary mixtures (1:1) of an excipient and TG02 Form X (citrate) were prepared, mixed, and stored at 40°/75% RH in open and closed containers for four weeks. Blend appearance and HPLC test results (chromatographic purity and assay) obtained after four weeks of storage in both open and closed configurations were compared to the initial results (Table 13 and Table 14). No significant appearance changes were noted over the course of the study. The list of compatible and incompatible excipients is provided in Table 15.

**Table 13: Excipient Compatibility Results, 40°C/ 75% RH, Open Configuration**

| **Excipient** | **Initial** | | **4 weeks** | |
|---|---|---|---|---|
| | **Purity (%)** | **Assay (%)** | **Purity (%)** | **Assay (%)** |
| Avicel | 96.06 | 100.92 | 96.69 | 99.99 |
| Prosolv | 96.48 | 101.04 | 96.66 | 101.24 |
| Mannitol | 96.67 | 100.91 | 96.65 | 99.06 |
| Lactose Monohydrate | 96.65 | 100.27 | 96.69 | 99.89 |
| HPMC | 96.66 | 101.85 | 96.67 | 98.41 |
| PVP | 96.67 | 100.75 | 96.67 | 98.81 |
| PRUV | 96.41 | 83.93 | 96.13 | 71.01 |
| Mg Stearate | 96.65 | 90.42 | 96.67 | 99.92 |
| Croscarmellose | 96.36 | 73.46 | 96.36 | 76.23 |
| Explotab | 96.58 | 91.95 | 96.60 | 92.77 |
| Crospovidone | 96.64 | 98.61 | 96.67 | 98.88 |
| Avicel + SLS | 96.67 | 100.29 | 96.65 | 100.32 |
| Control | 96.66 | 100.96 | 96.68 | 98.29 |

**Table 14: Excipient Compatibility Results, 40°C/ 75% RH, Closed Configuration**

| **Excipient** | **Initial** | | **4 weeks** | |
|---|---|---|---|---|
| | **Purity (%)** | **Assay (%)** | **Purity (%)** | **Assay (%)** |
| Avicel | 96.06 | 100.92 | 96.70 | 103.27 |
| Prosolv | 96.48 | 101.04 | 96.68 | 100.34 |
| Mannitol | 96.67 | 100.91 | 96.62 | 96.84 |
| Lactose Monohydrate | 96.65 | 100.27 | 96.63 | 98.71 |
| HPMC | 96.66 | 101.85 | 96.60 | 95.40 |
| PVP | 96.67 | 100.75 | 96.64 | 97.55 |
| PRUV | 96.41 | 83.93 | 96.24 | 72.60 |
| Mg Stearate | 96.65 | 90.42 | 96.68 | 96.10 |
| Croscarmellose | 96.36 | 73.46 | 96.29 | 69.92 |
| Explotab | 96.58 | 91.95 | 96.58 | 93.96 |
| Crospovidone | 96.64 | 98.61 | 96.67 | 100.14 |
| Avicel + SLS | 96.67 | 100.29 | 96.62 | 102.09 |
| Control | 96.66 | 100.96 | 96.66 | 94.38 |

**Table 15: Results Summary of TG02 Form X (citrate) Excipient Compatibility**

| **Excipient Function** | **Compatible Excipients** | **Potentially Incompatible Excipients** |
|---|---|---|
| Fillers | Microcrystalline cellulose | |
| | Silicified microcrystalline cellulose | |
| | Lactose monohydrate | |
| | Mannitol | |
| Binders | Crospovidone | |
| | Hydroxypropyl methycellulose | |
| Disintegrants | Crospovidone | Croscarmellose sodium |
| | Sodium starch glycolate | |
| Lubricants | Magnesium stearate | |
| | Sodium steoryl fumarate (PRUV) | |
| Wetting Agents | Sodium lauryl sulfate | |
| Capsule Shell | Gelatin | |

Representative TG02 Form X (citrate) formulation compositions are provided in Table 16. The capsules are an immediate release dosage form provided in two strengths: 50 mg and 150 mg. The capsule fill is a dry powder formulated blend of TG02 Form X (citrate) and excipients. The labelled strength of the TG02 Form X (citrate) capsules is in terms of TG02 base, while the batch formula is in terms of the TG02 Form X (citrate) salt. For example, the 50 mg strength of TG02 Form X (citrate) capsules contains about 76 mg of TG02 Form X (citrate) to account for the citric acid content of the drug substance total mass. Additional compositions are provided in Table 17.

**Table 16: Formulation Compositions for TG02 Form X (citrate) Capsules**

| | **Strength:** | **50 mg** | **150-mg** |
|---|---|---|---|
| | **Name:** | **TG02** | **TG02** |
| | Capsule Size | 2 | 0 |
| | Capsule Color | Swedish Orange | Light blue |

| **Ingredient** | **Function** | **Composition (mg/capsule)** | |
|---|---|---|---|
| TG02 citrate | Active | 76.23 | 228.6 |
| Silicified Microcrystalline Cellulose, NF | Filler | 111.72 | 93.6 |
| Crospovidone, Ph.Eur., NF, JP | Disintegrant | 10.50 | 18.0 |
| Hypromellose 2910, Ph.Eur., USP, JP | Binder | 10.50 | 18.0 |
| Magnesium Stearate, NF | Lubricant | 1.05 | 1.8 |
| Total Fill Weight (mg): | | 210.0 | 360.0 |

**Table 17: Formulation Composition for TG02 Form X (citrate) Capsules**

| **Ingredient** | **Strength (mg)** | | |
|---|---|---|---|
| | **10 mg** | **10 mg** | **150 mg** |
| TG02 Citrate | 15.6 | 15.6 | 234.0 |
| Silicified Microcrystalline Cellulose, NF | 172.3 | 172.3 | 106.2 |
| Crospovidone, NF | 10.5 | 10.5 | 19.0 |
| Hypromellose 2910, USP | 10.5 | 10.5 | 19.0 |
| Magnesium Stearate, NF | 1.1 | --- | 1.8 |
| Sodium stearyl fumarate, NF (PRUV) | --- | 1.1 | --- |

## Claims

1. A polymorphic form of the citrate salt of (16E)-14-methyl-20-oxa-5,7,14,26-tetraazatetracyclo[19.3.1.1(2,6).1(8,12)]heptacosa-1(25),2(26),3,5,8(27),9,11,16,21,23-decaene (TG02 Form X (citrate)) characterized as having a powder x-ray diffraction pattern with peaks at 15.2 ± 0.2 degrees 2Θ, 15.5 ± 0.2 degrees 2Θ, 21.7 ± 0.2 degrees 2Θ, 22.1 ± 0.2 degrees 2Θ, 23.0 ± 0.2 degrees 2Θ, 26.2 ± 0.2 degrees 2Θ, and 29.9 ± 0.2 degrees 2Θ, wherein the powder x-ray diffraction pattern is collected using Cu Kα radiation, for use in the treatment of cancer in a patient, wherein said treatment further comprises administering to the patient a therapeutically effective amount of an anti-PD-1 antibody, an anti-PD-L1 antibody, or temozolomide.

2. The polymorphic form for use of claim 1, wherein said treatment further comprises administering to the patient a therapeutically effective amount of an anti-PD-1 antibody.

3. The polymorphic form for use of claim 2, wherein the anti-PD-1 antibody is selected from the group consisting of nivolumab, pembrolizumab, pidilizumab and STI-1110.

4. The polymorphic form for use of claim 1, wherein said treatment further comprises administering to the patient a therapeutically effective amount of an anti-PD-L1 antibody.

5. The polymorphic form for use of claim 4, wherein the anti-PD-L1 antibody is selected from the group consisting of avelumab, atezolizumab, durvalumab, and STI-1014.

6. The polymorphic form for use of claim 1, wherein said treatment further comprises administering to the patient a therapeutically effective amount of temozolomide.

7. The polymorphic form for use of any one of claims 1-6, wherein the TG02 Form X (citrate) has an average particle size distribution of 10 µm or less.

8. The polymorphic form for use of any one of claims 1-7, wherein the cancer is a solid tumor.

9. The polymorphic form for use of any one of claims 1-7, wherein the cancer is a hematological malignancy.

10. The polymorphic form for use of any one of claims 1-7, wherein the cancer is selected from the group consisting of adrenal cancer, acinic cell carcinoma, acoustic neuroma, acral lentigious melanoma, acrospiroma, acute eosinophilic leukemia, acute erythroid leukemia, acute lymphoblastic leukemia, acute megakaryoblastic leukemia, acute monocytic leukemia, acute promyelocytic leukemia, adenocarcinoma, adenoid cystic carcinoma, adenoma, adenomatoid odontogenic tumor, adenosquamous carcinoma, adipose tissue neoplasm, adrenocortical carcinoma, adult T cell leukemia/lymphoma, aggressive NK-cell leukemia, AIDS-related lymphoma, alveolar rhabdomyosarcoma, alveolar soft part sarcoma, ameloblastic fibroma, anaplastic large cell lymphoma, anaplastic thyroid cancer, angioimmunoblastic T-cell lymphoma, angiomyolipoma, angiosarcoma, astrocytoma, atypical teratoid rhabdoid tumor, B-cell chronic lymphocytic leukemia, B-cell prolymphocytic leukemia, B-cell lymphoma, basal cell carcinoma, biliary tract cancer, bladder cancer, blastoma, bone cancer, Brenner tumor, Brown tumor, Burkitt's lymphoma, breast cancer, brain cancer, carcinoma, carcinoma in situ, carcinosarcoma, cartilage tumor, cementoma, myeloid sarcoma, chondroma, chordoma, choriocarcinoma, choroid plexus papilloma, clear-cell sarcoma of the kidney, craniopharyngioma, cutaneous T-cell lymphoma, cervical cancer, colorectal cancer, Degos disease, desmoplastic small round cell tumor, diffuse large B-cell lymphoma, dysembryoplastic neuroepithelial tumor, dysgerminoma, embryonal carcinoma, endocrine gland neoplasm, endodermal sinus tumor, enteropathy-associated T-cell lymphoma, esophageal cancer, fetus in fetu, fibroma, fibrosarcoma, follicular lymphoma, follicular thyroid cancer, ganglioneuroma, gastrointestinal cancer, germ cell tumor, gestational choriocarcinoma, giant cell fibroblastoma, giant cell tumor of the bone, glial tumor, glioblastoma, glioma, gliomatosis cerebri, glucagonoma, gonadoblastoma, granulosa cell tumor, gynandroblastoma, gallbladder cancer, gastric cancer, hairy cell leukemia, hemangioblastoma, head and neck cancer, hemangiopericytoma, hepatoblastoma, hepatocellular carcinoma, hepatosplenic T-cell lymphoma, Hodgkin's lymphoma, non-Hodgkin's lymphoma, invasive lobular carcinoma, intestinal cancer, kidney cancer, laryngeal cancer, lentigo maligna, lethal midline carcinoma, leukemia, leydig cell tumor, liposarcoma, lung cancer, lymphangioma, lymphangiosarcoma, lymphoepithelioma, lymphoma, acute lymphocytic leukemia, acute myelogenous leukemia, chronic lymphocytic leukemia, liver cancer, small cell lung cancer, non-small cell lung cancer, MALT lymphoma, malignant fibrous histiocytoma, malignant peripheral nerve sheath tumor, malignant triton tumor, mantle cell lymphoma, marginal zone B-cell lymphoma, mast cell leukemia, mediastinal germ cell tumor, medullary carcinoma of the breast, medullary thyroid cancer, medulloblastoma, melanoma, meningioma, merkel cell cancer, mesothelioma, metastatic urothelial carcinoma, mixed Mullerian tumor, mucinous tumor, multiple myeloma, muscle tissue neoplasm, mycosis fungoides, myxoid liposarcoma, myxoma, myxosarcoma, nasopharyngeal carcinoma, neurinoma, neuroblastoma, neurofibroma, neuroma, nodular melanoma, ocular cancer, oligoastrocytoma, oligodendroglioma, oncocytoma, optic nerve sheath meningioma, optic nerve tumor, oral cancer, osteosarcoma, ovarian cancer, Pancoast tumor, papillary thyroid cancer, paraganglioma, pinealoblastoma, pineocytoma, pituicytoma, pituitary adenoma, pituitary tumor, plasmacytoma, polyembryoma, precursor T-lymphoblastic lymphoma, primary central nervous system lymphoma, primary effusion lymphoma, preimary peritoneal cancer, prostate cancer, pancreatic cancer, pharyngeal cancer, pseudomyxoma periotonei, renal cell carcinoma, renal medullary carcinoma, retinoblastoma, rhabdomyoma, rhabdomyosarcoma, Richter's transformation, rectal cancer, sarcoma, Schwannomatosis, seminoma, Sertoli cell tumor, sex cord-gonadal stromal tumor, signet ring cell carcinoma, skin cancer, small blue round cell tumors, small cell carcinoma, soft tissue sarcoma, somatostatinoma, soot wart, spinal tumor, splenic marginal zone lymphoma, squamous cell carcinoma, synovial sarcoma, Sezary's disease, small intestine cancer, squamous carcinoma, stomach cancer, T-cell lymphoma, testicular cancer, thecoma, thyroid cancer, transitional cell carcinoma, throat cancer, urachal cancer, urogenital cancer, urothelial carcinoma, uveal melanoma, uterine cancer, verrucous carcinoma, visual pathway glioma, vulvar cancer, vaginal cancer, Waldenstrom's macroglobulinemia, Warthin's tumor, Wilms' tumor, and diffuse pontine glioma.

11. The polymorphic form for use of any one of claims 1-7, wherein the cancer is selected from the group consisting of multiple myeloma, hepatocellular carcinoma, glioblastoma, lung cancer, breast cancer, head and neck cancer, prostate cancer, melanoma, colorectal cancer, and diffuse pontine glioma.

12. The polymorphic form for use of claim 11, wherein the cancer is hepatocellular carcinoma.

13. The polymorphic form for use of claim 11, wherein the cancer is glioblastoma.

14. The polymorphic form for use of claim 11, wherein the cancer is prostate cancer.

15. The polymorphic form for use of claim 11, wherein the cancer is diffuse pontine glioma.

## Patentansprüche

1. Polymorphe Form des Citratsalzes von (16E)-14-Methyl-20-oxa-5,7,14,26-tetraazatetracyclo[19.3.1.1(2,6).1(8,12)]heptacosa-1(25),2(26),3,5,8(27),9,11,16,21,23-decaen (TG02-Form X (Citrat)), **dadurch gekennzeichnet, dass** sie ein Pulver-Röntgenbeugungsmuster mit Peaks bei 15,2 ± 0,2 Grad 2Θ, 15,5 ± 0,2 Grad 2Θ, 21,7 ± 0,2 Grad 2Θ, 22,1 ± 0,2 Grad 2Θ, 23,0 ± 0,2 Grad 2Θ, 26,2 ± 0,2 Grad 2Θ und 29,9 ± 0,2 Grad 2Θ aufweist, wobei das Pulver-Röntgenbeugungsmuster unter Verwendung von Cu-Kα-Strahlung erfasst wird, zur Verwendung bei der Behandlung von Krebs bei einem Patienten, wobei die Behandlung ferner Verabreichen einer therapeutisch wirksamen Menge eines Anti-PD-1-Antikörpers, eines Anti-PD-L1-Antikörpers oder von Temozolomid an den Patienten umfasst.

2. Polymorphe Form zur Verwendung nach Anspruch 1, wobei die Behandlung ferner Verabreichen einer therapeutisch wirksamen Menge eines Anti-PD-1-Antikörpers an den Patienten umfasst.

3. Polymorphe Form zur Verwendung nach Anspruch 2, wobei der Anti-PD-1-Antikörper ausgewählt ist aus der Gruppe bestehend aus Nivolumab, Pembrolizumab, Pidilizumab und STI-1110.

4. Polymorphe Form zur Verwendung nach Anspruch 1, wobei die Behandlung ferner Verabreichen einer therapeutisch wirksamen Menge eines Anti-PD-L1-Antikörpers an den Patienten umfasst.

5. Polymorphe Form zur Verwendung nach Anspruch 4, wobei der Anti-PD-L1-Antikörper ausgewählt ist aus der Gruppe bestehend aus Avelumab, Atezolizumab, Durvalumab und STI-1014.

6. Polymorphe Form zur Verwendung nach Anspruch 1, wobei die Behandlung ferner Verabreichen einer therapeutisch wirksamen Menge von Temozolomid an den Patienten umfasst.

7. Polymorphe Form zur Verwendung nach einem der Ansprüche 1-6, wobei die TG02-Form X (Citrat) eine durchschnittliche Partikelgrößenverteilung von 10 µm oder weniger aufweist.

8. Polymorphe Form zur Verwendung nach einem der Ansprüche 1-7, wobei der Krebs ein solider Tumor ist.

9. Polymorphe Form zur Verwendung nach einem der Ansprüche 1-7, wobei der Krebs eine hämatologische Malignität ist.

10. Polymorphe Form zur Verwendung nach einem der Ansprüche 1-7, wobei der Krebs ausgewählt ist aus der Gruppe bestehend aus Nebennierenkrebs, Azinuszellkarzinom, Akustikusneurinom, akral-lentiginösem Melanom, Akrospirom, akuter eosinophiler Leukämie, akuter erythroider Leukämie, akuter lymphoblastischer Leukämie, akuter megakaryoblastischer Leukämie, akuter Monozytenleukämie, akuter Promyelozytenleukämie, Adenokarzinom, adenoid-zystischem Karzinom, Adenom, adenomatoidem odontogenem Tumor, adenosquamösem Karzinom, Neoplasma des Fettgewebes, Nebennierenrindenkarzinom, adulter/adultem T-Zell-Leukämie/Lymphom, aggressiver NK-Zell-Leukämie, AIDS-assoziiertem Lymphom, alveolärem Rhabdomyosarkom, alveolärem Weichteilsarkom, ameloblastischem Fibrom, anaplastischem großzelligem Lymphom, anaplastischem Schilddrüsenkrebs, angioimmunoblastischem T-Zell-Lymphom, Angiomyolipom, Angiosarkom, Astrozytom, atypischem teratoidem rhabdoidem Tumor, chronischer lymphatischer B-Zell-Leukämie, prolymphozytischer B-Zell-Leukämie, B-Zell-Lymphom, Basalzellkarzinom, Gallengangskrebs, Blasenkrebs, Blastom, Knochenkrebs, Brenner-Tumor, braunem Tumor, Burkitt-Lymphom, Brustkrebs, Hirntumor, Karzinom, Carcinoma in situ, Karzinosarkom, Knorpeltumor, Zementom, myeloischem Sarkom, Chondrom, Chordom, Chorionkarzinom, Choroidplexuspapillom, Klarzellsarkom der Niere, Kraniopharyngiom, kutanem T-Zell-Lymphom, Gebärmutterhalskrebs, Kolorektalkrebs, Degos-Syndrom, desmoplastischem klein- und rundzelligem Tumor, diffusem großzelligem B-Zell-Lymphom, dysembryoplastischem neuroepithelialem Tumor, Dysgerminom, embryonalem Karzinom, Neoplasie der endokrinen Drüsen, endodermalem Sinustumor, Enteropathieassoziiertem T-Zell-Lymphom, Speiseröhrenkrebs, Foetus in foeto, Fibrom, Fibrosarkom, follikulärem Lymphom, follikulärem Schilddrüsenkrebs, Ganglioneurom, gastrointestinalem Krebs, Keimzelltumor, gestationalem Chorionkarzinom, Riesenzellfibroblastom, Riesenzelltumor des Knochens, Glia-Tumor, Glioblastom, Gliom, Gliomatosis cerebri, Glucagonoma, Gonadoblastom, Granulosazelltumor, Gynandroblastom, Gallenblasenkrebs, Magenkrebs, Haarzellenleukämie, Hämangioblastom, Kopf-Hals-Krebs, Hämangioperizytom, Hepatoblastom, hepatozellulärem Karzinom, hepatosplenischem T-Zell-Lymphom, Hodgkin-Lymphom, Non-Hodgkin-Lymphom, invasivem lobulärem Karzinom, Darmkrebs, Nierenkrebs, Larynxkrebs, Lentigo maligna, letalem Mittellinienkarzinom, Leukämie, Leydigzelltumor, Liposarkom, Lungenkrebs, Lymphangiom, Lymphangiosarkom, Lymphoepitheliom, Lymphom, akuter lymphatischer Leukämie, akuter myelogener Leukämie, chronischer lymphatischer Leukämie, Leberkrebs, kleinzelligem Lungenkrebs, nicht-kleinzelligem Lungenkrebs, MALT-Lymphom, malignem fibrösem Histiozytom, malignem peripherem Nervenscheidentumor, malignem Tritontumor, Mantelzell-Lymphom, Marginalzonen-B-Zell-Lymphom, Mastzellleukämie, mediastinalem Keimzelltumor, medullärem Karzinom der Brust, medullärem Schilddrüsenkrebs, Medulloblastom, Melanom, Meningiom, Merkelzellkrebs, Mesotheliom, metastasiertem Urothelkarzinom, Müllerschem Mischtumor, muzinösem Tumor, Multiplem Myelom, Muskelgewebsneoplasie, Mycosis fungoides, myxoidem Liposarkom, Myxom, Myxosarkom, Nasopharyngealkarzinom, Neurinom, Neuroblastom, Neurofibrom, Neurom, nodulärem Melanom, Augenkrebs, Oligoastrozytom, Oligodendrogliom, Onkozytom, Sehnervenscheidenmeningiom, Sehnerventumor, Mundkrebs, Osteosarkom, Eierstockkrebs, Pancoast-Tumor, papillärem Schilddrüsenkrebs, Paragangliom, Pinealoblastom, Pineozytom, Pituizytom, Hypophysenadenom, Hypophysentumor, Plasmazytom, Polyembryom, Vorläufer-T-lymphoblastischem Lymphom, primärem Lymphom des Zentralnervensystems, primärem Effusionlymphom, primärem Peritonealkrebs, Prostatakrebs, Bauchspeicheldrüsenkrebs, Pharynxkrebs, Pseudomyxom periotonei, Nierenzellkarzinom, Nierenmarkskarzinom, Retinoblastom, Rhabdomyom, Rhabdomyosarkom, Richter-Transformation, Rektumkrebs, Sarkom, Schwannomatose, Seminom, Sertoli-Zelltumor, Keimstrang-Gonaden-Stromatumor, Siegelringzellkarzinom, Hautkrebs, klein-, blau- und rundzelligen Tumoren, kleinzelligem Karzinom, Weichteilsarkom, Somatostatinom, Schornsteinfegerkrebs, Wirbelsäulentumor, Milzmarginalzonenlymphom, Plattenepithelzellenkarzinom, Synovialsarkom, Sezary-Syndrom, Dünndarmkrebs, Plattenepithelkarzinom, Magenkrebs, T-Zell-Lymphom, Hodenkrebs, Thekom, Schilddrüsenkrebs, Übergangszellkarzinom, Rachenkrebs, Urachuskrebs, Urogenitalkrebs, Urothelkarzinom, Uveamelanom, Gebärmutterkrebs, verrukösem Karzinom, Gliom der Sehbahn, Vulvakrebs, Vaginalkrebs, Waldenstrom-Makroglobulinämie, Warthin-Tumor, Wilms-Tumor und diffusem Ponsgliom.

11. Polymorphe Form zur Verwendung nach einem der Ansprüche 1-7, wobei der Krebs ausgewählt ist aus der Gruppe bestehend aus Multiplem Myelom, hepatozellulärem Karzinom, Glioblastom, Lungenkrebs, Brustkrebs, Kopf-Hals-Krebs, Prostatakrebs, Melanom, kolorektalem Krebs und diffusem Ponsgliom.

12. Polymorphe Form zur Verwendung nach Anspruch 11, wobei der Krebs ein hepatozelluläres Karzinom ist.

13. Polymorphe Form zur Verwendung nach Anspruch 11, wobei der Krebs ein Glioblastom ist.

14. Polymorphe Form zur Verwendung nach Anspruch 11, wobei der Krebs Prostatakrebs ist.

15. Polymorphe Form zur Verwendung nach Anspruch 11, wobei der Krebs ein diffuses Ponsgliom ist.

## Revendications

1. Forme polymorphe du sel de citrate de (16E)-14-méthyl-20-oxa-5,7,14,26-tétraazatétracyclo[19.3.1.1(2,6).1(8,12)]heptacosa-1(25),2(26),3,5,8(27),9,11,16,21,23-décaène (Forme X (citrate) de TG02) **caractérisée par** un diagramme de diffraction des rayons X sur poudre présentant des pics à 15,2 ± 0,2 degrés 2θ, 15,5 ± 0,2 degrés 2θ, 21,7 ± 0,2 degrés 2θ, 22,1 ± 0,2 degrés 2θ, 23,0 ± 0,2 degrés 2θ, 26,2 ± 0,2 degrés 2θ et 29,9 ± 0,2 degrés 2θ, dans laquelle le diagramme de diffraction des rayons X sur poudre est obtenu à l'aide d'un rayonnement CuKα, destinée à être utilisée dans le traitement d'un cancer chez un patient, dans laquelle ledit traitement comprend en outre l'administration au patient d'une quantité thérapeutiquement efficace d'un anticorps anti-PD-1, d'un anticorps anti-PD-L1 ou de témozolomide.

2. Forme polymorphe destinée à être utilisée selon la revendication 1, dans laquelle ledit traitement comprend en outre l'administration au patient d'une quantité thérapeutiquement efficace d'un anticorps anti-PD-1.

3. Forme polymorphe destinée à être utilisée selon la revendication 2, dans laquelle l'anticorps anti-PD-1 est choisi dans le groupe constitué par le nivolumab, le pembrolizumab, le pidilizumab et STI-1110.

4. Forme polymorphe destinée à être utilisée selon la revendication 1, dans laquelle ledit traitement comprend en outre l'administration au patient d'une quantité thérapeutiquement efficace d'un anticorps anti-PD-L1.

5. Forme polymorphe destinée à être utilisée selon la revendication 4, dans laquelle l'anticorps anti-PD-L1 est choisi dans le groupe constitué par l'avélumab, l'atézolizumab, le durvalumab et STI-1014.

6. Forme polymorphe destinée à être utilisée selon la revendication 1, dans laquelle ledit traitement comprend en outre l'administration au patient d'une quantité thérapeutiquement efficace de témozolomide.

7. Forme polymorphe destinée à être utilisée selon l'une quelconque des revendications 1 à 6, dans laquelle la Forme X (citrate) de TG02 présente une distribution granulométrique moyenne de 10 µm ou moins.

8. Forme polymorphe destinée à être utilisée selon l'une quelconque des revendications 1 à 7, dans laquelle le cancer est une tumeur solide.

9. Forme polymorphe destinée à être utilisée selon l'une quelconque des revendications 1 à 7, dans laquelle le cancer est une hémopathie maligne.

10. Forme polymorphe destinée à être utilisée selon l'une quelconque des revendications 1 à 7, dans laquelle le cancer est choisi dans le groupe constitué par le cancer des surrénales, le carcinome à cellules acineuses, le neurinome de l'acoustique, le mélanome lentigineux acral, l'acrospirome, la leucémie aiguë à éosinophiles, la leucémie érythroïde aiguë, la leucémie lymphoblastique aiguë, la leucémie mégacaryoblastique aiguë, la leucémie monocytaire aiguë, la leucémie promyélocytaire aiguë, l'adénocarcinome, le carcinome adénoïde kystique, l'adénome, l'adénoaméloblastome, le carcinome adénosquameux, le néoplasme du tissu adipeux, le carcinome corticosurrénalien, la leucémie/le lymphome à cellules T de l'adulte, la leucémie agressive à cellules NK, le lymphome lié au SIDA, le rhabdomyosarcome alvéolaire, le sarcome alvéolaire des parties molles, le fibrome améloblastique, le lymphome anaplastique à grandes cellules, le cancer anaplastique de la thyroïde, le lymphome à cellules T angio-immunoblastique, l'angiomyolipome, l'angiosarcome, l'astrocytome, la tumeur rhabdoïde tératoïde atypique, la leucémie lymphocytaire chronique à cellules B, la leucémie prolymphocytaire à cellules B, le lymphome à cellules B, le carcinome basocellulaire, le cancer des voies biliaires, le cancer de la vessie, le blastome, le cancer des os, la tumeur de Brenner, la tumeur brune, le lymphome de Burkitt, le cancer du sein, le cancer du cerveau, le carcinome, le carcinome in situ, le carcinosarcome, la tumeur du cartilage, le cémentome, le sarcome myéloïde, le chondrome, le chordome, le choriocarcinome, le papillome du plexus choroïde, le sarcome à cellules claires du rein, le craniopharyngiome, le lymphome cutané à cellules T, le cancer du col de l'utérus, le cancer colorectal, la maladie de Degos, la tumeur desmoplastique à petites cellules rondes, le lymphome diffus à grandes cellules B, la tumeur neuroépithéliale dysembryoplastique, le dysgerminome, le carcinome embryonnaire, le néoplasme des glandes endocrines, la tumeur du sinus endodermique, le lymphome à cellules T associé à une entéropathie, le cancer de l'œsophage, le fœtus in fœtu, le fibrome, le fibrosarcome, le lymphome folliculaire, le cancer folliculaire de la thyroïde, le ganglioneurome, le cancer gastro-intestinal, la tumeur germinale, le choriocarcinome gestationnel, le fibroblastome à cellules géantes, la tumeur à cellules géantes de l'os, la tumeur gliale, le glioblastome, le gliome, la gliomatose cérébrale, le glucagonome, le gonadoblastome, la tumeur de la granulosa, le gynandroblastome, le cancer de la vésicule biliaire, le cancer gastrique, la leucémie à tricholeucocytes, l'hémangioblastome, le cancer de la tête et du cou, l'hémangiopéricytome, l'hépatoblastome, le carcinome hépatocellulaire, le lymphome hépatosplénique à cellules T, le lymphome de Hodgkin, le lymphome non hodgkinien, le carcinome lobulaire invasif, le cancer de l'intestin, le cancer du rein, le cancer du larynx, le lentigo malin, le carcinome létal de la ligne médiane, la leucémie, la tumeur à cellules de Leydig, le liposarcome, le cancer du poumon, le lymphangiome, le lymphangiosarcome, le lymphoépithéliome, le lymphome, la leucémie lymphocytaire aiguë, la leucémie myéloïde aiguë, la leucémie lymphocytaire chronique, le cancer du foie, le cancer du poumon à petites cellules, le cancer du poumon non à petites cellules, le lymphome du MALT, l'histiocytome fibreux malin, la tumeur maligne de la gaine nerveuse périphérique, la tumeur maligne du triton, le lymphome à cellules du manteau, le lymphome de la zone marginale à cellules B, la leucémie à mastocytes, la tumeur germinale médiastinale, le carcinome médullaire du sein, le cancer médullaire de la thyroïde, le médulloblastome, le mélanome, le méningiome, le cancer à cellules de Merkel, le mésothéliome, le carcinome urothélial métastatique, la tumeur müllérienne mixte, la tumeur mucineuse, le myélome multiple, le néoplasme des tissus musculaires, le mycosis fongoïde, le liposarcome myxoïde, le myxome, le myxosarcome, le carcinome du nasopharynx, le neurinome, le neuroblastome, le neurofibrome, le névrome, le mélanome nodulaire, le cancer oculaire, l'oligoastrocytome, l'oligodendrogliome, l'oncocytome, le méningiome de la gaine du nerf optique, la tumeur du nerf optique, le cancer de la cavité buccale, l'ostéosarcome, le cancer de l'ovaire, la tumeur de Pancoast, le cancer papillaire de la thyroïde, le paragangliome, le pinéaloblastome, le pinéocytome, le pituicytome, l'adénome hypophysaire, la tumeur hypophysaire, le plasmocytome, le polyembryome, le ymphome lymphoblastique T précurseur, le lymphome primitif du système nerveux central, le lymphome primitif des séreuses, le cancer péritonéal primitif, le cancer de la prostate, le cancer du pancréas, le cancer du pharynx, le pseudomyxome péritonéal, le carcinome à cellules rénales, le carcinome médullaire rénal, le rétinoblastome, le rhabdomyome, le rhabdomyosarcome, la transformation de Richter, le cancer du rectum, le sarcome, le Schwannomatose, le séminome, la tumeur à cellules de Sertoli, la tumeur stromale des cordons sexuels et des gonades, le carcinome à cellules en bague à chaton, le cancer de la peau, les tumeurs à petites cellules rondes bleues, le carcinome à petites cellules, le sarcome des tissus mous, le somatostatinome, la cancer du ramoneur, la tumeur de la moelle épinière, le lymphome splénique de la zone marginale, le carcinome épidermoïde, le sarcome synovial, la maladie de Sézary, le cancer de l'intestin grêle, le carcinome épidermoïde, le cancer de l'estomac, le lymphome à cellules T, le cancer des testicules, le thécome, le cancer de la thyroïde, le carcinome transitionnel, le cancer de la gorge, le cancer de l'ouraque, le cancer urogénital, le carcinome urothélial, le mélanome uvéal, le cancer de l'utérus, le carcinome verruqueux, le gliome des voies optique, le cancer de la vulve, le cancer du vagin, la macroglobulinémie de Waldenström, la tumeur de Warthin, la tumeur de Wilms et le gliome pontique diffus.

11. Forme polymorphe destinée à être utilisée selon l'une quelconque des revendications 1 à 7, dans laquelle le cancer est choisi dans le groupe constitué par le myélome multiple, le carcinome hépatocellulaire, le glioblastome, le cancer du poumon, le cancer du sein, le cancer de la tête et du cou, le cancer de la prostate, le mélanome, le cancer colorectal et le gliome pontique diffus.

12. Forme polymorphe destinée à être utilisée selon la revendication 11, dans laquelle le cancer est un carcinome hépatocellulaire.

13. Forme polymorphe destinée à être utilisée selon la revendication 11, dans laquelle le cancer est un glioblastome.

14. Forme polymorphe destinée à être utilisée selon la revendication 11, dans laquelle le cancer est un cancer de la prostate.

15. Forme polymorphe destinée à être utilisée selon la revendication 11, dans laquelle le cancer est un gliome pontique diffus.
